# EUROPEAN PATENT APPLICATION

(11) **EP 4 524 160 A2**
(43) Date of publication of application: **19.03.2025**
(21) Application number: 24222564.7
(22) Date of filing: 01.11.2022
(51) Int. Cl.: C07K 16/28

(54) **COMPOSITIONS AND METHODS FOR THE MODULATION OF BETA CHAIN-MEDIATED IMMUNITY**

(30) Priority: 01.11.2021 US 202163274430 P; 01.11.2021 US 202163274446 P; 01.11.2021 US 202163274449 P
(62) Divisional of application: 22813861.6
(71) Applicant: Janssen Biotech, Inc., Horsham, PA 19044 (US)
(72) Inventor: GANESAN, Rajkumar, Blue Bell, PA 19422 (US); GREWAL, Iqbal, S., Spring House, PA 19477 (US); SINGH, Sanjaya, Spring House, PA 19477 (US); HANSEN, Michael, Riis, Spring House, PA 19477 (US)
(74) Representative: Carpmaels & Ransford LLP

(57) **Abstract**

Anti-Vβ17 antibodies or antigen binding fragments thereof are described. Also described are nucleic acids encoding the antibodies, compositions comprising the antibodies, methods of producing the antibodies, and methods of using the antibodies for treating or preventing diseases.

## Description

### CROSS REFERENCE TO RELATED APPLICATION

This application claims the benefit of U.S. Provisional Patent Application No. 63/274,430, filed November 1, 2021, U.S. Provisional Patent Application No. 63/274,446, filed November 1, 2021, and U.S. Provisional Patent Application No. 63/274,449, filed November 1, 2021, the disclosures of which are incorporated by reference herein in their entireties.

### REFERENCE TO SEQUENCE LISTING SUBMITTED ELECTRONICALLY

This application contains a sequence listing, which is submitted electronically. The content of the electronic sequence listing (065768-135W1_Sequence Listing.xml; size: 512,333 bytes; and date of creation: October 31, 2022) is herein incorporated by reference in its entirety.

### FIELD

Provided herein, in certain aspects, are antibodies that bind to Vβ17, as well as recombinant cells containing the vectors, and compositions comprising the antibodies. Methods of making and using the antibodies are also provided.

### SUMMARY

In one aspect, the disclosure provides an antibody that binds Vβ17 comprising a VH comprising a heavy chain variable region (VH) comprising a VH complementarity determining region 1 (CDR1), a VH CDR2, and a VH CDR3 having the amino acid sequences of a VH CDR1, a VH CDR2, and a VH CDR3, respectively, of a VH having the amino acid sequence of SEQ ID NO:31; and a light chain variable region (VL) comprising a VL CDR1, a VL CDR2, and a VL CDR3 having the amino acid sequences of a VL CDR1, a VL CDR2, and a VL CDR3, respectively, of a VL having the amino acid sequence of SEQ ID NO:32.

In another aspect, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:65; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:66.

In one embodiment, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:99; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO: 100.

In another embodiment, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO: 133; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO: 134.

In one aspect, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO: 167; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO: 168.

In another aspect, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:201; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:202.

In one embodiment, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:235; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:236.

In another embodiment, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:269; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:270.

In one aspect, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:303; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:304.

In another aspect, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:337; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:338.

In one embodiment, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:371; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:372.

In another embodiment, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:405; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:406.

In one aspect, the disclosure provides an antibody that binds Vβ17 comprising a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:439; and a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:440.

The disclosure also provides an antibody, wherein the VH CDR1, VH CDR2, VH CDR3, VL CDR1, VL CDR2, and VL CDR3 amino acid sequences are according to the Kabat numbering system.

The disclosure also provides an antibody, wherein the VH CDR1, VH CDR2, VH CDR3, VL CDR1, VL CDR2, and VL CDR3 amino acid sequences are according to the Chothia numbering system.

The disclosure also provides an antibody, wherein the VH CDR1, VH CDR2, VH CDR3, VL CDR1, VL CDR2, and VL CDR3 amino acid sequences are according to the AbM numbering system.

The disclosure also provides an antibody, wherein the VH CDR1, VH CDR2, VH CDR3, VL CDR1, VL CDR2, and VL CDR3 amino acid sequences are according to the Contact numbering system.

The disclosure also provides an antibody, wherein the VH CDR1, VH CDR2, VH CDR3, VL CDR1, VL CDR2, and VL CDR3 amino acid sequences are according to the IMGT numbering system.

The disclosure also provides an antibody, wherein the antibody is a humanized antibody.

The disclosure also provides an antibody, wherein the antibody is an IgG antibody. The disclosure also provides an antibody, wherein the IgG antibody is an IgG1, IgG2, IgG3, or IgG4 antibody.

The disclosure also provides an antibody, wherein the antibody comprises a kappa light chain. The disclosure also provides an antibody, wherein the antibody comprises a lambda light chain.

The disclosure also provides an antibody, wherein the antibody is a monoclonal antibody.

The disclosure also provides an antibody, wherein the antibody binds a Vβ17 antigen. The disclosure also provides an antibody, wherein antibody binds a Vβ17 epitope.

The disclosure also provides an antibody, wherein the antibody specifically binds to Vβ17.

The disclosure also provides an antibody, wherein the antibody is multivalent. The disclosure also provides an antibody, wherein the antibody is capable of binding at least three antigens. The disclosure also provides an antibody, wherein the antibody is capable of binding at least four antigens. The disclosure also provides an antibody, wherein the antibody is capable of binding at least five antigens.

The disclosure also provides an antibody, wherein the antibody is a multispecific antibody. The disclosure also provides an antibody, wherein the antibody is a bispecific antibody. The disclosure also provides an antibody, wherein the antibody is a trispecific antibody. The disclosure also provides an antibody, wherein the antibody is a quadraspecific antibody.

In one aspect, the disclosure provides a nucleic acid encoding the antibody disclosed herein.

In one aspect, the disclosure provides a vector comprising the nucleic acid disclosed herein.

In one aspect, the disclosure provides a host cell comprising the vector disclosed herein.

In one aspect, the disclosure provides a kit comprising the vector disclosed herein and instructions for using the vector. In one aspect, the disclosure provides a kit comprising the antibody disclosed herein and instructions for using the antibody.

In one aspect, the disclosure provides a pharmaceutical composition comprising the antibody disclosed herein, and a pharmaceutically acceptable carrier.

In one aspect, the disclosure provides a method of producing the pharmaceutical composition disclosed herein, comprising combining the antibody with a pharmaceutically acceptable carrier to obtain the pharmaceutical composition.

In one aspect, the disclosure provides a method of activating a T cell expressing Vβ17, comprising contacting the antibody disclosed herein with the T cell expressing Vβ17.

The disclosure also provides a method, wherein the contacting increases CD69, CD25, and/or Granzyme B expression, as compared to a T cell expressing Vβ17 that is not contacted with the antibody disclosed herein.

In one aspect, the disclosure provides a process for making an antibody that binds to more than one target molecule, the process comprising: a step for performing a function of obtaining a first binding domain that binds to Vβ17 present on a T cell; a step for performing a function of obtaining a second binding domain that binds to a second target on the surface of a target cell; and a step for performing a function of providing an antibody that binds to Vβ17 present on a T cell and a second target on the surface of a target cell.

The disclosure also provides a process, wherein the step for performing a function of obtaining a second binding domain that binds to a second target on the surface of a target cell is repeated n times, and further comprising n steps for performing a function of providing a first binding domain that binds to Vβ17 present on a T cell and n number of target molecules, wherein n is at least 2.

In one aspect, the disclosure provides a process for making the antibody disclosed herein, the process comprising growing the host cell disclosed herein under conditions to produce the antibody. In another aspect, the method further comprises isolating the antibody.

In one aspect, the disclosure provides a method of directing a T cell expressing Vβ17 to a target cell, comprising contacting the antibody disclosed herein with the target cell, wherein a second target is present on the surface of the target cell, and wherein the contacting directs the T cell to the target cell.

In one aspect, the disclosure provides a method of inhibiting the growth or proliferation of a target cell, comprising contacting the antibody disclosed herein with the target cell having a second target present on the surface of the target cell, wherein the contacting is in the presence of a T cell expressing the Vβ17, and wherein the contacting results in the inhibition of the growth or proliferation of the target cell.

In one aspect, the disclosure provides a method of eliminating a target cell in a subject, comprising contacting the antibody disclosed herein with the target cell having a second target present on the surface of the target cell, wherein the contacting is in the presence of a T cell expressing the Vβ17, and wherein the contacting results in the elimination of the target cell.

In one aspect, the disclosure provides a method of treating a disease in a subject, comprising administering an effective amount of the antibody disclosed herein to the subject, wherein the disease is caused all or in part by a target cell having a second target present on the surface of the target cell. In another embodiment, the subject is a human. In one embodiment, the subject is a subject in need thereof.

### BRIEF DESCRIPTION OF THE DRAWINGS

The foregoing summary, as well as the following detailed description of specific embodiments of the present application, will be better understood when read in conjunction with the appended drawings. It should be understood, however, that the application is not limited to the precise embodiments shown in the drawings.
**FIG. 1** shows several monoclones with specific binding to Vβ17 identified by flow-cytometry using different cell lines and primary cells.
**FIG. 2** shows 26 monoclones induce proliferation in Vβ17+ cells.
**FIG. 3** shows differential proliferation kinetics observed for 26 purified antibodies.
**FIG. 4** shows differential CD71 upregulation kinetics observed for 26 purified antibodies.
**FIG. 5** shows the 10 best agonistic monoclones binding specifically to Vβ17 using SKW3 cell line variants.
**FIG. 6** shows 10 purified Vβ17 antibodies bind well to PBMCs.
**FIG. 7** shows 10 shortlisted monoclones differ in isotypes based on their hybrid parent.
**FIG. 8** shows an internalization assay used to screen the 10 shortlisted antibodies. Monoclones from 28B2 parent shows good internalization. Monoclones from 28C9, which had agonistic properties, shows poor internalization. Monoclones from parent 28D2 and 14H7 show no internalization.
**FIGS. 9A-9C** show an epitope binning matrix for the 10 shortlisted antibodies using Octet RED966. Purified monoclonal antibodies from 3 different parents bin to 3 different groups **(****FIG. 9B****).**
**FIGS. 10A-10C** show the 10 shortlisted antibodies bin differently compared to a commercial antibody (TCR Vβ17-PE; Beckman coulter, catalogue no. IM2048) serving as a positive control.
**FIG. 11** shows monoclones from the same parent have the same VH sequence. Heavy chain sequences of monoclones belonging to 3 different hybrids were diverse from each other.
**FIG. 12** shows monoclones from the same parent have the same VK sequence. Light chain sequences of monoclones belonging to 3 different hybrids were diverse from each other.
**FIG. 13** shows 12 new antibodies that had moderate agonistic properties and good PBMC binding.
**FIGS. 14A-14C** show monoclones from hybrid parent 23A9 bin similarly with positive control antibody TCRVβ17-PE.
**FIGS. 15A-15C** show monoclones from parent 23A9 and positive control TCRVβ17-PE bin differently to monoclones from other hybrid parents.
**FIG. 16** shows that most monoclones do not compete with positive control TCRVβ17 to bind cells expressing Vβ17.

### DETAILED DESCRIPTION

Various publications, articles and patents are cited or described in the background and throughout the specification; each one of these references is herein incorporated by reference in its entirety. Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise stated, any numerical values, such as a concentration or a concentration range described herein, are to be understood as being modified in all instances by the term "about." Thus, a numerical value typically includes ± 10% of the recited value. For example, a concentration of 1 mg/mL typically includes 0.9 mg/mL to 1.1 mg/mL. Likewise, a concentration range of 1% to 10% (w/v) includes 0.9% (w/v) to 11% (w/v). As used herein, the use of a numerical range expressly includes all possible subranges, all individual numerical values within that range, including integers within such ranges and fractions of the values unless the context clearly indicates otherwise.

Unless otherwise indicated, the term "at least" preceding a series of elements is to be understood to refer to every element in the series.

Those skilled in the art will recognize or be able to ascertain using no more than routine experimentation, many equivalents to the specific embodiments described herein. Such equivalents are intended to be encompassed by the invention.

As used herein, the terms "comprises," "comprising," "includes," "including," "has," "having," "contains" or "containing," or any other variation thereof, will be understood to imply the inclusion of a stated integer or group of integers but not the exclusion of any other integer or group of integers and are intended to be non-exclusive or open-ended. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

As used herein, the term "consists of," or variations such as "consist of" or "consisting of," as used throughout the specification and claims, indicate the inclusion of any recited integer or group of integers, but that no additional integer or group of integers can be added to the specified method, structure, or composition.

As used herein, the term "consists essentially of," or variations such as "consist essentially of" or "consisting essentially of," as used throughout the specification and claims, indicate the inclusion of any recited integer or group of integers, and the optional inclusion of any recited integer or group of integers that do not materially change the basic or novel properties of the specified method, structure or composition.

As used herein, "subject" means any animal, preferably a mammal, most preferably a human. The term "mammal" as used herein, encompasses any mammal. Examples of mammals include, but are not limited to, cows, horses, sheep, pigs, cats, dogs, mice, rats, rabbits, guinea pigs, monkeys, humans, *etc..* In a specific embodiments, the subject is a human.

It should also be understood that the terms "about," "approximately," "generally," "substantially," and like terms, used herein when referring to a dimension or characteristic of a component of embodiments provided herein, indicate that the described dimension/characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (*e.g.,* rounding, measurement or other systematic errors, manufacturing tolerances, *etc*.), would not vary the least significant digit.

The terms "identical" or percent "identity," in the context of two or more nucleic acids or polypeptide sequences (*e.g.,* Vβ17 antibody and polynucleotides that encode them, Vβ17 polynucleotides that encode them), refer to two or more sequences or subsequences that are the same or have a specified percentage of amino acid residues or nucleotides that are the same, when compared and aligned for maximum correspondence, as measured using one of the following sequence comparison algorithms or by visual inspection.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g.,* by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally, Current Protocols in Molecular Biology, F.M. Ausubel et al., eds., Current Protocols, a joint venture between Greene Publishing Associates, Inc. and John Wiley & Sons, Inc., (1995 Supplement) (Ausubel)).

Examples of algorithms that are suitable for determining percent sequence identity and sequence similarity are the BLAST and BLAST 2.0 algorithms, which are described in Altschul et al. (1990) J. Mol. Biol. 215: 403-410 and Altschul et al. (1997) Nucleic Acids Res. 25: 3389-3402, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.,* supra). These initial neighborhood word hits act as seeds for initiating searches to find longer HSPs containing them. The word hits are then extended in both directions along each sequence for as far as the cumulative alignment score can be increased.

Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix (see Henikoff & Henikoff, Proc. Natl. Acad. Sci. USA 89:10915 (1989)).

In addition to calculating percent sequence identity, the BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

A further indication that two nucleic acid sequences or polypeptides are substantially identical is that the polypeptide encoded by the first nucleic acid is immunologically cross reactive with the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules hybridize to each other under stringent conditions.

As used herein, the term "polynucleotide," synonymously referred to as "nucleic acid molecule," "nucleotides" or "nucleic acids," refers to any polyribonucleotide or polydeoxyribonucleotide, which can be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that can be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications can be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short nucleic acid chains, often referred to as oligonucleotides.

As used herein, the term "vector" is a replicon in which another nucleic acid segment can be operably inserted so as to bring about the replication or expression of the segment.

As used herein, the term "host cell" refers to a cell comprising a nucleic acid molecule provided herein. The "host cell" can be any type of cell, *e.g.,* a primary cell, a cell in culture, or a cell from a cell line. In one embodiment, a "host cell" is a cell transfected with a nucleic acid molecule provided herein. In another embodiment, a "host cell" is a progeny or potential progeny of such a transfected cell. A progeny of a cell may or may not be identical to the parent cell, *e.g.,* due to mutations or environmental influences that can occur in succeeding generations or integration of the nucleic acid molecule into the host cell genome.

The term "expression" as used herein, refers to the biosynthesis of a gene product. The term encompasses the transcription of a gene into RNA. The term also encompasses translation of RNA into one or more polypeptides, and further encompasses all naturally occurring post-transcriptional and post-translational modifications. The expressed antibody can be within the cytoplasm of a host cell, into the extracellular milieu such as the growth medium of a cell culture or anchored to the cell membrane.

As used herein, the terms "peptide," "polypeptide," or "protein" can refer to a molecule comprised of amino acids and can be recognized as a protein by those of skill in the art. The conventional one-letter or three-letter code for amino acid residues is used herein. The terms "peptide," "polypeptide," and "protein" can be used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc*.), as well as other modifications known in the art.

The peptide sequences described herein are written according to the usual convention whereby the N-terminal region of the peptide is on the left and the C-terminal region is on the right. Although isomeric forms of the amino acids are known, it is the L-form of the amino acid that is represented unless otherwise expressly indicated.

### Antibodies

Provided herein are Vβ17 antibodies or antigen-binding fragments thereof, nucleic acids and expression vectors encoding the antibodies, recombinant cells containing the vectors, and compositions comprising the antibodies. Methods of making the antibodies, and methods of using the antibodies to treat diseases are also provided. The antibodies disclosed herein possess one or more desirable functional properties, including but not limited to high-affinity binding to Vβ17 or high specificity to Vβ17. In certain embodiments, the antibodies disclosed herein possess the ability to treat or prevent a disease or disorder when administered to a subject alone or in combination with other therapies.

As used herein, the term "antibody" is used in a broad sense and includes immunoglobulin or antibody molecules including human, humanized, composite and chimeric antibodies and antibody fragments that are monoclonal or polyclonal. In general, antibodies are proteins or peptide chains that exhibit binding specificity to a specific antigen. Antibody structures are well known. Immunoglobulins can be assigned to five major classes (i.e., IgA, IgD, IgE, IgG and IgM), depending on the heavy chain constant domain amino acid sequence. IgA and IgG are further sub-classified as the isotypes IgA1, IgA2, IgG1, IgG2, IgG3 and IgG4. Accordingly, the antibodies provided herein can be of any of the five major classes or corresponding sub-classes. In specific embodiments, the antibodies provided herein are IgG1, IgG2, IgG3 or IgG4. Antibody light chains of vertebrate species can be assigned to one of two clearly distinct types, namely kappa and lambda, based on the amino acid sequences of their constant domains. Accordingly, the antibodies provided herein can, in certain embodiments, contain a kappa light chain constant domain. The antibodies provided herein can, in certain embodiments, also contain a lambda light chain constant domain. According to particular embodiments, the antibodies provided herein include heavy and/or light chain constant regions from rat or human antibodies. In specific embodiments, the constant region is a human constant region.

In addition to the heavy and light constant domains, antibodies contain an antigen-binding region that is made up of a light chain variable region (VL) and a heavy chain variable region (VH), each of which contains three domains (*i.e*., complementarity determining regions 1 (CDR1), CDR2 and CDR3. A "CDR" refers to one of three hypervariable regions (HCDR1, HCDR2 or HCDR3) within the non-framework region of the immunoglobulin (Ig or antibody) VH β-sheet framework, or one of three hypervariable regions (LCDR1, LCDR2 or LCDR3) within the non-framework region of the antibody VL β-sheet framework. Accordingly, CDRs are variable region sequences interspersed within the framework region sequences. CDR regions are well known to those skilled in the art and have been defined by, for example, Kabat as the regions of most hypervariability within the antibody variable (V) domains (Kabat et al., J. Biol. Chem. 252:6609-6616 (1977); Kabat, Adv. Prot. Chem. 32:1-75 (1978)). CDR region sequences also have been defined structurally by Chothia as those residues that are not part of the conserved β-sheet framework, and thus are able to adapt different conformations (Chothia and Lesk, J. Mol. Biol. 196:901-917 (1987)). Both terminologies are well recognized in the art. CDR region sequences have also been defined by AbM, Contact and IMGT. Exemplary CDR region sequences are illustrated herein, for example, in the tables provided below. The positions of CDRs within a canonical antibody variable region have been determined by comparison of numerous structures (Al-Lazikani et al., J. Mol. Biol. 273:927-948 (1997); Morea et al., Methods 20:267-279 (2000)). Because the number of residues within a hypervariable region varies in different antibodies, additional residues relative to the canonical positions are conventionally numbered with a, b, c and so forth next to the residue number in the canonical variable region numbering scheme (Al-Lazikani *et al., supra* (1997)). Such nomenclature is similarly well known to those skilled in the art.

The light chain variable region CDR1 domain is interchangeably referred to herein as LCDR1 or VL CDR1. The light chain variable region CDR2 domain is interchangeably referred to herein as LCDR2 or VL CDR2. The light chain variable region CDR3 domain is interchangeably referred to herein as LCDR3 or VL CDR3. The heavy chain variable region CDR1 domain is interchangeably referred to herein as HCDR1 or VH CDR1. The heavy chain variable region CDR2 domain is interchangeably referred to herein as HCDR2 or VH CDR2. The heavy chain variable region CDR1 domain is interchangeably referred to herein as HCDR3 or VH CDR3.

The term "hypervariable region", such as a VH or VL, when used herein refers to the regions of an antibody variable region that are hypervariable in sequence and/or form structurally defined loops. Generally, antibodies comprise six hypervariable regions; three in the VH (HCDR1, HCDR2, HCDR3), and three in the VL (LCDR1, LCDR2, LCDR3). A number of hypervariable region delineations are in use and are encompassed herein. The "Kabat" CDRs are based on sequence variability and are the most commonly used (see, *e.g.,* Kabat et al., Sequences of Proteins of Immunological Interest, 5th Ed. Public Health Service, National Institutes of Health, Bethesda, MD. (1991)). "Chothia" refers instead to the location of the structural loops (see, *e.g.,* Chothia and Lesk, J. Mol. Biol. 196:901-917 (1987)). The end of the Chothia CDR-HCDR1 loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The "AbM" hypervariable regions represent a compromise between the Kabat CDRs and Chothia structural loops, and are used by Oxford Molecular's AbM antibody modeling software (see, e.g., Martin, in Antibody Engineering, Vol. 2, Chapter 3, Springer Verlag). "Contact" hypervariable regions are based on an analysis of the available complex crystal structures.

Recently, a universal numbering system has been developed and widely adopted, ImMunoGeneTics (IMGT) Information System^{®} (Lafranc et al., Dev. Comp. Immunol. 27(1):55-77 (2003)). IMGT is an integrated information system specializing in immunoglobulins (IG), T cell receptors (TR) and major histocompatibility complex (MHC) of human and other vertebrates. Herein, the CDRs are referred to in terms of both the amino acid sequence and the location within the light or heavy chain. As the "location" of the CDRs within the structure of the immunoglobulin variable domain is conserved between species and present in structures called loops, by using numbering systems that align variable domain sequences according to structural features, CDR and framework residues and are readily identified. This information can be used in grafting and replacement of CDR residues from immunoglobulins of one species into an acceptor framework from, typically, a human antibody. An additional numbering system (AHon) has been developed by Honegger and Plückthun, J. Mol. Biol. 309: 657-670 (2001). Correspondence between the numbering system, including, for example, the Kabat numbering and the IMGT unique numbering system, is well known to one skilled in the art (see, *e.g.,* Kabat, *supra;* Chothia and Lesk, *supra;* Martin, *supra;* Lefranc *et al., supra).* An Exemplary system, shown herein, combines Kabat and Chothia.

| | **Exemplary** | **IMGT** | **Kabat** | **AbM** | **Chothia** | **Contact** |
|---|---|---|---|---|---|---|
| V_{H} CDR1 | 26-35 | 27-38 | 31-35 | 26-35 | 26-32 | 30-35 |
| V_{H} CDR2 | 50-65 | 56-65 | 50-65 | 50-58 | 53-55 | 47-58 |
| V_{H} CDR3 | 95-102 | 105-117 | 95-102 | 95-102 | 96-101 | 93-101 |
| V_{L} CDR1 | 24-34 | 27-38 | 24-34 | 24-34 | 26-32 | 30-36 |
| V_{L} CDR2 | 50-56 | 56-65 | 50-56 | 50-56 | 50-52 | 46-55 |
| V_{L} CDR3 | 89-97 | 105-117 | 89-97 | 89-97 | 91-96 | 89-96 |

Hypervariable regions may comprise "extended hypervariable regions" as follows: 24-36 or 24-34 (LCDR1), 46-56 or 50-56 (LCDR2) and 89-97 or 89-96 (LCDR3) in the VL and 26-35 or 26-35A (HCDR1), 50-65 or 49-65 (HCDR2) and 93-102, 94-102, or 95-102 (HCDR3) in the VH. CDR sequences, reflecting each of the above numbering schemes, are provided herein, including in Tables 1-6.

The term "constant region" or "constant domain" refers to a carboxy terminal portion of the light and heavy chain which is not directly involved in binding of the antibody to antigen but exhibits various effector function, such as interaction with the Fc receptor. The terms refer to the portion of an immunoglobulin molecule having a more conserved amino acid sequence relative to the other portion of the immunoglobulin, the variable region, which contains the antigen binding site. The constant region may contain the CH1, CH2 and CH3 regions of the heavy chain and the CL region of the light chain.

The term "framework" or "FR" residues are those variable region residues flanking the CDRs. FR residues are present, for example, in chimeric, humanized, human, domain antibodies, diabodies, linear antibodies, and bispecific antibodies. FR residues are those variable domain residues other than the hypervariable region residues or CDR residues.

As used herein, the term an "isolated antibody" refers to an antibody which is substantially free of other antibodies having different antigenic specificities (*e.g.,* an isolated antibody that specifically binds to Vβ17 is substantially free of antibodies that do not bind to Vβ17). In addition, an isolated antibody is substantially free of other cellular material and/or chemicals.

As used herein, the term "monoclonal antibody" refers to an antibody obtained from a population of substantially homogeneous antibodies, i.e., the individual antibodies comprising the population are identical except for possible naturally occurring mutations that can be present in minor amounts. Monoclonal antibodies provided herein can be made by the hybridoma method, phage display technology, single lymphocyte gene cloning technology, or by recombinant DNA methods. For example, the monoclonal antibodies can be produced by a hybridoma which includes a B cell obtained from a transgenic nonhuman animal, such as a transgenic mouse or rat, having a genome comprising a human heavy chain transgene and a light chain transgene.

As used herein, the term "antigen-binding fragment" refers to an antibody fragment such as, for example, a diabody, a Fab, a Fab', a F(ab')2, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), a single domain antibody (sdAb) an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. An antigen-binding fragment is capable of binding to the same antigen to which the parent antibody or a parent antibody fragment binds. According to particular embodiments, the antigen-binding fragment comprises a light chain variable region, a light chain constant region, and an Fd segment of the heavy chain. According to other particular embodiments, the antigen-binding fragment comprises Fab and F(ab').

As used herein, the term "single-chain antibody" refers to a conventional single-chain antibody in the field, which comprises a heavy chain variable region and a light chain variable region connected by a short peptide of about 15 to about 20 amino acids. As used herein, the term "single domain antibody" refers to a conventional single domain antibody in the field, which comprises a heavy chain variable region and a heavy chain constant region or which comprises only a heavy chain variable region.

As used herein, the term "human antibody" refers to an antibody produced by a human or an antibody having an amino acid sequence corresponding to an antibody produced by a human made using any technique known in the art. This definition of a human antibody includes intact or full-length antibodies, fragments thereof, and/or antibodies comprising at least one human heavy and/or light chain polypeptide.

As used herein, the term "humanized antibody" refers to a non-human antibody that is modified to increase the sequence homology to that of a human antibody, such that the antigen-binding properties of the antibody are retained, but its antigenicity in the human body is reduced.

As used herein, the term "chimeric antibody" refers to an antibody wherein the amino acid sequence of the immunoglobulin molecule is derived from two or more species. The variable region of both the light and heavy chains often corresponds to the variable region of an antibody derived from one species of mammal (*e.g.,* mouse, rat, rabbit, *etc*.) having the desired specificity, affinity, and capability, while the constant regions correspond to the sequences of an antibody derived from another species of mammal (*e.g.,* human) to avoid eliciting an immune response in that species.

As used herein, the term "multispecific antibody" refers to an antibody that comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, the first and second epitopes do not overlap or do not substantially overlap. In an embodiment, the first and second epitopes are on different antigens, e.g., the different proteins (or different subunits of a multimeric protein). In an embodiment, a multispecific antibody comprises a third, fourth, or fifth immunoglobulin variable domain. In an embodiment, a multispecific antibody is a bispecific antibody molecule, a trispecific antibody molecule, or a tetraspecific antibody molecule.

As used herein, the term "bispecific antibody" refers to a multispecific antibody that binds no more than two epitopes or two antigens, e.g., two eptiopes or antigens that are different genetic targets (e.g., Vβ17, CD69, or CD25). A bispecific antibody is characterized by a first immunoglobulin variable domain sequence which has binding specificity for a first epitope (*e.g.,* an epitope on a Vβ17 antigen) and a second immunoglobulin variable domain sequence that has binding specificity for a second epitope. In an embodiment, the first and second epitopes are on different antigens, *e.g.,* the different proteins (or different subunits of a multimeric protein). In an embodiment, a bispecific antibody comprises a heavy chain variable domain sequence and a light chain variable domain sequence which have binding specificity for a first epitope and a heavy chain variable domain sequence and a light chain variable domain sequence which have binding specificity for a second epitope. In an embodiment, a bispecific antibody comprises a half antibody, or fragment thereof, having binding specificity for a first epitope and a half antibody, or fragment thereof, having binding specificity for a second epitope. In an embodiment, a bispecific antibody comprises a scFv, or fragment thereof, having binding specificity for a first epitope, and a scFv, or fragment thereof, having binding specificity for a second epitope.

As used herein, the term "Vβ17" refers to a T cell receptor, which is expressed in response to an immune response on a cytotoxic T cell. Vβ17-expressing CD8+ T cells are commonly produced in response to influenza A virus exposure in a subject. Vβ17-expressing CD8+ T cells provide great recall in response to influenza exposure in the subject. The term "Vβ17" includes any Vβ17 variant, isoform, and species homolog, which is naturally expressed by cells (including T cells) or can be expressed on cells transfected with genes or cDNA encoding the polypeptide. Unless noted, preferably the Vβ17 is a human Vβ17. An exemplary human Vβ17 amino acid sequence is provided by GenBank Accession Number AAB49730.1.

As used herein, an antibody that "specifically binds to Vβ17" refers to an antibody that binds to a Vβ17, preferably a human Vβ17, with a KD of 1×10⁻⁷ M or less, such as 1×10⁻⁸ M or less, 5×10⁻⁹ M or less, 1×10⁻⁹ M or less, 5×10⁻¹⁰ M or less, or 1×10⁻¹⁰ M or less.

The term "KD" refers to the dissociation constant, which is obtained from the ratio of Kd to Ka (*i.e.,* Kd/Ka) and is expressed as a molar concentration (M). KD values for antibodies can be determined using methods in the art in view of the present disclosure. For example, the KD of an antibody can be determined by using surface plasmon resonance, such as by using a biosensor system, *e.g.,* a Biacore^{®} system, or by using bio-layer interferometry technology, such as an Octet RED96 system. The smaller the value of the KD of an antibody, the higher affinity that the antibody binds to a target antigen.

In one aspect, provided herein is an antibody that binds to Vβ17. In some embodiments, the antibody comprises a heavy chain variable region and a light chain variable region. In a some embodiments, the Vβ17 antibody is not a single domain antibody or nanobody. In some embodiments, the Vβ17 antibody is a humanized antibody.

In certain embodiments, provided herein is a Vβ17 antibody comprising a VH region, VL region, VH CDR1, VH CDR2, VH CDR3, VL CDR1, VL CDR2, and/or VL CDR3 of any one of the antibodies described herein. In some embodiments, provided herein is a Vβ17 antibody comprising a VH region of any one of the antibodies described herein. In some embodiments, provided herein is a Vβ17 antibody comprising a VL region of any one of the antibodies described herein. In some embodiments, provided herein is a Vβ17 antibody comprising a VH region of any one of the antibodies described herein, and a VL region of any one of the antibodies described herein. In some embodiments, provided herein is a Vβ17 antibody comprising a VH CDR1, VH CDR2, and VH CDR3 of any one of the antibodies described herein. In some embodiments, provided herein is a Vβ17 antibody comprising a VL CDR1, VL CDR2, and VL CDR3 of any one of the antibodies described herein. In some embodiments, provided herein is a Vβ17 antibody comprising a VH CDR1, VH CDR2, and VH CDR3 of any one of the antibodies described herein; and a VL CDR1, VL CDR2, and VL CDR3 of any one of the antibodies described herein. Representative VH and VL amino acid sequences, including VH CDR1, VH CDR2, VH CDR3, VL CDR1, VL CDR2 and VL CDR3 amino acid sequences, of Vβ17 antibodies provided herein are provided in Tables 1-6.

In some embodiments, the antibody specifically binds Vβ17. In other embodiments, the Vβ17 is present on the surface of a T cell.

In some embodiments, the antibody is a humanized antibody. In certain embodiments, the antibody is an IgG antibody. In other embodiments, the IgG antibody is an IgG1, IgG2, IgG3, or IgG4 antibody. In some embodiments, the antibody is a bispecific antibody. In certain embodiments, the antibody is multivalent. In other embodiments, the antibody is capable of binding at least three antigens. In some embodiments, the antibody is capable of binding at least five antigens.

In certain embodiments, provided is a Vβ17 antibody that is an intact antibody. In other embodiments, provided is a Vβ17 antibody is an antigen binding fragment of the Vβ17 antibody. In some embodiments, the antigen binding fragment of the Vβ17 antibody is a functional fragment.

In some embodiments, the antigen binding fragment is a diabody. In some embodiments, the antigen binding fragment is a Fab. In some embodiments, the antigen binding fragment is a Fab'. In some embodiments, the antigen binding fragment is a F(ab')2. In some embodiments, the antigen binding fragment is a Fv fragment. In some embodiments, the antigen binding fragment is a disulfide stabilized Fv fragment (dsFv). In some embodiments, the antigen binding fragment is a (dsFv)₂. In some embodiments, the antigen binding fragment is a bispecific dsFv (dsFv-dsFv'). In some embodiments, the antigen binding fragment is a disulfide stabilized diabody (ds diabody). In some embodiments, the antigen binding fragment is a single-chain antibody molecule (scFv). In some embodiments, the antigen binding fragment is a single domain antibody (sdAb). In some embodiments, the antigen binding fragment is an scFv dimer (bivalent diabody). In some embodiments, the antigen binding fragment is a multispecific antibody formed from a portion of an antibody comprising one or more CDRs. In some embodiments, the antigen binding fragment is a camelized single domain antibody. In some embodiments, the antigen binding fragment is a nanobody. In some embodiments, the antigen binding fragment is a domain antibody. In some embodiments, the antigen binding fragment is a bivalent domain antibody. In some embodiments, the antigen binding fragment is an antibody fragment that binds to an antigen but does not comprise a complete antibody structure.

In specific embodiments, the Vβ17 antibody comprises a VH region and a VL region. In some embodiments, the Vβ17 antibody is a single chain antibody. In some embodiments, the Vβ17 antibody is a single domain antibody. In some embodiments, the Vβ17 antibody is a nanobody. In certain embodiments, the Vβ17 antibody is a VHH antibody. In certain embodiments, the Vβ17 antibody is a llama antibody. In some embodiments, the Vβ17 antibody is not a single chain antibody. In some embodiments, the Vβ17 antibody is not a single domain antibody. In some embodiments, the Vβ17 antibody is not a nanobody. In certain embodiments, the Vβ17 antibody is not a VHH antibody. In certain embodiments, the Vβ17 antibody is not a llama antibody. In some embodiments, the Vβ17 antibody is a multispecific antibody. In other embodiments, the Vβ17 is a bispecific antibody. In certain embodiments, the multispecific antibody comprises an antigen binding fragment of a Vβ17 antibody provided herein. In other embodiments, the bispecific antibody comprises an antigen binding fragment of a Vβ17 antibody provided herein. In some embodiments, the Vβ17 antibody is an agonistic antibody. In certain embodiments, the Vβ17 antibody activates T cells. In other embodiments, the Vβ17 antibody is an antagonistic antibody. In certain embodiments, the Vβ17 antibody inactivates T cells. In some embodiments, the Vβ17 antibody blocks activation of T cells. In some embodiments, the Vβ17 antibody modulates the activity of T cells. In some embodiments, the Vβ17 antibody neither activates or inactivates the activity of T cells. In specific embodiments, the T cells are human T cells.

In some embodiments, the VH CDR1, VH CDR2, VH CDR3, VL CDR1, VL CDR2, and VL CDR3 sequences are according to the Kabat numbering system. In some embodiments, the VH CDR1, VH CDR2, VH CDR3, VL CDR1, VL CDR2, and VL CDR3 sequences are according to the Chothia numbering system. In some embodiments, the VH CDR1, VH CDR2, VH CDR3, VL CDR1, VL CDR2, and VL CDR3 sequences are according to the Exemplary numbering system. In some embodiments, the VH CDR1, VH CDR2, VH CDR3, VL CDR1, VL CDR2, and VL CDR3 sequences are according to the Contact numbering system. In some embodiments, the VH CDR1, VH CDR2, VH CDR3, VL CDR1, VL CDR2, and VL CDR3 sequences are according to the IMGT numbering system. In some embodiments, the VH CDR1, VH CDR2, VH CDR3, VL CDR1, VL CDR2, and VL CDR3 sequences are according to the AbM numbering system. Exemplary sets of 6 CDRs (VH CDR1-3 and VL CDR1-3) of certain antibody embodiments are provided herein. Other sets of CDRs are contemplated and within the scope of the antibody embodiments provided herein.

**Table 1. Yβ17 Antibody VH and VL Amino Acid Sequences**

| **#** | **Protein Name** | **VH AA sequence** | **VL AA sequence** |
|---|---|---|---|
| 1 | 20A9A9 | EVQLQESGPGLVQPSQSLSITCTVSGFSLTSYGVHW VRQSPGKGLEWLGIIWSAGGTDYNAAFISRLSISKD NSKSQIFFKLNSLHADDTAIYFCARGSFYDYGRLDY WGQGTTLTVSS | DIVMTQSHRFMSTSVGDRVSITCKASQDVSTAV AWYQQKPGQSPKLLIYSSSYRYTGVPDRFTGSGS GTDFTFTISSVQAEDLAVYYCQQHYGTPYTFGG GTKLEIQ |
| | | 31 | 32 |
| 2 | 20C12A3 | EVQLQESGAELVRPGTSVKMSCKASGYTFTHYWID WTKQRPGHGLEWIGNIYPGGGYTNYNENFKGKATL TADKSSSTVFMQFSSLTSDDSAIYYCTRKGYYYGSD WYFDVWGTGTTVTVSS | STSVGDRVSITCKASQDVSTAVAWYHQKPGQSP KLLIYSASYRYTGVPDRFTGSGSGTDFTFTISSVQ AEDLAVYYCQQHYTTPYTFGGGTKLEMK |
| | | 65 | 66 |
| 3 | 21G11A8 | EVQLQESGPDLKKPGETVKISCKASGYTFTTYGMN WVKQAPGKGLKWMGYLNTYSGVSTYADDFKGRF AFSLETSANTAYLQINNLRNDDTATYFCARWGNYG AMDYWGQGTSVTVSS | DIKMTQSPLSLPVSLGDQASISCRSSQSIVRSNGFT YLEWYLQKPGQSPELLIYKVSNRFSGVPDRFSGS GSGTDFTLKISRVEAEDLGVYYCFQSSHVPRTFG GGTKLEIK |
| | | 99 | 100 |
| 4 | 21G11D5 | EVQLQESGPDLKKPGETVKISCKASGYTFTTYGMN WVKQAPGKGLKWMGYLNTYSGVSTYADDFKGRF AFSLETSANTAYLQINNLRNDDTATYFCARWGNYG AMDYWGQGTSVTVSS | DIVMTQSPLSLPVSLGDQASISCRSSQSIVRSNGFT YLEWYLQKPGQSPELLIYKVSNRFSGVPDRFSGS GSGTDFTLKISRVEAEDLGVYYCFQSSHVPRTFG GGTKLEIK |
| | | 133 | 134 |
| 5 | 23A9B10 | EVQLQESGPGLVKPSQSLSLTCSVTGYSITSGYYWN WIRQFLGNKLEWMGYITYDGSNNYNPSLKDRISITR DTSKNQFFLNLNSVTTDDTATYYCARGGGYVPVW GTGTTVTVSS | IQMTQSPAFLSVSPGERVSFSCRASQNIGTSIHWY QQRTNGSPRLLIKYASESISGIPSRFSGSGSGTDFT LSINSVESEDFADYYCHQSHNWPTTFGAGTKLEL K |
| | | 167 | 168 |
| 6 | 23A9C10 | EVQLQESGPGLVKPSQSLSLTCSVTGYSITSGYYWN WIRQFLGNKLEWMGYITYDGSNNYNPSLKDRISITR DTSKNQFFLNLNSVTTDDTATYYCARGGGYVPVW GTGTTVTVSS | DIQMTQSPAFLSVSPGERVSFSCRASQNIGTSIHW YQQRTNGSPRLLIKYASESISGIPSRFSGSGSGTDF TLSINSVESEDFADYYCHQSHNWPTTFGAGTKLE LK |
| | | 201 | 202 |
| 7 | 23A9F7 | EVQLQESGPGLVKPSQSLSLTCSVTGYSITSGYYWN WIRQFLGNKLEWMGYITYDGSNNYNPSLKDRISITR DTSKNQFFLNLNSVTTDDTATYYCARGGGYVPVW GTGTTVTVSS | DVQMTQSPAFLSVSPGERVSFSCRASQNIGTSIH WYQQRTNGSPRLLIKYASESISGIPSRFSGSGSGT DFTLSINSVESEDFADYYCHQSHNWPTTFGAGTK LELK |
| | | 235 | 236 |
| 8 | 28B2A2 | EVQLQESGPELVKPGASVKMSCKASGYTFTDDNMH WVKQSHGKSLEWIGYINPNNNHTNYNQKFKGKAT LTVNKSSSTAYMELRSLTSEDSAVYFCARSTVVPFY AMDYWGQGTSVTVSS | DIVMTQSPASLSMAIGEKVTIRCITITDIDDDMNW YQQKPGEPPKLLFSEGNTLRPGVPSRFSSSGYGS DFAFTIENMLSEDVADYYCLQSDNLPLTFGAGTK LELK |
| | | 269 | 270 |
| 9 | 28B2A4 | EVQLQESGPELVKPGASVKMSCKASGYTFTDDNMH WVKQSHGKSLEWIGYINPNNNHTNYNQKFKGKAT LTVNKSSSTAYMELRSLTSEDSAVYFCARSTVVPFY AMDYWGQGTSVTVSS | DIQMTQSPASLSMAIGEKVTIRCITITDIDDDMNW YQQKPGEPPKLLFSEGNTLRPGVPSRFSSSGYGS DFAFTIENMLSEDVADYYCLQSDNLPLTFGGGTK LEIK |
| | | 303 | 304 |
| 10 | 28B2F5 | EVQLQESGPELVKPGASVKMSCKASGYTFTDDNMH WVKQSHGKSLEWIGYINPNNNHTNYNQKFKGKAT LTVNKSSSTAYMELRSLTSEDSAVYFCARSTVVPFY AMDYWGQGTSVTVSS | DIKMTQSPASLSMAIGEKVTIRCITITDIDDDMNW YQQKPGEPPKLLFSEGNTLRPGVPSRFSSSGYGS DFAFTIENMLSEDVADYYCLQSDNLPLTFGAGTK LELK |
| | | 337 | 338 |
| 11 | 28C9A5 | EVQLQESGAELVRPGTSVKMSCKASGYTFTHYWID WTKQRPGHGLEWIGNIYPGGGYTNYNENFKGKATL TADKSSSTVFMQFSSLTSDDSAIYYCTRKGYYYGSD WYFDVWGTGTTVTVSS | QIVLSQSPAIMSASPGEKVTMTCSASSSVSYMHW YQQKSGTSPKRWIYDTSKLASGVPARFSGSGSGT SYSLTISSMEAEDAATYYCQQWSSNPYTFGGGT KLEIK |
| | | 371 | 372 |
| 12 | 28D2E6 | EVQLQESGPGLVKPSQSLSLTCSVTGYFISSGYYWK WIRQFPGNKLEWMGYISYDGSNNYNPSLKNRISITR DTSKNQFFLKLNSVTAEDTATYYCARGGGYVPVW GTGTTVTVSS | DIQMTQSPLSLPVSLGDQASISCRSSQSIVRSNGN TYLEWYLQKPGQSPKLLIYKVSNRFSGVPDRFSG SGSGTDFTLKISRVEAEDLGVYYCFQSSHVPRTF GGGTKLEIK |
| | | 405 | 406 |
| 13 | 28D2E8 | EVQLQESGPGLVKPSQSLSLTCSVTGYFISSGYYWK WIRQFPGNKLEWMGYISYDGSNNYNPSLKNRISITR DTSKNQFFLKLNSVTAEDTATYYCARGGGYVPVW GTGTTVTVSS | DIKMTQSPLSLPVSLGDQASISCRSSQSIVRSNGN TYLEWYLQKPGQSPKLLIYKVSNRFSGVPDRFSG SGSGTDFTLKISRVEAEDLGVYYCFQSSHVPRTF GGGTKLEIK |
| | | 439 | 440 |

**Table 2. Yβ17 Antibody Kabat CDR Amino Acid Sequences**

| **#** | **Protein Name** | **HC KABAT CDR1** | **HC KABAT CDR2** | **HC KABAT CDR3** | **LC KABAT CDR1** | **LC KABAT CDR2** | **LC KABAT CDR3** |
|---|---|---|---|---|---|---|---|
| 1 | 20A9A9 | SYGVH | IIWSAGGTDYNAAFIS | GSFYDYGRLDY | KASQDVSTAVA | SSSYRYT | QQHYGTPYT |
| | | 1 | 2 | 3 | 4 | 5 | 6 |
| 2 | 20C12A3 | HYWID | NIYPGGGYTNYNENFKG | KGYYYGSDWYFDV | KASQDVSTAVA | SASYRYT | QQHYTTPYT |
| | | 35 | 36 | 37 | 38 | 39 | 40 |
| 3 | 21G11A8 | TYGMN | YLNTYSGVSTYADDFKG | WGNYGAMDY | RSSQSIVRSNGFTYLE | KVSNRFS | FQSSHVPRT |
| | | 69 | 70 | 71 | 72 | 73 | 74 |
| 4 | 21G11D5 | TYGMN | YLNTYSGVSTYADDFKG | WGNYGAMDY | RSSQSIVRSNGFTYLE | KVSNRFS | FQSSHVPRT |
| | | 103 | 104 | 105 | 106 | 107 | 108 |
| 5 | 23 A9B 10 | SGYYWN | YITYDGSNNYNPSLKD | GGGYVPV | RASQNIGTSIH | YASESIS | HQSHNWPTT |
| | | 137 | 138 | 139 | 140 | 141 | 142 |
| 6 | 23A9C10 | SGYYWN | YITYDGSNNYNPSLKD | GGGYVPV | RASQNIGTSIH | YASESIS | HQSHNWPTT |
| | | 171 | 172 | 173 | 174 | 175 | 176 |
| 7 | 23A9F7 | SGYYWN | YITYDGSNNYNPSLKD | GGGYVPV | RASQNIGTSIH | YASESIS | HQSHNWPTT |
| | | 205 | 206 | 207 | 208 | 209 | 210 |
| 8 | 28B2A2 | DDNMH | YINPNNNHTNYNQKFKG | STWPFYAMDY | ITITDIDDDMN | EGNTLRP | LQSDNLPLT |
| | | 239 | 240 | 241 | 242 | 243 | 244 |
| 9 | 28B2A4 | DDNMH | YINPNNNHTNYNQKFKG | STWPFYAMDY | ITITDIDDDMN | EGNTLRP | LQSDNLPLT |
| | | 273 | 274 | 275 | 276 | 277 | 278 |
| 10 | 28B2F5 | DDNMH | YINPNNNHTNYNQKFKG | STWPFYAMDY | ITITDIDDDMN | EGNTLRP | LQSDNLPLT |
| | | 307 | 308 | 309 | 310 | 311 | 312 |
| 11 | 28C9A5 | HYWID | NIYPGGGYTNYNENFKG | KGYYYGSDWYFDV | SASSSVSYMH | DTSKLAS | QQWSSNPYT |
| | | 341 | 342 | 343 | 344 | 345 | 346 |
| 12 | 28D2E6 | SGYYWK | YISYDGSNNYNPSLKN | GGGYVPV | RSSQSIVRSNGNTYLE | KVSNRFS | FQSSHVPRT |
| | | 375 | 376 | 377 | 378 | 379 | 380 |
| 13 | 28D2E8 | SGYYWK | YISYDGSNNYNPSLKN | GGGYVPV | RSSQSIVRSNGNTYLE | KVSNRFS | FQSSHVPRT |
| | | 409 | 410 | 411 | 412 | 413 | 414 |

**Table 3. Vβ17 Antibody Chothia CDR Amino Acid Sequences**

| **#** | **Protein Name** | **HC CHOTHIA CDR1** | **HC CHOTHIA CDR2** | **HC CHOTHIA CDR3** | **LC CHOTHIA CDR1** | **LC CHOTHIA CDR2** | **LC CHOTHIA CDR3** |
|---|---|---|---|---|---|---|---|
| 1 | 20A9A9 | GFSLTSY | WSAGG | GSFYDYGRLDY | KASQDVSTAVA | SSSYRYT | QQHYGTPYT |
| | | 7 | 8 | 9 | 10 | 11 | 12 |
| 2 | 20C12A3 | GYTFTHY | YPGGGY | KGYYYGSDWYFDV | KASQDVSTAVA | SASYRYT | QQHYTTPYT |
| | | 41 | 42 | 43 | 44 | 45 | 46 |
| 3 | 21G11A8 | GYTFTTY | NTYSGV | WGNYGAMDY | RSSQSIVRSNGFTYLE | KVSNRFS | FQSSHVPRT |
| | | 75 | 76 | 77 | 78 | 79 | 80 |
| 4 | 21G11D5 | GYTFTTY | NTYSGV | WGNYGAMDY | RSSQSIVRSNGFTYLE | KVSNRFS | FQSSHVPRT |
| | | 109 | 110 | 111 | 112 | 113 | 114 |
| 5 | 23A9B10 | GYSITSGY | TYDGS | GGGYVPV | RASQNIGTSIH | YASESIS | HQSHNWPTT |
| | | 143 | 144 | 145 | 146 | 147 | 147 |
| 6 | 23A9C10 | GYSITSGY | TYDGS | GGGYVPV | RASQNIGTSIH | YASESIS | HQSHNWPTT |
| | | 177 | 178 | 179 | 180 | 181 | 182 |
| 7 | 23A9F7 | GYSITSGY | TYDGS | GGGYVPV | RASQNIGTSIH | YASESIS | HQSHNWPTT |
| | | 211 | 212 | 213 | 214 | 215 | 216 |
| 8 | 28B2A2 | GYTFTDD | NPNNNH | STWPFYAMDY | ITITDIDDDMN | EGNTLRP | LQSDNLPLT |
| | | 245 | 246 | 247 | 248 | 249 | 250 |
| 9 | 28B2A4 | GYTFTDD | NPNNNH | STWPFYAMDY | ITITDIDDDMN | EGNTLRP | LQSDNLPLT |
| | | 279 | 280 | 281 | 282 | 283 | 284 |
| 10 | 28B2F5 | GYTFTDD | NPNNNH | STWPFYAMDY | ITITDIDDDMN | EGNTLRP | LQSDNLPLT |
| | | 313 | 314 | 315 | 316 | 317 | 318 |
| 11 | 28C9A5 | GYTFTHY | YPGGGY | KGYYYGSDWYFDV | SASSSVSYMH | DTSKLAS | QQWSSNPYT |
| | | 347 | 348 | 349 | 350 | 351 | 352 |
| 12 | 28D2E6 | GYFISSGY | SYDGS | GGGYVPV | RSSQSIVRSNGNTYLE | KVSNRFS | FQSSHVPRT |
| | | 381 | 382 | 383 | 384 | 385 | 386 |
| 13 | 28D2E8 | GYFISSGY | SYDGS | GGGYVPV | RSSQSIVRSNGNTYLE | KVSNRFS | FQSSHVPRT |
| | | 415 | 416 | 417 | 418 | 419 | 420 |

**Table 4. Vβ17 Antibody AbM CDR Amino Acid Sequences**

| **#** | **Protein Name** | **HC AbM CDR1** | **HC AbM CDR2** | **HC AbM CDR3** | **LC AbM CDR1** | **LC AbM CDR2** | **LC AbM CDR3** |
|---|---|---|---|---|---|---|---|
| 1 | 20A9A9 | GFSLTSYGVH | IIWSAGGTD | GSFYDYGRLDY | KASQDVSTAVA | SSSYRYT | QQHYGTPYT |
| | | 13 | 14 | 15 | 16 | 17 | 18 |
| 2 | 20C12A3 | GYTFTHYWID | NIYPGGGYTN | KGYYYGSDWYFDV | KASQDVSTAVA | SASYRYT | QQHYTTPYT |
| | | 47 | 48 | 49 | 50 | 51 | 52 |
| 3 | 21G11A8 | GYTFTTYGMN | YLNTYSGVST | WGNYGAMDY | RSSQSIVRSNGFTYLE | KVSNRFS | FQSSHVPRT |
| | | 81 | 82 | 83 | 84 | 85 | 86 |
| 4 | 21G11D5 | GYTFTTYGMN | YLNTYSGVST | WGNYGAMDY | RSSQSIVRSNGFTYLE | KVSNRFS | FQSSHVPRT |
| | | 115 | 116 | 117 | 118 | 119 | 120 |
| 5 | 23 A9B 10 | GYSITSGYYWN | YITYDGSNN | GGGYVPV | RASQNIGTSIH | YASESIS | HQSHNWPTT |
| | | 149 | 150 | 151 | 152 | 153 | 154 |
| 6 | 23A9C10 | GYSITSGYYWN | YITYDGSNN | GGGYVPV | RASQNIGTSIH | YASESIS | HQSHNWPTT |
| | | 183 | 184 | 185 | 186 | 187 | 188 |
| 7 | 23A9F7 | GYSITSGYYWN | YITYDGSNN | GGGYVPV | RASQNIGTSIH | YASESIS | HQSHNWPTT |
| | | 217 | 218 | 219 | 220 | 221 | 222 |
| 8 | 28B2A2 | GYTFTDDNMH | YINPNNNHTN | STWPFYAMDY | ITITDIDDDMN | EGNTLRP | LQSDNLPLT |
| | | 251 | 252 | 253 | 254 | 255 | 256 |
| 9 | 28B2A4 | GYTFTDDNMH | YINPNNNHTN | STWPFYAMDY | ITITDIDDDMN | EGNTLRP | LQSDNLPLT |
| | | 285 | 286 | 287 | 288 | 289 | 290 |
| 10 | 28B2F5 | GYTFTDDNMH | YINPNNNHTN | STWPFYAMDY | ITITDIDDDMN | EGNTLRP | LQSDNLPLT |
| | | 319 | 320 | 321 | 322 | 323 | 324 |
| 11 | 28C9A5 | GYTFTHYWID | NIYPGGGYTN | KGYYYGSDWYFDV | SASSSVSYMH | DTSKLAS | QQWSSNPYT |
| | | 353 | 354 | 355 | 356 | 356 | 358 |
| 12 | 28D2E6 | GYFISSGYYWK | YISYDGSNN | GGGYVPV | RSSQSIVRSNGNTYLE | KVSNRFS | FQSSHVPRT |
| | | 387 | 388 | 389 | 390 | 391 | 392 |
| 13 | 28D2E8 | GYFISSGYYWK | YISYDGSNN | GGGYVPV | RSSQSIVRSNGNTYLE | KVSNRFS | FQSSHVPRT |
| | | 421 | 422 | 423 | 424 | 425 | 426 |

**Table 5. Yβ17 Antibody Contact CDR Amino Acid Sequences**

| **#** | **Protein Name** | **HC CONTACT CDR1** | **HC CONTACT CDR2** | **HC CONTACT CDR3** | **LC CONTACT CDR1** | **LC CONTACT CDR2** | **LC CONTACT CDR3** |
|---|---|---|---|---|---|---|---|
| 1 | 20A9A9 | TSYGVH | WLGIIWSAGGTD | ARGSFYDYGRLD | STAVAWY | LLIYSSSYRY | QQHYGTPY |
| | | 19 | 20 | 21 | 22 | 23 | 24 |
| 2 | 20C12A3 | THYWID | WIGNIYPGGGYTN | TRKGYYYGSDWYFD | STAVAWY | LLIYSASYRY | QQHYTTPY |
| | | 53 | 54 | 55 | 56 | 57 | 58 |
| 3 | 21G11A8 | TTYGMN | WMGYLNTYSGVST | ARWGNYGAMD | VRSNGFTYLEWY | LLIYKVSNRF | FQSSHVPR |
| | | 87 | 88 | 89 | 90 | 91 | 92 |
| 4 | 21G11D5 | TTYGMN | WMGYLNTYSGVST | ARWGNYGAMD | VRSNGFTYLEWY | LLIYKVSNRF | FQSSHVPR |
| | | 121 | 122 | 123 | 124 | 125 | 126 |
| 5 | 23 A9B 10 | TSGYYWN | WMGYITYDGSNN | ARGGGYVP | GTSIHWY | LLIKYASESI | HQSHNWPT |
| | | 155 | 156 | 157 | 158 | 159 | 160 |
| 6 | 23A9C10 | TSGYYWN | WMGYITYDGSNN | ARGGGYVP | GTSIHWY | LLIKYASESI | HQSHNWPT |
| | | 189 | 190 | 191 | 192 | 193 | 194 |
| 7 | 23A9F7 | TSGYYWN | WMGYITYDGSNN | ARGGGYVP | GTSIHWY | LLIKYASESI | HQSHNWPT |
| | | 223 | 224 | 225 | 226 | 227 | 228 |
| 8 | 28B2A2 | TDDNMH | WIGYINPNNNHTN | ARSTWPFYAMD | DDDMNWY | LLFSEGNTLR | LQSDNLPL |
| | | 257 | 258 | 259 | 260 | 261 | 262 |
| 9 | 28B2A4 | TDDNMH | WIGYINPNNNHTN | ARSTWPFYAMD | DDDMNWY | LLFSEGNTLR | LQSDNLPL |
| | | 291 | 292 | 293 | 294 | 295 | 296 |
| 10 | 28B2F5 | TDDNMH | WIGYINPNNNHTN | ARSTWPFYAMD | DDDMNWY | LLFSEGNTLR | LQSDNLPL |
| | | 325 | 326 | 327 | 328 | 329 | 330 |
| 11 | 28C9A5 | THYWID | WIGNIYPGGGYTN | TRKGYYYGSDWYFD | SYMHWY | RWIYDTSKLA | QQWSSNPY |
| | | 359 | 360 | 361 | 362 | 363 | 364 |
| 12 | 28D2E6 | SSGYYWK | WMGYISYDGSNN | ARGGGYVP | VRSNGNTYLEWY | LLIYKVSNRF | FQSSHVPR |
| | | 393 | 394 | 395 | 396 | 397 | 398 |
| 13 | 28D2E8 | SSGYYWK | WMGYISYDGSNN | ARGGGYVP | VRSNGNTYLEWY | LLIYKVSNRF | FQSSHVPR |
| | | 427 | 428 | 429 | 430 | 431 | 432 |

**Table 6. Yβ17 Antibody IMGT CDR Amino Acid Sequences**

| **#** | **Protein Name** | **HC IMGT CDR1** | **HC IMGT CDR2** | **HC IMGT CDR3** | **LC IMGT CDR1** | **LC IMGT CDR2** | **LC IMGT CDR3** |
|---|---|---|---|---|---|---|---|
| 1 | 20A9A9 | GFSLTSYG | IWSAGGT | ARGSFYDYGRLDY | QDVSTA | SSS | QQHYGTPYT |
| | | 25 | 26 | 27 | 28 | 29 | 30 |
| 2 | 20C12A3 | GYTFTHYW | IYPGGGYT | TRKGYYYGSDWYFDV | QDVSTA | SAS | QQHYTTPYT |
| | | 59 | 60 | 61 | 62 | 63 | 64 |
| 3 | 21G11A8 | GYTFTTYG | LNTYSGVS | ARWGNYGAMDY | QSIVRSNGFTY | KVS | FQSSHVPRT |
| | | 93 | 94 | 95 | 96 | 97 | 98 |
| 4 | 21G11D5 | GYTFTTYG | LNTYSGVS | ARWGNYGAMDY | QSIVRSNGFTY | KVS | FQSSHVPRT |
| | | 127 | 128 | 129 | 130 | 131 | 132 |
| 5 | 23 A9B 10 | GYSITSGYY | ITYDGSN | ARGGGYVPV | QNIGTS | YAS | HQSHNWPTT |
| | | 161 | 162 | 163 | 164 | 164 | 166 |
| 6 | 23A9C10 | GYSITSGYY | ITYDGSN | ARGGGYVPV | QNIGTS | YAS | HQSHNWPTT |
| | | 195 | 196 | 197 | 198 | 199 | 200 |
| 7 | 23A9F7 | GYSITSGYY | ITYDGSN | ARGGGYVPV | QNIGTS | YAS | HQSHNWPTT |
| | | 229 | 230 | 231 | 232 | 233 | 234 |
| 8 | 28B2A2 | GYTFTDDN | INPNNNHT | ARSTWPFYAMDY | TDIDDD | EGN | LQSDNLPLT |
| | | 263 | 264 | 265 | 266 | 267 | 268 |
| 9 | 28B2A4 | GYTFTDDN | INPNNNHT | ARSTWPFYAMDY | TDIDDD | EGN | LQSDNLPLT |
| | | 297 | 298 | 299 | 300 | 301 | 302 |
| 10 | 28B2F5 | GYTFTDDN | INPNNNHT | ARSTWPFYAMDY | TDIDDD | EGN | LQSDNLPLT |
| | | 331 | 332 | 333 | 334 | 335 | 336 |
| 11 | 28C9A5 | GYTFTHYW | IYPGGGYT | TRKGYYYGSDWYFDV | SSVSY | DTS | QQWSSNPYT |
| | | 365 | 366 | 367 | 368 | 369 | 370 |
| 12 | 28D2E6 | GYFISSGYY | ISYDGSN | ARGGGYVPV | QSIVRSNGNTY | KVS | FQSSHVPRT |
| | | 399 | 400 | 401 | 402 | 403 | 404 |
| 13 | 28D2E8 | GYFISSGYY | ISYDGSN | ARGGGYVPV | QSIVRSNGNTY | KVS | FQSSHVPRT |
| | | 433 | 434 | 435 | 436 | 437 | 438 |

In one aspect, provided herein is an antibody that binds Vβ17, comprising: (i) a VH comprising a VH CDR1, a VH CDR2, and a VH CDR3 having an amino acid sequence of SEQ ID NOs:1, 2, and 3, respectively, and (ii) a VL comprising a VL CDR1, VL CDR2, and VL CDR3 having an amino acid sequence of SEQ ID NOs:4, 5, and 6, respectively. In one aspect, provided herein is an antibody that binds Vβ17, comprising: (i) a VH comprising a VH CDR1, a VH CDR2, and a VH CDR3 having an amino acid sequence of SEQ ID NOs:7, 8, and 9, respectively, and (ii) a VL comprising a VL CDR1, VL CDR2, and VL CDR3 having an amino acid sequence of SEQ ID NOs:10, 11, and 12, respectively. In one aspect, provided herein is an antibody that binds Vβ17, comprising: (i) a VH comprising a VH CDR1, a VH CDR2, and a VH CDR3 having an amino acid sequence of SEQ ID NOs:13, 14, and 15, respectively, and (ii) a VL comprising a VL CDR1, VL CDR2, and VL CDR3 having an amino acid sequence of SEQ ID NOs:16, 17, and 18, respectively. In one aspect, provided herein is an antibody that binds Vβ17, comprising: (i) a VH comprising a VH CDR1, a VH CDR2, and a VH CDR3 having an amino acid sequence of SEQ ID NOs:19, 20, and 21, respectively, and (ii) a VL comprising a VL CDR1, VL CDR2, and VL CDR3 having an amino acid sequence of SEQ ID NOs:22, 23, and 24, respectively. In one aspect, provided herein is an antibody that binds Vβ17, comprising: (i) a VH comprising a VH CDR1, a VH CDR2, and a VH CDR3 having an amino acid sequence of SEQ ID NOs:25, 26, and 27, respectively, and (ii) a VL comprising a VL CDR1, VL CDR2, and VL CDR3 having an amino acid sequence of SEQ ID NOs:28, 29, and 30, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:31; and (ii) a VL comprising a VL CDR1, a VL CDR2, and a VL CDR3 having an amino acid sequence of a VL CDR1, a VL CDR2, and a VL CDR3, respectively, of SEQ ID NO:32. In one aspect, provided herein is an antibody that binds Vβ17, comprising a VH having an amino acid sequence of SEQ ID NO:31. In one aspect, provided herein is an antibody that binds Vβ17, comprising a VL having an amino acid sequence of SEQ ID NO:32. In one aspect, provided herein is an antibody that binds Vβ17, comprising a VH having an amino acid sequence of SEQ ID NO:31, and a VL having an amino acid sequence of SEQ ID NO:32. In one aspect, provided herein is an antibody that binds Vβ17, comprising a VH having an amino acid sequence having at least 95% identity to an amino acid sequence of SEQ ID NO:31. In one aspect, provided herein is an antibody that binds Vβ17, comprising a VL having an amino acid sequence having at least 95% identity to an amino acid sequence of SEQ ID NO:32. In one aspect, provided herein is an antibody that binds Vβ17, comprising a VH having an amino acid sequence having at least 95% identity to an amino acid sequence of SEQ ID NO:31, and a VL having an amino acid sequence having at least 95% identity to an amino acid sequence of SEQ ID NO:32.

In one aspect, provided herein is an antibody that binds Vβ17, comprising: (i) a VH comprising a VH CDR1, a VH CDR2, and a VH CDR3 having an amino acid sequence of SEQ ID NOs:35, 36, and 37, respectively, and (ii) a VL comprising a VL CDR1, VL CDR2, and VL CDR3 having an amino acid sequence of SEQ ID NOs:38, 39, and 40, respectively. In one aspect, provided herein is an antibody that binds Vβ17, comprising: (i) a VH comprising a VH CDR1, a VH CDR2, and a VH CDR3 having an amino acid sequence of SEQ ID NOs:41, 42, and 43, respectively, and (ii) a VL comprising a VL CDR1, VL CDR2, and VL CDR3 having an amino acid sequence of SEQ ID NOs:44, 45, and 46, respectively. In one aspect, provided herein is an antibody that binds Vβ17, comprising: (i) a VH comprising a VH CDR1, a VH CDR2, and a VH CDR3 having an amino acid sequence of SEQ ID NOs:47, 48, and 49, respectively, and (ii) a VL comprising a VL CDR1, VL CDR2, and VL CDR3 having an amino acid sequence of SEQ ID NOs:50, 51, and 52, respectively. In one aspect, provided herein is an antibody that binds Vβ17, comprising: (i) a VH comprising a VH CDR1, a VH CDR2, and a VH CDR3 having an amino acid sequence of SEQ ID NOs:53, 54, and 55, respectively, and (ii) a VL comprising a VL CDR1, VL CDR2, and VL CDR3 having an amino acid sequence of SEQ ID NOs:56, 57, and 58, respectively. In one aspect, provided herein is an antibody that binds Vβ17, comprising: (i) a VH comprising a VH CDR1, a VH CDR2, and a VH CDR3 having an amino acid sequence of SEQ ID NOs:59, 60, and 61, respectively, and (ii) a VL comprising a VL CDR1, VL CDR2, and VL CDR3 having an amino acid sequence of SEQ ID NOs:62, 63, and 64, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:65; and (ii) a VL comprising a VL CDR1, a VL CDR2, and a VL CDR3 having an amino acid sequence of a VL CDR1, a VL CDR2, and a VL CDR3, respectively, of SEQ ID NO:66. In one aspect, provided herein is an antibody that binds Vβ17, comprising a VH having an amino acid sequence of SEQ ID NO:65. In one aspect, provided herein is an antibody that binds Vβ17, comprising a VL having an amino acid sequence of SEQ ID NO:66. In one aspect, provided herein is an antibody that binds Vβ17, comprising a VH having an amino acid sequence of SEQ ID NO:65, and a VL having an amino acid sequence of SEQ ID NO:66. In one aspect, provided herein is an antibody that binds Vβ17, comprising a VH having an amino acid sequence having at least 95% identity to an amino acid sequence of SEQ ID NO:65. In one aspect, provided herein is an antibody that binds Vβ17, comprising a VL having an amino acid sequence having at least 95% identity to an amino acid sequence of SEQ ID NO:66. In one aspect, provided herein is an antibody that binds Vβ17, comprising a VH having an amino acid sequence having at least 95% identity to an amino acid sequence of SEQ ID NO:65, and a VL having an amino acid sequence having at least 95% identity to an amino acid sequence of SEQ ID NO:66.

In one aspect, provided herein is an antibody that binds Vβ17, comprising: (i) a VH comprising a VH CDR1, a VH CDR2, and a VH CDR3 having an amino acid sequence of SEQ ID NOs:69, 70, and 71, respectively, and (ii) a VL comprising a VL CDR1, VL CDR2, and VL CDR3 having an amino acid sequence of SEQ ID NOs:72, 73, and 74, respectively. In one aspect, provided herein is an antibody that binds Vβ17, comprising: (i) a VH comprising a VH CDR1, a VH CDR2, and a VH CDR3 having an amino acid sequence of SEQ ID NOs:75, 76, and 77, respectively, and (ii) a VL comprising a VL CDR1, VL CDR2, and VL CDR3 having an amino acid sequence of SEQ ID NOs:78, 79, and 80, respectively. In one aspect, provided herein is an antibody that binds Vβ17, comprising: (i) a VH comprising a VH CDR1, a VH CDR2, and a VH CDR3 having an amino acid sequence of SEQ ID NOs:81, 82, and 83, respectively, and (ii) a VL comprising a VL CDR1, VL CDR2, and VL CDR3 having an amino acid sequence of SEQ ID NOs:84, 85, and 86, respectively. In one aspect, provided herein is an antibody that binds Vβ17, comprising: (i) a VH comprising a VH CDR1, a VH CDR2, and a VH CDR3 having an amino acid sequence of SEQ ID NOs:87, 88, and 89, respectively, and (ii) a VL comprising a VL CDR1, VL CDR2, and VL CDR3 having an amino acid sequence of SEQ ID NOs:90, 91, and 92, respectively. In one aspect, provided herein is an antibody that binds Vβ17, comprising: (i) a VH comprising a VH CDR1, a VH CDR2, and a VH CDR3 having an amino acid sequence of SEQ ID NOs:93, 94, and 95, respectively, and (ii) a VL comprising a VL CDR1, VL CDR2, and VL CDR3 having an amino acid sequence of SEQ ID NOs:96, 97, and 98, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:99; and (ii) a VL comprising a VL CDR1, a VL CDR2, and a VL CDR3 having an amino acid sequence of a VL CDR1, a VL CDR2, and a VL CDR3, respectively, of SEQ ID NO:100. In one aspect, provided herein is an antibody that binds Vβ17, comprising a VH having an amino acid sequence of SEQ ID NO:99. In one aspect, provided herein is an antibody that binds Vβ17, comprising a VL having an amino acid sequence of SEQ ID NO:100. In one aspect, provided herein is an antibody that binds Vβ17, comprising a VH having an amino acid sequence of SEQ ID NO:99, and a VL having an amino acid sequence of SEQ ID NO:100. In one aspect, provided herein is an antibody that binds Vβ17, comprising a VH having an amino acid sequence having at least 95% identity to an amino acid sequence of SEQ ID NO:99. In one aspect, provided herein is an antibody that binds Vβ17, comprising a VL having an amino acid sequence having at least 95% identity to an amino acid sequence of SEQ ID NO:100. In one aspect, provided herein is an antibody that binds Vβ17, comprising a VH having an amino acid sequence having at least 95% identity to an amino acid sequence of SEQ ID NO:99, and a VL having an amino acid sequence having at least 95% identity to an amino acid sequence of SEQ ID NO:100.

In one aspect, provided herein is an antibody that binds Vβ17, comprising: (i) a VH comprising a VH CDR1, a VH CDR2, and a VH CDR3 having an amino acid sequence of SEQ ID NOs:103, 104, and 105, respectively, and (ii) a VL comprising a VL CDR1, VL CDR2, and VL CDR3 having an amino acid sequence of SEQ ID NOs:106, 107, and 108, respectively. In one aspect, provided herein is an antibody that binds Vβ17, comprising: (i) a VH comprising a VH CDR1, a VH CDR2, and a VH CDR3 having an amino acid sequence of SEQ ID NOs:109, 110, and 111, respectively, and (ii) a VL comprising a VL CDR1, VL CDR2, and VL CDR3 having an amino acid sequence of SEQ ID NOs:112, 113, and 114, respectively. In one aspect, provided herein is an antibody that binds Vβ17, comprising: (i) a VH comprising a VH CDR1, a VH CDR2, and a VH CDR3 having an amino acid sequence of SEQ ID NOs:115, 116, and 117, respectively, and (ii) a VL comprising a VL CDR1, VL CDR2, and VL CDR3 having an amino acid sequence of SEQ ID NOs:118, 119, and 120, respectively. In one aspect, provided herein is an antibody that binds Vβ17, comprising: (i) a VH comprising a VH CDR1, a VH CDR2, and a VH CDR3 having an amino acid sequence of SEQ ID NOs:121, 122, and 123, respectively, and (ii) a VL comprising a VL CDR1, VL CDR2, and VL CDR3 having an amino acid sequence of SEQ ID NOs: 124, 125, and 126, respectively. In one aspect, provided herein is an antibody that binds Vβ17, comprising: (i) a VH comprising a VH CDR1, a VH CDR2, and a VH CDR3 having an amino acid sequence of SEQ ID NOs:127, 128, and 129, respectively, and (ii) a VL comprising a VL CDR1, VL CDR2, and VL CDR3 having an amino acid sequence of SEQ ID NOs: 130, 131, and 132, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO: 133; and (ii) a VL comprising a VL CDR1, a VL CDR2, and a VL CDR3 having an amino acid sequence of a VL CDR1, a VL CDR2, and a VL CDR3, respectively, of SEQ ID NO: 134. In one aspect, provided herein is an antibody that binds Vβ17, comprising a VH having an amino acid sequence of SEQ ID NO: 133. In one aspect, provided herein is an antibody that binds Vβ17, comprising a VL having an amino acid sequence of SEQ ID NO: 134. In one aspect, provided herein is an antibody that binds Vβ17, comprising a VH having an amino acid sequence of SEQ ID NO: 133, and a VL having an amino acid sequence of SEQ ID NO: 134. In one aspect, provided herein is an antibody that binds Vβ17, comprising a VH having an amino acid sequence having at least 95% identity to an amino acid sequence of SEQ ID NO: 133. In one aspect, provided herein is an antibody that binds Vβ17, comprising a VL having an amino acid sequence having at least 95% identity to an amino acid sequence of SEQ ID NO: 134. In one aspect, provided herein is an antibody that binds Vβ17, comprising a VH having an amino acid sequence having at least 95% identity to an amino acid sequence of SEQ ID NO: 133, and a VL having an amino acid sequence having at least 95% identity to an amino acid sequence of SEQ ID NO: 134.

In one aspect, provided herein is an antibody that binds Vβ17, comprising: (i) a VH comprising a VH CDR1, a VH CDR2, and a VH CDR3 having an amino acid sequence of SEQ ID NOs:137, 138, and 139, respectively, and (ii) a VL comprising a VL CDR1, VL CDR2, and VL CDR3 having an amino acid sequence of SEQ ID NOs: 140, 141, and 142, respectively. In one aspect, provided herein is an antibody that binds Vβ17, comprising: (i) a VH comprising a VH CDR1, a VH CDR2, and a VH CDR3 having an amino acid sequence of SEQ ID NOs: 143, 144, and 145, respectively, and (ii) a VL comprising a VL CDR1, VL CDR2, and VL CDR3 having an amino acid sequence of SEQ ID NOs: 146, 147, and 148, respectively. In one aspect, provided herein is an antibody that binds Vβ17, comprising: (i) a VH comprising a VH CDR1, a VH CDR2, and a VH CDR3 having an amino acid sequence of SEQ ID NOs:149, 150, and 151, respectively, and (ii) a VL comprising a VL CDR1, VL CDR2, and VL CDR3 having an amino acid sequence of SEQ ID NOs: 152, 153, and 154, respectively. In one aspect, provided herein is an antibody that binds Vβ17, comprising: (i) a VH comprising a VH CDR1, a VH CDR2, and a VH CDR3 having an amino acid sequence of SEQ ID NOs: 155, 156, and 157, respectively, and (ii) a VL comprising a VL CDR1, VL CDR2, and VL CDR3 having an amino acid sequence of SEQ ID NOs: 158, 159, and 160, respectively. In one aspect, provided herein is an antibody that binds Vβ17, comprising: (i) a VH comprising a VH CDR1, a VH CDR2, and a VH CDR3 having an amino acid sequence of SEQ ID NOs: 161, 162, and 163, respectively, and (ii) a VL comprising a VL CDR1, VL CDR2, and VL CDR3 having an amino acid sequence of SEQ ID NOs: 164, 165, and 166, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO: 167; and (ii) a VL comprising a VL CDR1, a VL CDR2, and a VL CDR3 having an amino acid sequence of a VL CDR1, a VL CDR2, and a VL CDR3, respectively, of SEQ ID NO: 168. In one aspect, provided herein is an antibody that binds Vβ17, comprising a VH having an amino acid sequence of SEQ ID NO: 167. In one aspect, provided herein is an antibody that binds Vβ17, comprising a VL having an amino acid sequence of SEQ ID NO: 168. In one aspect, provided herein is an antibody that binds Vβ17, comprising a VH having an amino acid sequence of SEQ ID NO: 167, and a VL having an amino acid sequence of SEQ ID NO: 168. In one aspect, provided herein is an antibody that binds Vβ17, comprising a VH having an amino acid sequence having at least 95% identity to an amino acid sequence of SEQ ID NO: 167. In one aspect, provided herein is an antibody that binds Vβ17, comprising a VL having an amino acid sequence having at least 95% identity to an amino acid sequence of SEQ ID NO: 168. In one aspect, provided herein is an antibody that binds Vβ17, comprising a VH having an amino acid sequence having at least 95% identity to an amino acid sequence of SEQ ID NO: 167, and a VL having an amino acid sequence having at least 95% identity to an amino acid sequence of SEQ ID NO: 168.

In one aspect, provided herein is an antibody that binds Vβ17, comprising: (i) a VH comprising a VH CDR1, a VH CDR2, and a VH CDR3 having an amino acid sequence of SEQ ID NOs: 171, 172, and 173, respectively, and (ii) a VL comprising a VL CDR1, VL CDR2, and VL CDR3 having an amino acid sequence of SEQ ID NOs: 174, 175, and 176, respectively. In one aspect, provided herein is an antibody that binds Vβ17, comprising: (i) a VH comprising a VH CDR1, a VH CDR2, and a VH CDR3 having an amino acid sequence of SEQ ID NOs: 177, 178, and 179, respectively, and (ii) a VL comprising a VL CDR1, VL CDR2, and VL CDR3 having an amino acid sequence of SEQ ID NOs: 180, 181, and 182, respectively. In one aspect, provided herein is an antibody that binds Vβ17, comprising: (i) a VH comprising a VH CDR1, a VH CDR2, and a VH CDR3 having an amino acid sequence of SEQ ID NOs: 183, 184, and 185, respectively, and (ii) a VL comprising a VL CDR1, VL CDR2, and VL CDR3 having an amino acid sequence of SEQ ID NOs: 186, 187, and 188, respectively. In one aspect, provided herein is an antibody that binds Vβ17, comprising: (i) a VH comprising a VH CDR1, a VH CDR2, and a VH CDR3 having an amino acid sequence of SEQ ID NOs: 189, 190, and 191, respectively, and (ii) a VL comprising a VL CDR1, VL CDR2, and VL CDR3 having an amino acid sequence of SEQ ID NOs: 192, 193, and 194, respectively. In one aspect, provided herein is an antibody that binds Vβ17, comprising: (i) a VH comprising a VH CDR1, a VH CDR2, and a VH CDR3 having an amino acid sequence of SEQ ID NOs: 195, 196, and 197, respectively, and (ii) a VL comprising a VL CDR1, VL CDR2, and VL CDR3 having an amino acid sequence of SEQ ID NOs: 198, 199, and 200, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:201; and (ii) a VL comprising a VL CDR1, a VL CDR2, and a VL CDR3 having an amino acid sequence of a VL CDR1, a VL CDR2, and a VL CDR3, respectively, of SEQ ID NO:202. In one aspect, provided herein is an antibody that binds Vβ17, comprising a VH having an amino acid sequence of SEQ ID NO:201. In one aspect, provided herein is an antibody that binds Vβ17, comprising a VL having an amino acid sequence of SEQ ID NO:202. In one aspect, provided herein is an antibody that binds Vβ17, comprising a VH having an amino acid sequence of SEQ ID NO:201, and a VL having an amino acid sequence of SEQ ID NO:202. In one aspect, provided herein is an antibody that binds Vβ17, comprising a VH having an amino acid sequence having at least 95% identity to an amino acid sequence of SEQ ID NO:201. In one aspect, provided herein is an antibody that binds Vβ17, comprising a VL having an amino acid sequence having at least 95% identity to an amino acid sequence of SEQ ID NO:202. In one aspect, provided herein is an antibody that binds Vβ17, comprising a VH having an amino acid sequence having at least 95% identity to an amino acid sequence of SEQ ID NO:201, and a VL having an amino acid sequence having at least 95% identity to an amino acid sequence of SEQ ID NO:202.

In one aspect, provided herein is an antibody that binds Vβ17, comprising: (i) a VH comprising a VH CDR1, a VH CDR2, and a VH CDR3 having an amino acid sequence of SEQ ID NOs:205, 206, and 207, respectively, and (ii) a VL comprising a VL CDR1, VL CDR2, and VL CDR3 having an amino acid sequence of SEQ ID NOs:208, 209, and 210, respectively. In one aspect, provided herein is an antibody that binds Vβ17, comprising: (i) a VH comprising a VH CDR1, a VH CDR2, and a VH CDR3 having an amino acid sequence of SEQ ID NOs:211, 212, and 213, respectively, and (ii) a VL comprising a VL CDR1, VL CDR2, and VL CDR3 having an amino acid sequence of SEQ ID NOs:214, 215, and 216, respectively. In one aspect, provided herein is an antibody that binds Vβ17, comprising: (i) a VH comprising a VH CDR1, a VH CDR2, and a VH CDR3 having an amino acid sequence of SEQ ID NOs:217, 218, and 219, respectively, and (ii) a VL comprising a VL CDR1, VL CDR2, and VL CDR3 having an amino acid sequence of SEQ ID NOs:220, 221, and 222, respectively. In one aspect, provided herein is an antibody that binds Vβ17, comprising: (i) a VH comprising a VH CDR1, a VH CDR2, and a VH CDR3 having an amino acid sequence of SEQ ID NOs:223, 224, and 225, respectively, and (ii) a VL comprising a VL CDR1, VL CDR2, and VL CDR3 having an amino acid sequence of SEQ ID NOs:226, 227, and 228, respectively. In one aspect, provided herein is an antibody that binds Vβ17, comprising: (i) a VH comprising a VH CDR1, a VH CDR2, and a VH CDR3 having an amino acid sequence of SEQ ID NOs:229, 230, and 231, respectively, and (ii) a VL comprising a VL CDR1, VL CDR2, and VL CDR3 having an amino acid sequence of SEQ ID NOs:232, 233, and 234, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:235; and (ii) a VL comprising a VL CDR1, a VL CDR2, and a VL CDR3 having an amino acid sequence of a VL CDR1, a VL CDR2, and a VL CDR3, respectively, of SEQ ID NO:236. In one aspect, provided herein is an antibody that binds Vβ17, comprising a VH having an amino acid sequence of SEQ ID NO:235. In one aspect, provided herein is an antibody that binds Vβ17, comprising a VL having an amino acid sequence of SEQ ID NO:236. In one aspect, provided herein is an antibody that binds Vβ17, comprising a VH having an amino acid sequence of SEQ ID NO:235, and a VL having an amino acid sequence of SEQ ID NO:236. In one aspect, provided herein is an antibody that binds Vβ17, comprising a VH having an amino acid sequence having at least 95% identity to an amino acid sequence of SEQ ID NO:235. In one aspect, provided herein is an antibody that binds Vβ17, comprising a VL having an amino acid sequence having at least 95% identity to an amino acid sequence of SEQ ID NO:236. In one aspect, provided herein is an antibody that binds Vβ17, comprising a VH having an amino acid sequence having at least 95% identity to an amino acid sequence of SEQ ID NO:235, and a VL having an amino acid sequence having at least 95% identity to an amino acid sequence of SEQ ID NO:236.

In one aspect, provided herein is an antibody that binds Vβ17, comprising: (i) a VH comprising a VH CDR1, a VH CDR2, and a VH CDR3 having an amino acid sequence of SEQ ID NOs:239, 240, and 241, respectively, and (ii) a VL comprising a VL CDR1, VL CDR2, and VL CDR3 having an amino acid sequence of SEQ ID NOs:242, 243, and 244, respectively. In one aspect, provided herein is an antibody that binds Vβ17, comprising: (i) a VH comprising a VH CDR1, a VH CDR2, and a VH CDR3 having an amino acid sequence of SEQ ID NOs:245, 246, and 247, respectively, and (ii) a VL comprising a VL CDR1, VL CDR2, and VL CDR3 having an amino acid sequence of SEQ ID NOs:248, 249, and 250, respectively. In one aspect, provided herein is an antibody that binds Vβ17, comprising: (i) a VH comprising a VH CDR1, a VH CDR2, and a VH CDR3 having an amino acid sequence of SEQ ID NOs:251, 252, and 253, respectively, and (ii) a VL comprising a VL CDR1, VL CDR2, and VL CDR3 having an amino acid sequence of SEQ ID NOs:254, 255, and 256, respectively. In one aspect, provided herein is an antibody that binds Vβ17, comprising: (i) a VH comprising a VH CDR1, a VH CDR2, and a VH CDR3 having an amino acid sequence of SEQ ID NOs:257, 258, and 259, respectively, and (ii) a VL comprising a VL CDR1, VL CDR2, and VL CDR3 having an amino acid sequence of SEQ ID NOs:260, 261, and 262, respectively. In one aspect, provided herein is an antibody that binds Vβ17, comprising: (i) a VH comprising a VH CDR1, a VH CDR2, and a VH CDR3 having an amino acid sequence of SEQ ID NOs:263, 264, and 265, respectively, and (ii) a VL comprising a VL CDR1, VL CDR2, and VL CDR3 having an amino acid sequence of SEQ ID NOs:266, 267, and 268, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:269; and (ii) a VL comprising a VL CDR1, a VL CDR2, and a VL CDR3 having an amino acid sequence of a VL CDR1, a VL CDR2, and a VL CDR3, respectively, of SEQ ID NO:270. In one aspect, provided herein is an antibody that binds Vβ17, comprising a VH having an amino acid sequence of SEQ ID NO:269. In one aspect, provided herein is an antibody that binds Vβ17, comprising a VL having an amino acid sequence of SEQ ID NO:270. In one aspect, provided herein is an antibody that binds Vβ17, comprising a VH having an amino acid sequence of SEQ ID NO:269, and a VL having an amino acid sequence of SEQ ID NO:270. In one aspect, provided herein is an antibody that binds Vβ17, comprising a VH having an amino acid sequence having at least 95% identity to an amino acid sequence of SEQ ID NO:269. In one aspect, provided herein is an antibody that binds Vβ17, comprising a VL having an amino acid sequence having at least 95% identity to an amino acid sequence of SEQ ID NO:270. In one aspect, provided herein is an antibody that binds Vβ17, comprising a VH having an amino acid sequence having at least 95% identity to an amino acid sequence of SEQ ID NO:269, and a VL having an amino acid sequence having at least 95% identity to an amino acid sequence of SEQ ID NO:270.

In one aspect, provided herein is an antibody that binds Vβ17, comprising: (i) a VH comprising a VH CDR1, a VH CDR2, and a VH CDR3 having an amino acid sequence of SEQ ID NOs:273, 274, and 275, respectively, and (ii) a VL comprising a VL CDR1, VL CDR2, and VL CDR3 having an amino acid sequence of SEQ ID NOs:276, 277, and 278, respectively. In one aspect, provided herein is an antibody that binds Vβ17, comprising: (i) a VH comprising a VH CDR1, a VH CDR2, and a VH CDR3 having an amino acid sequence of SEQ ID NOs:279, 280, and 281, respectively, and (ii) a VL comprising a VL CDR1, VL CDR2, and VL CDR3 having an amino acid sequence of SEQ ID NOs:282, 283, and 284, respectively. In one aspect, provided herein is an antibody that binds Vβ17, comprising: (i) a VH comprising a VH CDR1, a VH CDR2, and a VH CDR3 having an amino acid sequence of SEQ ID NOs:285, 286, and 287, respectively, and (ii) a VL comprising a VL CDR1, VL CDR2, and VL CDR3 having an amino acid sequence of SEQ ID NOs:288, 289, and 290, respectively. In one aspect, provided herein is an antibody that binds Vβ17, comprising: (i) a VH comprising a VH CDR1, a VH CDR2, and a VH CDR3 having an amino acid sequence of SEQ ID NOs:291, 292, and 293, respectively, and (ii) a VL comprising a VL CDR1, VL CDR2, and VL CDR3 having an amino acid sequence of SEQ ID NOs:294, 295, and 296, respectively. In one aspect, provided herein is an antibody that binds Vβ17, comprising: (i) a VH comprising a VH CDR1, a VH CDR2, and a VH CDR3 having an amino acid sequence of SEQ ID NOs:297, 298, and 299, respectively, and (ii) a VL comprising a VL CDR1, VL CDR2, and VL CDR3 having an amino acid sequence of SEQ ID NOs:300, 301, and 302, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:303; and (ii) a VL comprising a VL CDR1, a VL CDR2, and a VL CDR3 having an amino acid sequence of a VL CDR1, a VL CDR2, and a VL CDR3, respectively, of SEQ ID NO:304. In one aspect, provided herein is an antibody that binds Vβ17, comprising a VH having an amino acid sequence of SEQ ID NO:303. In one aspect, provided herein is an antibody that binds Vβ17, comprising a VL having an amino acid sequence of SEQ ID NO:304. In one aspect, provided herein is an antibody that binds Vβ17, comprising a VH having an amino acid sequence of SEQ ID NO:303, and a VL having an amino acid sequence of SEQ ID NO:304. In one aspect, provided herein is an antibody that binds Vβ17, comprising a VH having an amino acid sequence having at least 95% identity to an amino acid sequence of SEQ ID NO:303. In one aspect, provided herein is an antibody that binds Vβ17, comprising a VL having an amino acid sequence having at least 95% identity to an amino acid sequence of SEQ ID NO:304. In one aspect, provided herein is an antibody that binds Vβ17, comprising a VH having an amino acid sequence having at least 95% identity to an amino acid sequence of SEQ ID NO:303, and a VL having an amino acid sequence having at least 95% identity to an amino acid sequence of SEQ ID NO:304.

In one aspect, provided herein is an antibody that binds Vβ17, comprising: (i) a VH comprising a VH CDR1, a VH CDR2, and a VH CDR3 having an amino acid sequence of SEQ ID NOs:307, 308, and 309, respectively, and (ii) a VL comprising a VL CDR1, VL CDR2, and VL CDR3 having an amino acid sequence of SEQ ID NOs:310, 311, and 312, respectively. In one aspect, provided herein is an antibody that binds Vβ17, comprising: (i) a VH comprising a VH CDR1, a VH CDR2, and a VH CDR3 having an amino acid sequence of SEQ ID NOs:313, 314, and 315, respectively, and (ii) a VL comprising a VL CDR1, VL CDR2, and VL CDR3 having an amino acid sequence of SEQ ID NOs:316, 317, and 318, respectively. In one aspect, provided herein is an antibody that binds Vβ17, comprising: (i) a VH comprising a VH CDR1, a VH CDR2, and a VH CDR3 having an amino acid sequence of SEQ ID NOs:319, 320, and 321, respectively, and (ii) a VL comprising a VL CDR1, VL CDR2, and VL CDR3 having an amino acid sequence of SEQ ID NOs:322, 323, and 324, respectively. In one aspect, provided herein is an antibody that binds Vβ17, comprising: (i) a VH comprising a VH CDR1, a VH CDR2, and a VH CDR3 having an amino acid sequence of SEQ ID NOs:325, 326, and 327, respectively, and (ii) a VL comprising a VL CDR1, VL CDR2, and VL CDR3 having an amino acid sequence of SEQ ID NOs:328, 329, and 330, respectively. In one aspect, provided herein is an antibody that binds Vβ17, comprising: (i) a VH comprising a VH CDR1, a VH CDR2, and a VH CDR3 having an amino acid sequence of SEQ ID NOs:331, 332, and 333, respectively, and (ii) a VL comprising a VL CDR1, VL CDR2, and VL CDR3 having an amino acid sequence of SEQ ID NOs:334, 335, and 336, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:337; and (ii) a VL comprising a VL CDR1, a VL CDR2, and a VL CDR3 having an amino acid sequence of a VL CDR1, a VL CDR2, and a VL CDR3, respectively, of SEQ ID NO:338. In one aspect, provided herein is an antibody that binds Vβ17, comprising a VH having an amino acid sequence of SEQ ID NO:337. In one aspect, provided herein is an antibody that binds Vβ17, comprising a VL having an amino acid sequence of SEQ ID NO:338. In one aspect, provided herein is an antibody that binds Vβ17, comprising a VH having an amino acid sequence of SEQ ID NO:337, and a VL having an amino acid sequence of SEQ ID NO:338. In one aspect, provided herein is an antibody that binds Vβ17, comprising a VH having an amino acid sequence having at least 95% identity to an amino acid sequence of SEQ ID NO:337. In one aspect, provided herein is an antibody that binds Vβ17, comprising a VL having an amino acid sequence having at least 95% identity to an amino acid sequence of SEQ ID NO:338. In one aspect, provided herein is an antibody that binds Vβ17, comprising a VH having an amino acid sequence having at least 95% identity to an amino acid sequence of SEQ ID NO:337, and a VL having an amino acid sequence having at least 95% identity to an amino acid sequence of SEQ ID NO:338.

In one aspect, provided herein is an antibody that binds Vβ17, comprising: (i) a VH comprising a VH CDR1, a VH CDR2, and a VH CDR3 having an amino acid sequence of SEQ ID NOs:341, 342, and 343, respectively, and (ii) a VL comprising a VL CDR1, VL CDR2, and VL CDR3 having an amino acid sequence of SEQ ID NOs:344, 345, and 346, respectively. In one aspect, provided herein is an antibody that binds Vβ17, comprising: (i) a VH comprising a VH CDR1, a VH CDR2, and a VH CDR3 having an amino acid sequence of SEQ ID NOs:347, 348, and 349, respectively, and (ii) a VL comprising a VL CDR1, VL CDR2, and VL CDR3 having an amino acid sequence of SEQ ID NOs:350, 351, and 352, respectively. In one aspect, provided herein is an antibody that binds Vβ17, comprising: (i) a VH comprising a VH CDR1, a VH CDR2, and a VH CDR3 having an amino acid sequence of SEQ ID NOs:353, 354, and 355, respectively, and (ii) a VL comprising a VL CDR1, VL CDR2, and VL CDR3 having an amino acid sequence of SEQ ID NOs:356, 357, and 358, respectively. In one aspect, provided herein is an antibody that binds Vβ17, comprising: (i) a VH comprising a VH CDR1, a VH CDR2, and a VH CDR3 having an amino acid sequence of SEQ ID NOs:359, 360, and 361, respectively, and (ii) a VL comprising a VL CDR1, VL CDR2, and VL CDR3 having an amino acid sequence of SEQ ID NOs:362, 363, and 364, respectively. In one aspect, provided herein is an antibody that binds Vβ17, comprising: (i) a VH comprising a VH CDR1, a VH CDR2, and a VH CDR3 having an amino acid sequence of SEQ ID NOs:365, 366, and 367, respectively, and (ii) a VL comprising a VL CDR1, VL CDR2, and VL CDR3 having an amino acid sequence of SEQ ID NOs:368, 369, and 370, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:371; and (ii) a VL comprising a VL CDR1, a VL CDR2, and a VL CDR3 having an amino acid sequence of a VL CDR1, a VL CDR2, and a VL CDR3, respectively, of SEQ ID NO:372. In one aspect, provided herein is an antibody that binds Vβ17, comprising a VH having an amino acid sequence of SEQ ID NO:371. In one aspect, provided herein is an antibody that binds Vβ17, comprising a VL having an amino acid sequence of SEQ ID NO:372. In one aspect, provided herein is an antibody that binds Vβ17, comprising a VH having an amino acid sequence of SEQ ID NO:371, and a VL having an amino acid sequence of SEQ ID NO:372. In one aspect, provided herein is an antibody that binds Vβ17, comprising a VH having an amino acid sequence having at least 95% identity to an amino acid sequence of SEQ ID NO:371. In one aspect, provided herein is an antibody that binds Vβ17, comprising a VL having an amino acid sequence having at least 95% identity to an amino acid sequence of SEQ ID NO:372. In one aspect, provided herein is an antibody that binds Vβ17, comprising a VH having an amino acid sequence having at least 95% identity to an amino acid sequence of SEQ ID NO:371, and a VL having an amino acid sequence having at least 95% identity to an amino acid sequence of SEQ ID NO:372.

In one aspect, provided herein is an antibody that binds Vβ17, comprising: (i) a VH comprising a VH CDR1, a VH CDR2, and a VH CDR3 having an amino acid sequence of SEQ ID NOs:375, 376, and 377, respectively, and (ii) a VL comprising a VL CDR1, VL CDR2, and VL CDR3 having an amino acid sequence of SEQ ID NOs:378, 379, and 380, respectively. In one aspect, provided herein is an antibody that binds Vβ17, comprising: (i) a VH comprising a VH CDR1, a VH CDR2, and a VH CDR3 having an amino acid sequence of SEQ ID NOs:381, 382, and 383, respectively, and (ii) a VL comprising a VL CDR1, VL CDR2, and VL CDR3 having an amino acid sequence of SEQ ID NOs:384, 385, and 386, respectively. In one aspect, provided herein is an antibody that binds Vβ17, comprising: (i) a VH comprising a VH CDR1, a VH CDR2, and a VH CDR3 having an amino acid sequence of SEQ ID NOs:387, 388, and 389, respectively, and (ii) a VL comprising a VL CDR1, VL CDR2, and VL CDR3 having an amino acid sequence of SEQ ID NOs:390, 391, and 392, respectively. In one aspect, provided herein is an antibody that binds Vβ17, comprising: (i) a VH comprising a VH CDR1, a VH CDR2, and a VH CDR3 having an amino acid sequence of SEQ ID NOs:393, 394, and 395, respectively, and (ii) a VL comprising a VL CDR1, VL CDR2, and VL CDR3 having an amino acid sequence of SEQ ID NOs:396, 397, and 398, respectively. In one aspect, provided herein is an antibody that binds Vβ17, comprising: (i) a VH comprising a VH CDR1, a VH CDR2, and a VH CDR3 having an amino acid sequence of SEQ ID NOs:399, 400, and 401, respectively, and (ii) a VL comprising a VL CDR1, VL CDR2, and VL CDR3 having an amino acid sequence of SEQ ID NOs:402, 403, and 404, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:405; and (ii) a VL comprising a VL CDR1, a VL CDR2, and a VL CDR3 having an amino acid sequence of a VL CDR1, a VL CDR2, and a VL CDR3, respectively, of SEQ ID NO:406. In one aspect, provided herein is an antibody that binds Vβ17, comprising a VH having an amino acid sequence of SEQ ID NO:405. In one aspect, provided herein is an antibody that binds Vβ17, comprising a VL having an amino acid sequence of SEQ ID NO:406. In one aspect, provided herein is an antibody that binds Vβ17, comprising a VH having an amino acid sequence of SEQ ID NO:405, and a VL having an amino acid sequence of SEQ ID NO:406. In one aspect, provided herein is an antibody that binds Vβ17, comprising a VH having an amino acid sequence having at least 95% identity to an amino acid sequence of SEQ ID NO:405. In one aspect, provided herein is an antibody that binds Vβ17, comprising a VL having an amino acid sequence having at least 95% identity to an amino acid sequence of SEQ ID NO:406. In one aspect, provided herein is an antibody that binds Vβ17, comprising a VH having an amino acid sequence having at least 95% identity to an amino acid sequence of SEQ ID NO:405, and a VL having an amino acid sequence having at least 95% identity to an amino acid sequence of SEQ ID NO:406.

In one aspect, provided herein is an antibody that binds Vβ17, comprising: (i) a VH comprising a VH CDR1, a VH CDR2, and a VH CDR3 having an amino acid sequence of SEQ ID NOs:409, 410, and 411, respectively, and (ii) a VL comprising a VL CDR1, VL CDR2, and VL CDR3 having an amino acid sequence of SEQ ID NOs:412, 413, and 414, respectively. In one aspect, provided herein is an antibody that binds Vβ17, comprising: (i) a VH comprising a VH CDR1, a VH CDR2, and a VH CDR3 having an amino acid sequence of SEQ ID NOs:415, 416, and 417, respectively, and (ii) a VL comprising a VL CDR1, VL CDR2, and VL CDR3 having an amino acid sequence of SEQ ID NOs:418, 419, and 420, respectively. In one aspect, provided herein is an antibody that binds Vβ17, comprising: (i) a VH comprising a VH CDR1, a VH CDR2, and a VH CDR3 having an amino acid sequence of SEQ ID NOs:421, 422, and 423, respectively, and (ii) a VL comprising a VL CDR1, VL CDR2, and VL CDR3 having an amino acid sequence of SEQ ID NOs:424, 425, and 426, respectively. In one aspect, provided herein is an antibody that binds Vβ17, comprising: (i) a VH comprising a VH CDR1, a VH CDR2, and a VH CDR3 having an amino acid sequence of SEQ ID NOs:427, 428, and 429, respectively, and (ii) a VL comprising a VL CDR1, VL CDR2, and VL CDR3 having an amino acid sequence of SEQ ID NOs:430, 431, and 432, respectively. In one aspect, provided herein is an antibody that binds Vβ17, comprising: (i) a VH comprising a VH CDR1, a VH CDR2, and a VH CDR3 having an amino acid sequence of SEQ ID NOs:433, 434, and 435, respectively, and (ii) a VL comprising a VL CDR1, VL CDR2, and VL CDR3 having an amino acid sequence of SEQ ID NOs:436, 437, and 438, respectively. In one aspect, provided herein is an antibody that binds Vβ17, comprising: (i) a VH comprising a VH CDR1, a VH CDR2, and a VH CDR3 having an amino acid sequence of a VH CDR1, a VH CDR2, and a VH CDR3, respectively, of SEQ ID NO:439; and (ii) a VL comprising a VL CDR1, a VL CDR2, and a VL CDR3 having an amino acid sequence of a VL CDR1, a VL CDR2, and a VL CDR3, respectively, of SEQ ID NO:440. In one aspect, provided herein is an antibody that binds Vβ17, comprising a VH having an amino acid sequence of SEQ ID NO:439. In one aspect, provided herein is an antibody that binds Vβ17, comprising a VL having an amino acid sequence of SEQ ID NO:440. In one aspect, provided herein is an antibody that binds Vβ17, comprising a VH having an amino acid sequence of SEQ ID NO:439, and a VL having an amino acid sequence of SEQ ID NO:440. In one aspect, provided herein is an antibody that binds Vβ17, comprising a VH having an amino acid sequence having at least 95% identity to an amino acid sequence of SEQ ID NO:439. In one aspect, provided herein is an antibody that binds Vβ17, comprising a VL having an amino acid sequence having at least 95% identity to an amino acid sequence of SEQ ID NO:440. In one aspect, provided herein is an antibody that binds Vβ17, comprising a VH having an amino acid sequence having at least 95% identity to an amino acid sequence of SEQ ID NO:439, and a VL having an amino acid sequence having at least 95% identity to an amino acid sequence of SEQ ID NO:440.

In another aspect, provided herein is an antibody that competes for binding to Vβ17 with any of the Vβ17 antibodies described herein. In another aspect, provided herein is an antibody that binds to the same epitope as any of the Vβ17 antibodies described herein. In another aspect, provided is a Vβ17 antibody that binds an epitope on Vβ17 that overlaps with the epitope on Vβ17 bound by a Vβ17 antibody described herein.

In one aspect, provided is an antibody that competes for binding to Vβ17 with a Vβ17 reference antibody. In another aspect, provided is a Vβ17 antibody that binds to the same Vβ17 epitope as a Vβ17 reference antibody. In another aspect, provided is a Vβ17 antibody that binds an epitope on Vb 17 that overlaps with the epitope on Vβ17 bound by a Vb 17 reference antibody.

In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:31; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:32. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:65; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:66. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:99; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 100. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO: 133; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:\134. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:167; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 168. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:201; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:202. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:235; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:236. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:269; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:270. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:303; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:304. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:337; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:338. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:371; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:372. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:405; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:406. In one embodiment, the Vβ17 reference antibody comprises: (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:439; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:440.

In some embodiments, a multispecific antibody provided herein is a diabody, a cross-body, or a multispecific antibody obtained via a controlled Fab arm exchange as those described herein.

In some embodiments, the multispecific antibodies include IgG-like molecules with complementary CH3 domains that promote heterodimerization; recombinant IgG-like dual targeting molecules, wherein the two sides of the molecule each contain the Fab fragment or part of the Fab fragment of at least two different antibodies; IgG fusion molecules, wherein full length IgG antibodies are fused to an extra Fab fragment or parts of Fab fragment; Fc fusion molecules, wherein single chain Fv molecules or stabilized diabodies are fused to heavy-chain constant-domains, Fc-regions or parts thereof; Fab fusion molecules, wherein different Fab-fragments are fused together; ScFv- and diabody-based and heavy chain antibodies (e.g., domain antibodies, nanobodies) wherein different single chain Fv molecules or different diabodies or different heavy-chain antibodies (e.g. domain antibodies, nanobodies) are fused to each other or to another protein or carrier molecule.

In some embodiments, IgG-like molecules with complementary CH3 domains molecules include the Triomab/Quadroma (Trion Pharma/Fresenius Biotech), the Knobs-into-Holes (Genentech), CrossMAbs (Roche) and the electrostatically-matched (Amgen), the LUZ-Y (Genentech), the Strand Exchange Engineered Domain body (SEEDbody) (EMD Serono), the Biclonic (Merus) and the DuoBody (Genmab A/S).

In some embodiments, recombinant IgG-like dual targeting molecules include Dual Targeting (DT)-Ig (GSK/Domantis), Two-in-one Antibody (Genentech), Cross-linked Mabs (Karmanos Cancer Center), mAb2 (F-Star) and CovX-body (CovX/Pfizer).

In some embodiments, IgG fusion molecules include Dual Variable Domain (DVD)-Ig (Abbott), IgG-like Bispecific (ImClone/Eli Lilly), Ts2Ab (MedImmune/AZ) and BsAb (Zymogenetics), HERCULES (Biogen Idec) and TvAb (Roche).

In some embodiments, Fc fusion molecules can include ScFv/Fc Fusions (Academic Institution), SCORPION (Emergent BioSolutions/Trubion, ZymogeneticsBMS), Dual Affinity Retargeting Technology (Fc-DART) (MacroGenics) and Dual(ScFv)₂-Fab (National Research Center for Antibody Medicine--China).

In some embodiments, Fab fusion bispecific antibodies include F(ab)₂ (Medarex/AMGEN), Dual-Action or Bis-Fab (Genentech), Dock-and-Lock (DNL) (ImmunoMedics), Bivalent Bispecific (Biotecnol) and Fab-Fv (UCB-Celltech). ScFv-, diabody-based, and domain antibodies, include but are not limited to, Bispecific T Cell Engager (BiTE) (Micromet), Tandem Diabody (Tandab) (Affimed), Dual Affinity Retargeting Technology (DART) (MacroGenics), Single-chain Diabody (Academic), TCR-like Antibodies (AIT, ReceptorLogics), Human Serum Albumin ScFv Fusion (Merrimack) and COMBODY (Epigen Biotech), dual targeting nanobodies (Ablynx), dual targeting heavy chain only domain antibodies.

Full length bispecific antibodies provided herein can be generated for example using Fab arm exchange (or half molecule exchange) between two mono specific bivalent antibodies by introducing substitutions at the heavy chain CH3 interface in each half molecule to favor heterodimer formation of two antibody half molecules having distinct specificity either *in vitro* in cell-free environment or using co-expression. The Fab arm exchange reaction is the result of a disulfide-bond isomerization reaction and dissociation-association of CH3 domains. The heavy-chain disulfide bonds in the hinge regions of the parent mono specific antibodies are reduced. The resulting free cysteines of one of the parent monospecific antibodies form an inter heavy-chain disulfide bond with cysteine residues of a second parent mono specific antibody molecule and simultaneously CH3 domains of the parent antibodies release and reform by dissociation-association. The CH3 domains of the Fab arms can be engineered to favor heterodimerization over homodimerization. The resulting product is a bispecific antibody having two Fab arms or half molecules which each binding a distinct epitope, *e.g.,* an epitope on a first target and an epitope on a second target. Other methods of making multispecific antibodies are known and contemplated.

"Homodimerization" as used herein refers to an interaction of two heavy chains having identical CH3 amino acid sequences. "Homodimer" as used herein refers to an antibody having two heavy chains with identical CH3 amino acid sequences.

"Heterodimerization" as used herein refers to an interaction of two heavy chains having non-identical CH3 amino acid sequences. "Heterodimer" as used herein refers to an antibody having two heavy chains with non-identical CH3 amino acid sequences.

The "knob-in-hole" strategy (see, *e.g.,* PCT Publ. No. WO2006/028936) can be used to generate full length bispecific antibodies. Briefly, selected amino acids forming the interface of the CH3 domains in human IgG can be mutated at positions affecting CH3 domain interactions to promote heterodimer formation. An amino acid with a small side chain (hole) is introduced into a heavy chain of an antibody specifically binding a first antigen and an amino acid with a large side chain (knob) is introduced into a heavy chain of an antibody specifically binding a second antigen. After co-expression of the two antibodies, a heterodimer is formed as a result of the preferential interaction of the heavy chain with a "hole" with the heavy chain with a "knob." Exemplary CH3 substitution pairs forming a knob and a hole are (expressed as modified position in the first CH3 domain of the first heavy chain/modified position in the second CH3 domain of the second heavy chain): T366Y/F405A, T366W/ F405W, F405W/Y407A, T394W/Y407T, T394S/Y407A, T366W/T394S, F405W/T394S and T366W/T366S_L368A_Y407V.

Other strategies such as promoting heavy chain heterodimerization using electrostatic interactions by substituting positively charged residues at one CH3 surface and negatively charged residues at a second CH3 surface can be used, as described in US Pat. Publ. No. US2010/0015133; US Pat. Publ. No. US2009/0182127; US Pat. Publ. No. US2010/028637; or US Pat. Publ. No. US2011/0123532. In other strategies, heterodimerization can be promoted by the following substitutions (expressed as modified position in the first CH3 domain of the first heavy chain/modified position in the second CH3 domain of the second heavy chain): L3 51 Y _F 405A Y 407V /T394W, T366I_K392M_T394W/F405A_Y407V, T366L_K392M_T394W/F405A_Y407V, L351Y_Y407A/T366A_K409F, L351Y_Y407A/T366V K409F Y407A/T366A_K409F, or T350V_L351Y_F405A Y407V/T350V_T366L_K392L_T394W as described in U.S. Pat. Publ. No. US2012/0149876 or U.S. Pat. Publ. No. US2013/0195849.

In addition to methods described above, bispecific antibodies provided herein can be generated *in vitro* in a cell-free environment by introducing asymmetrical mutations in the CH3 regions of two mono specific homodimeric antibodies and forming the bispecific heterodimeric antibody from two parent monospecific homodimeric antibodies in reducing conditions to allow disulfide bond isomerization according to methods described in PCT Pat. Publ. No. WO2011/131746. In the methods, the first monospecific bivalent antibody and the second monospecific bivalent antibody are engineered to have certain substitutions at the CH3 domain that promotes heterodimer stability; the antibodies are incubated together under reducing conditions sufficient to allow the cysteines in the hinge region to undergo disulfide bond isomerization; thereby generating the bispecific antibody by Fab arm exchange. The incubation conditions can optionally be restored to non-reducing conditions. Exemplary reducing agents that can be used are 2-mercaptoethylamine (2-MEA), dithiothreitol (DTT), dithioerythritol (DTE), glutathione, tris (2-carboxyethyl) phosphine (TCEP), L-cysteine and betamercaptoethanol, preferably a reducing agent selected from the group consisting of 2-mercaptoethylamine, dithiothreitol and tris (2-carboxyethyl) phosphine. For example, incubation for at least 90 min at a temperature of at least 20°C in the presence of at least 25 mM 2-MEA or in the presence of at least 0.5 mM dithiothreitol at a pH from 5-8, for example at pH of 7.0 or at pH of 7.4 can be used.

In some embodiments, the Vβ17 antibody comprises a single chain antibody. In some embodiments, the Vβ17 antibody comprises a single domain antibody. In certain embodiments, the Vβ17 antibody comprises a nanobody. In certain embodiments, the Vβ17 antibody comprises a VHH antibody. In certain embodiments, the Vβ17 antibody comprises a llama antibody. In some embodiments, the Vβ17 antibody does not comprise a single chain antibody. In some embodiments, the Vβ17 antibody does not comprise a single domain antibody. In certain embodiments, the Vβ17 antibody does not comprise a nanobody. In certain embodiments, the Vβ17 antibody does not comprise a VHH antibody. In certain embodiments, the Vβ17 antibody does not comprise a llama antibody.

According to another particular aspect, provided herein is a Vβ17 antibody or antigen-binding fragment thereof that induces antibody-dependent cell-mediated cytotoxicity (ADCC). The antibody or antigen-binding fragment thereof can, for example, induce ADCC *in vitro.*

In certain embodiments, the TRGV9 antibody induces T cell dependent cytotoxicity of a second cell *in vitro* with an EC₅₀ of less than about 160 pM, when assessed *in vitro* at an effector to target cell ratio of 1:1.

In some embodiments, Vβ17 is present on the surface of a T cell. In some embodiments, the Vβ17 is present on the surface of a T cell, and the second target antigen is on the surface of a second cell. In some embodiments, the second cell is killed when the multispecific antibody binds to the Vβ17 on the surface of the T cell and the second target antigen on the surface of the second cell.

In some embodiments, the multispecific antibody induces T cell dependent cytotoxicity of the second cell *in vitro* with an EC₅₀ of less than about 500 pM. In some embodiments, the multispecific antibody induces T cell dependent cytotoxicity of the second cell *in vitro* with an EC₅₀ of less than about 300 pM. In some embodiments, the multispecific antibody induces γδ T cell dependent cytotoxicity of the second cell *in vitro* with an EC₅₀ of less than about 160 pM. In some embodiments, the EC₅₀ is assessed with a mixture of γδ T effector cells and target cells expressing the second target antigen. In some embodiments, the effector cell to target cell ratio is about 0.01 to 1 to about 5 to 1. In some embodiments, the effector cell to target cell ratio is about 0.1 to 1 to about 2 to 1. In some embodiments, the effector cell to target cell ratio is about 1:1.

In certain embodiments, the EC₅₀ is less than about 1000 pM, less than about 900 pM, less than about 800 pM, less than about 700 pM, less than about 600 pM, less than about 500 pM, less than about 400 pM, less than about 300 pM, less than about 200 pM, less than about 190 pM, less than about 180 pM, less than about 170 pM, less than about 160 pM, less than about 150 pM, less than about 140 pM, less than about 130 pM, less than about 120 pM, less than about 110 pM, less than about 100 pM, less than about 90 pM, less than about 80 pM, less than about 70 pM, less than about 60 pM, less than about 50 pM, less than about 40 pM, less than about 30 pM, less than about 20 pM, or less than about 10 pM.

In certain embodiments, the effector to target cell ratio can, for example, be 0.01:1, 0.02:1, 0.03:1, 0.04:1, 0.05:1, 0.06:1, 0.07:1, 0.08:1, 0.09:1, 1:1, 2:1, 3:1, 4:1, 5:1, 6:1, 7:1, 8:1, 9:1, or 10:1. In certain embodiments, the concentration of the multispecific antibody is about 0.000005 ng/mL, about 0.00005 ng/mL, about 0.0005, about 0.005 ng/mL, about 0.01 ng/mL, about 0.02 ng/mL, about 0.03 ng/mL, about 0.04 ng/mL, about 0.05 ng/mL, about 0.06 ng/mL, about 0.07 ng/mL, about 0.08 ng/mL, about 0.09 ng/mL, about 0.1 ng/mL, about 0.5 ng/mL, about 1.0 ng/mL, about 10 ng/mL, about 20 ng/mL about, about 30 ng/mL about 40 ng/mL, about 50 ng/mL, about 60 ng/mL, about 70 ng/mL, about 80 ng/mL, about 90 ng/mL, about 100 ng/mL, or about 1000 ng/mL.

In another aspect, provided herein is an antibody that competes for binding to Vβ17 with any of the Vβ17 antibodies described herein. In another aspect, provided herein is an antibody that binds to the same epitope as any of the Vβ17 antibodies described herein. In another aspect, provided is a Vβ17 antibody that binds an epitope on Vβ17 that overlaps with the epitope on Vβ17 bound by a Vβ17 antibody described herein. In some embodiments, the Vβ17 antibody comprises a VH CDR1, VH CDR2, and VH CDR3 of a Vβ17 antibody provided herein. In some embodiments, the Vβ17 antibody comprises a VL CDR1, VL CDR2, and VL CDR3 of a Vβ17 antibody provided herein. In some embodiments, the Vβ17 antibody comprises a VH CDR1, VH CDR2, VH CDR3, a VL CDR1, VL CDR2, and VL CDR3 of a Vβ17 antibody provided herein. In some embodiments, the Vβ17 antibody comprises a VH of a Vβ17 antibody provided herein. In some embodiments, the Vβ17 antibody comprises a VL of a Vβ17 antibody provided herein. In some embodiments, the Vβ17 antibody comprises a VH and a VL of a Vβ17 antibody provided herein. In some embodiments, the Vβ17 antibody comprises a VH CDR1, VH CDR2, VH CDR3, a VL CDR1, VL CDR2, and VL CDR3 of a Vβ17 antibody provided herein. In some embodiments, the VH CDR1, VH CDR2, VH CDR3, VL CDR1, VL CDR2, and VL CDR3 amino acid sequences of the Vβ17 antibody are according to the Kabat numbering system. In some embodiments, the VH CDR1, VH CDR2, VH CDR3, VL CDR1, VL CDR2, and VL CDR3 amino acid sequences of the Vβ17 antibody are according to the Chothia numbering system. In some embodiments, the VH CDR1, VH CDR2, VH CDR3, VL CDR1, VL CDR2, and VL CDR3 amino acid sequences of the Vβ17 antibody are according to the AbM numbering system. In some embodiments, the VH CDR1, VH CDR2, VH CDR3, VL CDR1, VL CDR2, and VL CDR3 amino acid sequences of the Vβ17 antibody are according to the Contact numbering system. In some embodiments, the VH CDR1, VH CDR2, VH CDR3, VL CDR1, VL CDR2, and VL CDR3 amino acid sequences of the Vβ17 antibody are according to the IMGT numbering system. In certain embodiments, the Vβ17 antibody is a multispecific antibody. In some embodiments, the Vβ17 antibody is a bispecific antibody.

In another aspect, provided is an antibody that competes for binding to Vβ17 with a Vβ17 reference antibody. In another aspect, provided is a Vβ17 antibody that binds to the same Vβ17 epitope as a Vβ17 reference antibody. In another aspect, provided is a Vβ17 antibody that binds an epitope on Vβ17 that overlaps with the epitope on Vβ17 bound by a Vβ17 reference antibody. In some embodiments, the Vβ17 reference antibody comprises a VH CDR1, VH CDR2, and VH CDR3 of a Vβ17 reference antibody provided herein. In some embodiments, the Vβ17 reference antibody comprises a VL CDR1, VL CDR2, and VL CDR3 of a Vβ17 reference antibody provided herein. In some embodiments, the Vβ17 reference antibody comprises a VH CDR1, VH CDR2, VH CDR3, a VL CDR1, VL CDR2, and VL CDR3 of a Vβ17 reference antibody provided herein. In some embodiments, the Vβ17 reference antibody comprises a VH of a Vβ17 reference antibody provided herein. In some embodiments, the Vβ17 reference antibody comprises a VL of a Vβ17 reference antibody provided herein. In some embodiments, the Vβ17 reference antibody comprises a VH and a VL of a Vβ17 reference antibody provided herein. In some embodiments, the Vβ17 reference antibody comprises a VH CDR1, VH CDR2, VH CDR3, a VL CDR1, VL CDR2, and VL CDR3 of a Vβ17 reference antibody provided herein. In some embodiments, the VH CDR1, VH CDR2, VH CDR3, VL CDR1, VL CDR2, and VL CDR3 amino acid sequences of the Vβ17 reference antibody are according to the Kabat numbering system. In some embodiments, the VH CDR1, VH CDR2, VH CDR3, VL CDR1, VL CDR2, and VL CDR3 amino acid sequences of the Vβ17 reference antibody are according to the Chothia numbering system. In some embodiments, the VH CDR1, VH CDR2, VH CDR3, VL CDR1, VL CDR2, and VL CDR3 amino acid sequences of the Vβ17 reference antibody are according to the AbM numbering system. In some embodiments, the VH CDR1, VH CDR2, VH CDR3, VL CDR1, VL CDR2, and VL CDR3 amino acid sequences of the Vβ17 reference antibody are according to the Contact numbering system. In some embodiments, the VH CDR1, VH CDR2, VH CDR3, VL CDR1, VL CDR2, and VL CDR3 amino acid sequences of the Vβ17 reference antibody are according to the IMGT numbering system. In certain embodiments, the antibody is a multispecific antibody. In some embodiments, the antibody is a bispecific antibody. In certain embodiments, the Vβ17 reference antibody is a multispecific antibody. In some embodiments, the Vβ17 reference antibody is a bispecific antibody.

In some embodiments described herein, immune effector properties of the antibodies provided herein can be enhanced or silenced through Fc modifications by techniques known to those skilled in the art. For example, Fc effector functions such as Clq binding, complement dependent cytotoxicity (CDC), antibody-dependent cell-mediated cytotoxicity (ADCC), antibody-dependent cell-mediated phagocytosis (ADCP), down regulation of cell surface receptors (*e.g.,* B cell receptor; BCR), *etc.* can be provided and/or controlled by modifying residues in the Fc responsible for these activities.

"Antibody-dependent cell-mediated cytotoxicity" or "ADCC" refers to a cell-mediated reaction in which non-specific cytotoxic cells that express Fc receptors (FcRs) (*e.g.* Natural Killer (NK) cells, neutrophils, and macrophages) recognize bound antibody on a target cell and subsequently cause lysis of the target cell.

The ability of antibodies to induce ADCC can be enhanced by engineering their oligosaccharide component. Human IgG1 or IgG3 are N-glycosylated at Asn297 with the majority of the glycans in the well-known biantennary G0, G0F, G1, G1F, G2 or G2F forms. Antibodies produced by non-engineered CHO cells typically have a glycan fucose content of about at least 85%. The removal of the core fucose from the biantennary complex-type oligosaccharides attached to the Fc regions enhances the ADCC of antibodies via improved FcyRIIIa binding without altering antigen binding or CDC activity. Such Abs can be achieved using different methods reported to lead to the successful expression of relatively high defucosylated antibodies bearing the biantennary complex-type of Fc oligosaccharides such as control of culture osmolality (Konno et al., Cytotechnology 64:249-65, 2012), application of a variant CHO line Lec13 as the host cell line (Shields et al., J Biol Chem 277:26733-26740, 2002), application of a variant CHO line EB66 as the host cell line (Olivier et al., MAbs; 2(4), 2010; Epub ahead of print; PMID:20562582), application of a rat hybridoma cell line YB2/0 as the host cell line (Shinkawa et al., J Biol Chem 278:3466-3473, 2003), introduction of small interfering RNA specifically against the α-1,6-fucosyltrasferase (FUT8) gene (Mori et al., Biotechnol Bioeng 88:901-908, 2004), or coexpression of β-1,4-N-acetylglucosaminyltransferase III and golgi α-mannosidase II or a potent alpha-mannosidase I inhibitor, kifunensine (Ferrara et al., J Biol Chem 281:5032-5036, 2006, Ferrara et al., Biotechnol Bioeng 93:851-861, 2006; Xhou et al., Biotechnol Bioeng 99:652-65, 2008).

In some embodiments described herein, ADCC elicited by the antibodies provided herein can also be enhanced by certain substitutions in the antibody Fc. Exemplary substitutions are for example substitutions at amino acid positions 256, 290, 298, 312, 356, 330, 333, 334, 360, 378 or 430 (residue numbering according to the EU index) as described in U.S. Pat. No. 6,737,056.

In some embodiments, a Vβ17 antibody provided herein is chimeric. In some embodiments, a Vβ17 antibody provided herein is human. In some embodiments, a Vβ17 antibody provided herein is humanized. In certain embodiments, a Vβ17 antibody provided herein is an isolated Vβ17 antibody. In some embodiments, a Vβ17 antigen binding fragment provided herein is chimeric. In some embodiments, a Vβ17 antigen binding fragment provided herein is human. In some embodiments, a Vβ17 antigen binding fragment provided herein is humanized. In certain embodiments, a Vβ17 antigen binding fragment provided herein is an isolated Vβ17 antigen binding fragment. In some embodiments, a Vβ17 antibody provided herein is an IgG antibody. In some embodiments, the IgG antibody is an IgG1 antibody. In some embodiments, the IgG antibody is an IgG2 antibody. In some embodiments, the IgG antibody is an IgG3 antibody. In some embodiments, the IgG antibody is an IgG4 antibody. In some embodiments, a Vβ17 antibody provided herein is multivalent. In some embodiments, the Vβ17 antibody is capable of binding at least three antigens. In some embodiments, the Vβ17 antibody is capable of binding at least four antigens. In some embodiments, the Vβ17 antibody is capable of binding at least five antigens.

Also provided is a nucleic acid encoding an antibody provided herein. In another general aspect, provide is a vector comprising an isolated nucleic acid encoding an antibody provided herein. In another general aspect, provided is a vector comprising an isolated nucleic acid encoding an antibody provided herein. Also provided is a vector comprising a nucleic acid encoding an antibody provided herein. Also provided is a host cell comprising a vector comprising a nucleic acid encoding an antibody provided herein. Also provided is a kit comprising the vector comprising a nucleic acid encoding an antibody provided herein, and packaging for the same. In another general aspect, provided herein is an isolated nucleic acid encoding a monoclonal antibody or antigen-binding fragment thereof provided herein. In certain embodiments, the antibody is a Vβ17 antibody.

It will be appreciated by those skilled in the art that the coding sequence of a protein can be changed (*e*.*g*., replaced, deleted, inserted, *etc.*) without changing the amino acid sequence of the protein. Accordingly, it will be understood by those skilled in the art that nucleic acid sequences encoding antibodies provided herein can be altered without changing the amino acid sequences of the proteins.

Any vector known to those skilled in the art in view of the present disclosure can be used, such as a plasmid, a cosmid, a phage vector or a viral vector. In some embodiments, the vector is a recombinant expression vector such as a plasmid. The vector can include any element to establish a conventional function of an expression vector, for example, a promoter, ribosome binding element, terminator, enhancer, selection marker, and origin of replication. The promoter can be a constitutive, inducible or repressible promoter. A number of expression vectors capable of delivering nucleic acids to a cell are known in the art and can be used herein for production of an antibody or antigen-binding fragment thereof in the cell. Conventional cloning techniques or artificial gene synthesis can be used to generate a recombinant expression vector according to certain embodiments. Such techniques are well known to those skilled in the art in view of the present disclosure.

Also provided is a host cell comprising an isolated nucleic acid encoding an antibody provided herein. Also provided is a host cell comprising an isolated nucleic acid encoding an antigen binding fragment provided herein. Any host cell known to those skilled in the art in view of the present disclosure can be used for recombinant expression of antibodies or antigen-binding fragments thereof provided herein. In some embodiments, the host cells are E. coli TG1 or BL21 cells (for expression of, *e.g.,* an scFv or Fab antibody), CHO-DG44 or CHO-K1 cells or HEK293 cells (for expression of, *e.g.,* a full-length IgG antibody). According to particular embodiments, the recombinant expression vector is transformed into host cells by conventional methods such as chemical transfection, heat shock, or electroporation, where it is stably integrated into the host cell genome such that the recombinant nucleic acid is effectively expressed.

Also provided are methods of producing an antibody disclosed herein. The methods comprise culturing a cell comprising a nucleic acid encoding the antibody under conditions to produce an antibody and recovering the antibody from the cell or cell culture (*e.g.,* from the supernatant). Expressed antibodies can be harvested from the cells and purified according to conventional techniques known in the art and as described herein.

### Pharmaceutical Compositions

In another general aspect, provided is a pharmaceutical composition comprising a Vβ17 antibody provided herein and a pharmaceutically acceptable carrier. In certain embodiments, the antibody is isolated. Also provided is a method of producing the pharmaceutical composition, comprising combining the antibody with a pharmaceutically acceptable carrier to obtain the pharmaceutical composition.

In another general aspect, provided is a pharmaceutical composition comprising a Vβ17 multispecific antibody provided herein and a pharmaceutically acceptable carrier. In certain embodiments, the multispecific antibody is isolated. Also provided is a method of producing the pharmaceutical composition, comprising combining the multispecific antibody with a pharmaceutically acceptable carrier to obtain the pharmaceutical composition. Any of the antibodies provided herein are contemplated in the pharmaceutical compositions.

The term "pharmaceutical composition" as used herein means a product comprising an antibody provided herein together with a pharmaceutically acceptable carrier. Antibodies of provided herein and compositions comprising them are also useful in the manufacture of a medicament for therapeutic applications.

As used herein, the term "carrier" refers to any excipient, diluent, filler, salt, buffer, stabilizer, solubilizer, oil, lipid, lipid containing vesicle, microsphere, liposomal encapsulation, or other material well known in the art for use in pharmaceutical formulations. It will be understood that the characteristics of the carrier, excipient or diluent will depend on the route of administration for a particular application. As used herein, the term "pharmaceutically acceptable carrier" refers to a non-toxic material that does not interfere with the effectiveness of a composition provided herein the biological activity of a composition provided herein. According to particular embodiments, in view of the present disclosure, any pharmaceutically acceptable carrier suitable for use in an antibody pharmaceutical composition can be used herein.

The formulation of pharmaceutically active ingredients with pharmaceutically acceptable carriers is known in the art, *e.g*., Remington: The Science and Practice of Pharmacy (e.g. 21st edition (2005), and any later editions). Non-limiting examples of additional ingredients include: buffers, diluents, solvents, tonicity regulating agents, preservatives, stabilizers, and chelating agents. One or more pharmaceutically acceptable carriers can be used in formulating the pharmaceutical compositions provided herein.

In one embodiment, the pharmaceutical composition is a liquid formulation. A preferred example of a liquid formulation is an aqueous formulation, *i.e.,* a formulation comprising water. The liquid formulation can comprise a solution, a suspension, an emulsion, a microemulsion, a gel, and the like. An aqueous formulation typically comprises at least 50% w/w water, or at least 60%, 70%, 75%, 80%, 85%, 90%, or at least 95% w/w of water.

In one embodiment, the pharmaceutical composition can be formulated as an injectable which can be injected, for example, via an injection device (*e.g*., a syringe or an infusion pump). The injection can be delivered subcutaneously, intramuscularly, intraperitoneally, intravitreally, or intravenously, for example.

In another embodiment, the pharmaceutical composition is a solid formulation, *e.g*., a freeze-dried or spray-dried composition, which can be used as is, or whereto the physician or the patient adds solvents, and/or diluents prior to use. Solid dosage forms can include tablets, such as compressed tablets, and/or coated tablets, and capsules (*e.g*., hard or soft gelatin capsules). The pharmaceutical composition can also be in the form of sachets, dragees, powders, granules, lozenges, or powders for reconstitution, for example.

The dosage forms can be immediate release, in which case they can comprise a water-soluble or dispersible carrier, or they can be delayed release, sustained release, or modified release, in which case they can comprise water-insoluble polymers that regulate the rate of dissolution of the dosage form in the gastrointestinal tract or under the skin.

In other embodiments, the pharmaceutical composition can be delivered intranasally, intrabuccally, or sublingually.

The pH in an aqueous formulation can be between pH 3 and pH 10. In one embodiment, the pH of the formulation is from about 7.0 to about 9.5. In another embodiment, the pH of the formulation is from about 3.0 to about 7.0.

In another embodiment, the pharmaceutical composition comprises a buffer. Non-limiting examples of buffers include: arginine, aspartic acid, bicine, citrate, disodium hydrogen phosphate, fumaric acid, glycine, glycylglycine, histidine, lysine, maleic acid, malic acid, sodium acetate, sodium carbonate, sodium dihydrogen phosphate, sodium phosphate, succinate, tartaric acid, tricine, and tris(hydroxymethyl)-aminomethane, and mixtures thereof. The buffer can be present individually or in the aggregate, in a concentration from about 0.01 mg/ml to about 50 mg/ml, for example from about 0.1 mg/ml to about 20 mg/ml. Pharmaceutical compositions comprising each one of these specific buffers constitute alternative embodiments.

In another embodiment, the pharmaceutical composition comprises a preservative. Non-limiting examples of preservatives include: benzethonium chloride, benzoic acid, benzyl alcohol, bronopol, butyl 4-hydroxybenzoate, chlorobutanol, chlorocresol, chlorohexidine, chlorphenesin, o-cresol, m-cresol, p-cresol, ethyl 4-hydroxybenzoate, imidurea, methyl 4-hydroxybenzoate, phenol, 2-phenoxyethanol, 2-phenylethanol, propyl 4-hydroxybenzoate, sodium dehydroacetate, thiomerosal, and mixtures thereof. The preservative can be present individually or in the aggregate, in a concentration from about 0.01 mg/ml to about 50 mg/ml, for example from about 0.1 mg/ml to about 20 mg/ml. Pharmaceutical compositions comprising each one of these specific preservatives constitute alternative embodiments.

In another embodiment, the pharmaceutical composition comprises an isotonic agent. Non-limiting examples of isotonic agents include a salt (such as sodium chloride), an amino acid (such as glycine, histidine, arginine, lysine, isoleucine, aspartic acid, tryptophan, and threonine), an alditol (such as glycerol, 1,2-propanediol propyleneglycol), 1,3-propanediol, and 1,3-butanediol), polyethyleneglycol (*e.g*. PEG400), and mixtures thereof. Another example of an isotonic agent includes a sugar. Non-limiting examples of sugars can include mono-, di-, or polysaccharides, or water-soluble glycans, including for example fructose, glucose, mannose, sorbose, xylose, maltose, lactose, sucrose, trehalose, dextran, pullulan, dextrin, cyclodextrin, alpha and beta- HPCD, soluble starch, hydroxyethyl starch, and sodium carboxymethylcellulose. Another example of an isotonic agent is a sugar alcohol, wherein the term "sugar alcohol" is defined as a C(4-8) hydrocarbon having at least one -OH group. Non-limiting examples of sugar alcohols include mannitol, sorbitol, inositol, galactitol, dulcitol, xylitol, and arabitol. The isotonic agent can be present individually or in the aggregate, in a concentration from about 0.01 mg/ml to about 50 mg/ml, for example from about 0.1 mg/ml to about 20 mg/ml. Pharmaceutical compositions comprising each one of these specific isotonic agents constitute alternative embodiments.

In another embodiment, the pharmaceutical composition comprises a chelating agent. Non-limiting examples of chelating agents include citric acid, aspartic acid, salts of ethylenediaminetetraacetic acid (EDTA), and mixtures thereof. The chelating agent can be present individually or in the aggregate, in a concentration from about 0.01 mg/ml to about 50 mg/ml, for example from about 0.1 mg/ml to about 20 mg/ml. Pharmaceutical compositions comprising each one of these specific chelating agents constitute alternative embodiments.

In another embodiment, the pharmaceutical composition comprises a stabilizer. Non-limiting examples of stabilizers include one or more aggregation inhibitors, one or more oxidation inhibitors, one or more surfactants, and/or one or more protease inhibitors.

In another embodiment, the pharmaceutical composition comprises a stabilizer, wherein said stabilizer is carboxy-/hydroxycellulose and derivates thereof (such as HPC, HPC-SL, HPC-L and HPMC), cyclodextrins, 2-methylthioethanol, polyethylene glycol (such as PEG 3350), polyvinyl alcohol (PVA), polyvinyl pyrrolidone, salts (such as sodium chloride), sulphur-containing substances such as monothioglycerol), or thioglycolic acid. The stabilizer can be present individually or in the aggregate, in a concentration from about 0.01 mg/ml to about 50 mg/ml, for example from about 0.1 mg/ml to about 20 mg/ml. Pharmaceutical compositions comprising each one of these specific stabilizers constitute alternative embodiments.

In further embodiments, the pharmaceutical composition comprises one or more surfactants, preferably a surfactant, at least one surfactant, or two different surfactants. The term "surfactant" refers to any molecules or ions that are comprised of a water-soluble (hydrophilic) part, and a fat-soluble (lipophilic) part. The surfactant can, for example, be selected from the group consisting of anionic surfactants, cationic surfactants, nonionic surfactants, and/or zwitterionic surfactants. The surfactant can be present individually or in the aggregate, in a concentration from about 0.1 mg/ml to about 20 mg/ml. Pharmaceutical compositions comprising each one of these specific surfactants constitute alternative embodiments.

In a further embodiment, the pharmaceutical composition comprises one or more protease inhibitors, such as, *e.g*., EDTA, and/or benzamidine hydrochloric acid (HCl). The protease inhibitor can be present individually or in the aggregate, in a concentration from about 0.1 mg/ml to about 20 mg/ml. Pharmaceutical compositions comprising each one of these specific protease inhibitors constitute alternative embodiments.

In another general aspect, provided herein is a method of producing a pharmaceutical composition comprising an antibody or antigen-binding fragment thereof provided herein, comprising combining an antibody or antigen-binding fragment thereof with a pharmaceutically acceptable carrier to obtain the pharmaceutical composition.

### Methods of use

The functional activity of antibodies provided herein can be characterized by methods known in the art and as described herein. Methods for characterizing antibodies and antigen-binding fragments thereof include, but are not limited to, affinity and specificity assays including Biacore, ELISA, and OctetRed analysis; binding assays to detect the binding of antibodies to target cells by FACS; binding assays to detect the binding of antibodies to the target antigen on cells. According to particular embodiments, the methods for characterizing antibodies and antigen-binding fragments thereof include those described below. In certain embodiments, the antibody is a Vβ17 antibody.

Also provided is a method of activating a T cell expressing Vβ17, comprising contacting the T cell with a Vβ17 antibody provided herein. In some embodiments, the contacting results in an increase in CD69, CD25, and/or Granzyme B expression, as compared to a control T cell expressing Vβ17.

In another general aspect, provided is a method of inactivating a T cell expressing Vβ17, comprising contacting the T cell with an antibody that binds to a Vβ17 provided herein. In another general aspect, provided is a method of blocking activation of a T cell expressing Vβ17, comprising contacting the T cell with an antibody that binds to a Vβ17 provided herein. In another general aspect, provided is a method of modulating the activation of a T cell expressing Vβ17, comprising contacting the T cell with an antibody that binds to a Vβ17 provided herein.

Also provided herein is a method of treating a disease or disorder in a subject, comprising administering to the subject a Vβ17 antibody provided herein. Also provided herein is a method of treating a disease or disorder in a subject, comprising administering to the subject a Vβ17 antigen binding fragment provided herein. Also provided herein is a method of treating a disease or disorder in a subject, comprising administering to the subject a pharmaceutical composition comprising a Vβ17 antibody provided herein. Also provided herein is a method of treating a disease or disorder in a subject, comprising administering to the subject a pharmaceutical composition comprising a Vβ17 antigen binding fragment provided herein.

Also provided herein is a Vβ17 antibody for use in the treatment of a disease, wherein the Vβ17 antibody is provided herein. In some embodiments, the disease is caused all or in part by a target cell having the second target present on the surface of the target cell. In some embodiments, the subject is human. In some embodiments, the subject is a subject in need thereof.

Also provided herein is use of a Vβ17 antibody in the manufacture of a medicament for the treatment of a disease, wherein the Vβ17 antibody is provided herein. In some embodiments, the disease is caused all or in part by a target cell having the second target present on the surface of the target cell. In some embodiments, the subject is human. In some embodiments, the subject is a subject in need thereof.

In some embodiments, the subject is a subject in need thereof. In some embodiments, the subject is a human. In specific embodiments, the subject is administered an effective amount.

As used herein, the term "effective amount" refers to an amount of an active ingredient or component that elicits the desired biological or medicinal response in a subject.

According to particular embodiments, an effective amount refers to the amount of therapy which is sufficient to achieve one, two, three, four, or more of the following effects: (i) reduce or ameliorate the severity of the disease, disorder or condition to be treated or a symptom associated therewith; (ii) reduce the duration of the disease, disorder or condition to be treated, or a symptom associated therewith; (iii) prevent the progression of the disease, disorder or condition to be treated, or a symptom associated therewith; (iv) cause regression of the disease, disorder or condition to be treated, or a symptom associated therewith; (v) prevent the development or onset of the disease, disorder or condition to be treated, or a symptom associated therewith; (vi) prevent the recurrence of the disease, disorder or condition to be treated, or a symptom associated therewith; (vii) reduce hospitalization of a subject having the disease, disorder or condition to be treated, or a symptom associated therewith; (viii) reduce hospitalization length of a subject having the disease, disorder or condition to be treated, or a symptom associated therewith; (ix) increase the survival of a subject with the disease, disorder or condition to be treated, or a symptom associated therewith; (xi) inhibit or reduce the disease, disorder or condition to be treated, or a symptom associated therewith in a subject; and/or (xii) enhance or improve the prophylactic or therapeutic effect(s) of another therapy.

The effective amount or dosage can vary according to various factors, such as the disease, disorder or condition to be treated, the means of administration, the target site, the physiological state of the subject (including, *e.g*., age, body weight, health), whether the subject is a human or an animal, other medications administered, and whether the treatment is prophylactic or therapeutic. Treatment dosages are optimally titrated to optimize safety and efficacy.

According to particular embodiments, the compositions described herein are formulated to be suitable for the intended route of administration to a subject. For example, the compositions described herein can be formulated to be suitable for intravenous, subcutaneous, or intramuscular administration.

As used herein, the terms "treat," "treating," and "treatment" are all intended to refer to an amelioration or reversal of at least one measurable physical parameter related to a cancer, which is not necessarily discernible in the subject, but can be discernible in the subject. The terms "treat," "treating," and "treatment," can also refer to causing regression, preventing the progression, or at least slowing down the progression of the disease, disorder, or condition. In a particular embodiment, "treat," "treating," and "treatment" refer to an alleviation, prevention of the development or onset, or reduction in the duration of one or more symptoms associated with the disease, disorder, or condition, such as a tumor or more preferably a cancer. In a particular embodiment, "treat," "treating," and "treatment" refer to prevention of the recurrence of the disease, disorder, or condition. In a particular embodiment, "treat," "treating," and "treatment" refer to an increase in the survival of a subject having the disease, disorder, or condition. In a particular embodiment, "treat," "treating," and "treatment" refer to elimination of the disease, disorder, or condition in the subject.

In some embodiments, a Vβ17 antibody provided herein is used in combination with a supplemental therapy.

As used herein, the term "in combination," in the context of the administration of two or more therapies to a subject, refers to the use of more than one therapy. The use of the term "in combination" does not restrict the order in which therapies are administered to a subject. For example, a first therapy (*e.g*., a composition described herein) can be administered prior to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject.

Vβ17 antibodies provided herein may also be used as agents to detect Vβ17-expressing cells. Thus, in another methods, provided is a method of detecting a cell expressing Vβ17, comprising contacting a cell with a Vβ17 antibody provided herein. In certain embodiments, the detecting is by ELISA. In some embodiments, the detecting is by FACS analysis. Also provided are kits comprising a Vβ17 antibody provided herein, and instructions for use.

### Enrichment and detection methods

In one aspect, the Vβ17 antibodies provided herein are used as agents to detect Vβ17-expressing cells. Thus, in other methods, provided is a method of detecting a cell expressing Vβ17, comprising contacting a cell with a Vβ17 antibody provided herein. In certain embodiments, the detecting is by ELISA. In some embodiments, the detecting is by FACS analysis. Also provided are kits comprising a Vβ17 antibody provided herein, and instructions for use.

Enrichment, isolation, separation, purification, sorting, selecting, capturing or detecting, or any combination thereof can be done using known technologies such as bead, microfluidics, solid support, columns, and the like. For example, Vβ17 cells may be separated or visualized using known methods when bound to the Vβ17 antibodies provided herein.

The Vβ17 antibodies or multi specific Vβ17 antibodies provided herein can be used to selectively enrich, isolate, separate, purify, sort, select, capture or detect Vβ17-expressing cells. The Vβ17 antibodies or multispecific Vβ17 antibodies provided herein may be utilized in a bispecific format, *e.g*. containing a first antigen binding domain that specifically binds Vβ17 and a second antigen binding domain that specifically binds a second target. In other embodiments, the multispecific Vβ17 antibodies provided herein may be utilized in a format that further incorporates a third antigen binding domain that specifically binds a third antigen (*e.g.,* at a trispecific antibody). In other embodiments, the multispecific Vβ17 antibodies provided herein may be utilized in a format that further incorporates a fourth antigen binding domain that specifically binds a fourth antigen. (*e.g*., as a quadraspecific antibody).

In one aspect, provided herein is a method of enriching a Vβ17-expressing cell comprising: providing a sample comprising the Vβ17-expressing cell; contacting the sample with a Vβ17 antibody provided herein; and enriching the Vβ17-expressing cell bound to the Vβ17 antibody. In one aspect, provided herein is a method of isolating a Vβ17-expressing cell comprising: providing a sample comprising the Vβ17-expressing cell; contacting the sample with a Vβ17 antibody provided herein; and isolating the Vβ17-expressing cell bound to the Vβ17 antibody. In one aspect, provided herein is a method of separating a Vβ17-expressing cell comprising: providing a sample comprising the Vβ17-expressing cell; contacting the sample with a Vβ17 antibody provided herein; and separating the Vβ17-expressing cell bound to the Vβ17 antibody. In one aspect, provided herein is a method of purifying a Vβ17-expressing cell comprising: providing a sample comprising the Vβ17-expressing cell; contacting the sample with a Vβ17 antibody provided herein; and purifying the Vβ17-expressing cell bound to the Vβ17 antibody. In one aspect, provided herein is a method of sorting a Vβ17-expressing cell comprising: providing a sample comprising the Vβ17-expressing cell; contacting the sample with a Vβ17 antibody provided herein; and sorting the Vβ17-expressing cell bound to the Vβ17 antibody. In one aspect, provided herein is a method of selecting a Vβ17-expressing cell comprising: providing a sample comprising the Vβ17-expressing cell; contacting the sample with a Vβ17 antibody provided herein; and selecting the Vβ17-expressing cell bound to the Vβ17 antibody. In one aspect, provided herein is a method of capturing a Vβ17-expressing cell comprising: providing a sample comprising the Vβ17-expressing cell; contacting the sample with a Vβ17 antibody provided herein; and capturing the Vβ17-expressing cell bound to the Vβ17 antibody. In one aspect, provided herein is a method of detecting a Vβ17-expressing cell comprising: providing a sample comprising the Vβ17-expressing cell; contacting the sample with a Vβ17 antibody provided herein; and detecting the Vβ17-expressing cell bound to the Vβ17 antibody.

In one aspect, provided herein is a method of enriching a Vβ17-expressing cell comprising: contacting a Vβ17-expressing cell with a Vβ17 antibody provided herein; and enriching the Vβ17-expressing cell bound to the Vβ17 antibody. In one aspect, provided herein is a method of isolating a Vβ17-expressing cell comprising: contacting a Vβ17-expressing cell with a Vβ17 antibody provided herein; and isolating the Vβ17-expressing cell bound to the Vβ17 antibody. In one aspect, provided herein is a method of separating a Vβ17-expressing cell comprising: contacting a Vβ17-expressing cell with a Vβ17 antibody provided herein; and separating the Vβ17-expressing cell bound to the Vβ17 antibody. In one aspect, provided herein is a method of purifying a Vβ17-expressing cell comprising: contacting a Vβ17-expressing cell with a Vβ17 antibody provided herein; and purifying the Vβ17-expressing cell bound to the Vβ17 antibody. In one aspect, provided herein is a method of sorting a Vβ17-expressing cell comprising: contacting a Vβ17-expressing cell with a Vβ17 antibody provided herein; and sorting the Vβ17-expressing cell bound to the Vβ17 antibody. In one aspect, provided herein is a method of selecting a Vβ17-expressing cell comprising: contacting a Vβ17-expressing cell with a Vβ17 antibody provided herein; and selecting the Vβ17-expressing cell bound to the Vβ17 antibody. In one aspect, provided herein is a method of capturing a Vβ17-expressing cell comprising: contacting a Vβ17-expressing cell with a Vβ17 antibody provided herein; and capturing the Vβ17-expressing cell bound to the Vβ17 antibody. In one aspect, provided herein is a method of detecting a Vβ17-expressing cell comprising: contacting a Vβ17-expressing cell with a Vβ17 antibody provided herein; and detecting the Vβ17-expressing cell bound to the Vβ17 antibody.

In one aspect, provided herein is a method of enriching a Vβ17-expressing cell comprising: contacting a Vβ17-expressing cell with a Vβ17 antibody provided herein; and enriching the Vβ17-expressing cell based on binding of the Vβ17-expressing cell to the Vβ17 antibody. In one aspect, provided herein is a method of isolating a Vβ17-expressing cell comprising: contacting a Vβ17-expressing cell with a Vβ17 antibody provided herein; and isolating the Vβ17-expressing cell based on binding of the Vβ17-expressing cell to the Vβ17 antibody. In one aspect, provided herein is a method of separating a Vβ17-expressing cell comprising: contacting a Vβ17-expressing cell with a Vβ17 antibody provided herein; and separating the Vβ17-expressing cell based on binding of the Vβ17-expressing cell to the Vβ17 antibody. In one aspect, provided herein is a method of purifying a Vβ17-expressing cell comprising: contacting a Vβ17-expressing cell with a Vβ17 antibody provided herein; and purifying the Vβ17-expressing cell based on binding of the Vβ17-expressing cell to the Vβ17 antibody. In one aspect, provided herein is a method of sorting a Vβ17-expressing cell comprising: contacting a Vβ17-expressing cell with a Vβ17 antibody provided herein; and sorting the Vβ17-expressing cell based on binding of the Vβ17-expressing cell to the Vβ17 antibody. In one aspect, provided herein is a method of selecting a Vβ17-expressing cell comprising: contacting a Vβ17-expressing cell with a Vβ17 antibody provided herein; and selecting the Vβ17-expressing cell based on binding of the Vβ17-expressing cell to the Vβ17 antibody. In one aspect, provided herein is a method of capturing a Vβ17-expressing cell comprising: contacting a Vβ17-expressing cell with a Vβ17 antibody provided herein; and capturing the Vβ17-expressing cell based on binding of the Vβ17-expressing cell to the Vβ17 antibody. In one aspect, provided herein is a method of detecting a Vβ17-expressing cell comprising: contacting a Vβ17-expressing cell with a Vβ17 antibody provided herein; and detecting the Vβ17-expressing cell based on binding of the Vβ17-expressing cell to the Vβ17 antibody.

In certain embodiments of the methods, the Vβ17-expressing cell is a T cell. In some embodiments of the methods, the Vβ17-expressing cell is in a population of cells. In some embodiments of the methods, the Vβ17-expressing cell is in a population of lymphocytes. In some embodiments of the methods, the Vβ17-expressing cell is in a population of T cells. In some embodiments of the methods, the Vβ17-expressing cell is provided as a population of cells. In some embodiments of the methods, the Vβ17-expressing cell is provided as a population of lymphocytes. In some embodiments of the methods, the Vβ17-expressing cell is provided as a population of T cells. In some embodiments of the methods, the Vβ17-expressing cell is provided as a sample comprising a population of cells. In some embodiments of the methods, the Vβ17-expressing cell is provided as a sample comprising a population of lymphocytes. In some embodiments of the methods, the Vβ17-expressing cell is provided as a sample comprising a population of T cells. In some embodiments of the methods, the sample is a blood sample. In some embodiments of the methods, the sample is a tissue sample. In some embodiments of the methods, the sample is a tissue culture sample.

In some embodiments of the methods, the Vβ17 antibody is a multispecific Vβ17 antibody provided herein. In some embodiments of the methods, the Vβ17 antibody is a bispecific Vβ17 antibody provided herein. In some embodiments of the methods, the Vβ17 antibody is a trispecific Vβ17 antibody provided herein. In some embodiments of the methods, the Vβ17 antibody is a quadraspecific Vβ17 antibody provided herein. In certain embodiments, the Vβ17 antibody specifically binds to Vβ17. In one embodiment, the multispecific Vβ17 antibody comprises: (a) a first binding domain that binds Vβ17, and (b) a second binding domain that binds to a second target (*e.g*., a target that is not Vβ17). In one embodiment, the multispecific Vβ17 antibody comprises: (a) a first binding domain that binds Vβ17, and (b) a second binding domain that binds to a second target (*e.g*., a target that is not Vβ17), and (c) a third binding domain that binds to a third target (*e.g*., a target that is not Vβ17). In one embodiment, the multispecific Vβ17 antibody comprises: (a) a first binding domain that binds Vβ17, and (b) a second binding domain that binds to a second target (*e.g*., a target that is not Vβ17), (c) a third binding domain that binds to a third target (*e.g*., a target that is not Vβ17), and (d) a fourth binding domain that binds to a fourth target (*e.g*., a target that is not Vβ17). In one embodiment, the multispecific Vβ17 antibody comprises: (a) a first binding domain that specifically binds Vβ17, and (b) a second binding domain that specifically binds to a second target (*e.g*., a target that is not Vβ17). In one embodiment, the multispecific Vβ17 antibody comprises: (a) a first binding domain that specifically binds Vβ17, and (b) a second binding domain that specifically binds to a second target (*e.g.,* a target that is not Vβ17), and (c) a third binding domain that specifically binds to a third target (*e.g*., a target that is not Vβ17). In one embodiment, the multispecific Vβ17 antibody comprises: (a) a first binding domain that specifically binds Vβ17, and (b) a second binding domain that specifically binds to a second target (*e.g*., a target that is not Vβ17), (c) a third binding domain that specifically binds to a third target (*e.g.*, a target that is not Vβ17), and (d) a fourth binding domain that specifically binds to a fourth target (*e.g*., a target that is not Vβ17).

In specific embodiments of the methods provided herein, the method uses multi-marker detection. In some embodiments, the multi-marker detection uses a multispecific Vβ17 antibody provided herein. In some embodiments, the multi-marker detection uses a bispecific Vβ17 antibody provided herein. In some embodiments, the multi-marker detection uses a trispecific Vβ17 antibody provided herein. In some embodiments, the multi-marker detection uses a quadraspecific Vβ17 antibody provided herein.

In certain embodiments of the methods provided herein, the methods are included as steps in a T cell manufacturing process. In certain embodiments, the cells are CAR-T cells. In certain embodiments of the methods provided herein, the methods are included as steps in a T cell modification process.

In certain embodiments of the methods provided herein, the methods are included as steps in a diagnostic method. In certain embodiments of the methods provided herein, the methods are included as steps in a method to quantify the Vβ17-expressing T cells.

In certain embodiments of the methods provided herein, the method further comprises expanding the enriched, isolated, separated, purified, sorted, selected, captured or detected Vβ17-expressing cells. In certain embodiments, the expanding is in vitro. In certain embodiments, the expanding is in vivo. In certain embodiments of the methods provided herein, the method further comprises growing the enriched, isolated, separated, purified, sorted, selected, captured or detected Vβ17-expressing cells. In certain embodiments, the growing is in vitro. In certain embodiments, the growing is in vivo. In certain embodiments of the methods provided herein, the method further comprises quantifying the enriched, isolated, separated, purified, sorted, selected, captured or detected Vβ17-expressing cells.

### EMBODIMENTS

This invention provides the following non-limiting embodiments.

In one set of embodiments, provided are:
A1. An antibody that binds Vβ17 comprising:
   (1) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:31; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:32;
   (2) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:65; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:66;
   (3) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:99; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 100;
   (4) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:133; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 134;
   (5) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:167; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 168;
   (6) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:201; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:202;
   (7) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:235; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:236;
   (8) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:269; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:270;
   (9) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:303; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:304;
   (10) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:337; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:338;
   (11) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:371; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:372;
   (12) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:405; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:406; or
   (13) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:439; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:440.
A2. The antibody of embodiment A1, wherein the VH CDR1, VH CDR2, VH CDR3, VL CDR1, VL CDR2, and VL CDR3 amino acid sequences are according to the Kabat numbering system.
A3. The antibody of embodiment A1, wherein the VH CDR1, VH CDR2, VH CDR3, VL CDR1, VL CDR2, and VL CDR3 amino acid sequences are according to the Chothia numbering system.
A4. The antibody of embodiment A1, wherein the VH CDR1, VH CDR2, VH CDR3, VL CDR1, VL CDR2, and VL CDR3 amino acid sequences are according to the AbM numbering system.
A5. The antibody of embodiment A1, wherein the VH CDR1, VH CDR2, VH CDR3, VL CDR1, VL CDR2, and VL CDR3 amino acid sequences are according to the Contact numbering system.
A6. The antibody of embodiment A1, wherein the VH CDR1, VH CDR2, VH CDR3, VL CDR1, VL CDR2, and VL CDR3 amino acid sequences are according to the IMGT numbering system.
A7. The antibody of any one of embodiments A1 to A6, wherein the antibody is a humanized antibody.
A8. The antibody of any one of embodiments A1 to A7, wherein the antibody is an IgG antibody.
A9. The antibody of embodiment A8, wherein the IgG antibody is an IgG1, IgG2, IgG3, or IgG4 antibody.
A10. The antibody of any one of embodiments A1 to A9, wherein the antibody comprises a kappa light chain.
A11. The antibody of any one of embodiments A1 to A9, wherein the antibody comprises a lambda light chain.
A12. The antibody of any one of embodiments A1 to A11, wherein the antibody is a monoclonal antibody.
A13. The antibody of any one of embodiments A1 to A12, wherein the antibody binds a Vβ17 antigen.
A14. The antibody of any one of embodiments A1 to A12, wherein antibody binds a Vβ17 epitope.
A15. The antibody of any one of embodiments A1 to A14, wherein the antibody specifically binds to Vβ17.
A16. The antibody of any one of embodiments A1 to A15, wherein the VH CDR1, VH CDR2, VH CDR3, VL CDR1, VL CDR2 and VL CDR3 form a binding site for an antigen of the Vβ17.
A17. The antibody of any one of embodiments A1 to A15, wherein the VH CDR1, VH CDR2, VH CDR3, VL CDR1, VL CDR2 and VL CDR3 form a binding site for an epitope of the Vβ17.
A18. The antibody of any one of embodiments A1 to A17, wherein the Vβ17 is present on the surface of a T cell.
A19. The antibody of any one of embodiments A1 to A18, wherein the antibody is multivalent.
A20. The antibody of embodiment A19, wherein the antibody is capable of binding at least three antigens.
A21. The antibody of embodiment A19, wherein the antibody is capable of binding at least four antigens.
A22. The antibody of embodiment A19, wherein the antibody is capable of binding at least five antigens.
A23. The antibody of any one of embodiments A1 to A22, wherein the antibody is a multispecific antibody.
A24. The antibody of embodiment A23, wherein the antibody is a bispecific antibody.
A25. The antibody of embodiment A23, wherein the antibody is a trispecific antibody.
A26. The antibody of embodiment A23, wherein the antibody is a quadraspecific antibody.
A27. A nucleic acid encoding the antibody of any one of embodiments A1 to A26.
A28 A vector comprising the nucleic acid of embodiment A27.
A29. A host cell comprising the vector of embodiment A28.
A30. A kit comprising the vector of embodiment A28 and packaging for the same.
A31. A kit comprising the antibody of any one of embodiments A1 to A26 and packaging for the same.
A32. A method of directing a T cell expressing Vβ17 to a target cell, comprising contacting the Vβ17 antibody of any one of embodiments A1 to A26 with the target cell, wherein the second target is present on the surface of the target cell, and wherein the contacting directs the T cell to the target cell.
A33. A method of inhibiting the growth or proliferation of a target cell, comprising contacting the Vβ17 antibody of any one of embodiments A1 to A26 with the target cell having the second target present on the surface of the target cell, wherein the contacting is in the presence of a T cell expressing the Vβ17, and wherein the contacting results in the inhibition of the growth or proliferation of the target cell.
A34. A method of eliminating a target cell in a subject, comprising contacting the Vβ17 antibody of any one of embodiments A1 to A26 with the target cell having the second target present on the surface of the target cell, wherein the contacting is in the presence of a T cell expressing the Vβ17, and wherein the contacting results in the elimination of the target cell.

In one set of embodiments, provided are:
B1. A pharmaceutical composition comprising an antibody that binds Vβ17, and a pharmaceutically acceptable carrier.
B2. The pharmaceutical composition of embodiment B1, wherein the antibody that binds Vβ17 comprises
   (1) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:31; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:32;
   (2) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:65; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:66;
   (3) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:99; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 100;
   (4) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:133; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 134;
   (5) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:167; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 168;
   (6) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:201; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:202;
   (7) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:235; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:236;
   (8) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:269; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:270;
   (9) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:303; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:304;
   (10) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:337; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:338;
   (11) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:371; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:372;
   (12) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:405; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:406; or
   (13) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:439; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:440.
B3. The pharmaceutical composition of embodiment B2, wherein the VH CDR1, VH CDR2, VH CDR3, VL CDR1, VL CDR2, and VL CDR3 amino acid sequences are according to the Kabat numbering system.
B4. The pharmaceutical composition of embodiment B2, wherein the VH CDR1, VH CDR2, VH CDR3, VL CDR1, VL CDR2, and VL CDR3 amino acid sequences are according to the Chothia numbering system.
B5. The pharmaceutical composition of embodiment B2, wherein the VH CDR1, VH CDR2, VH CDR3, VL CDR1, VL CDR2, and VL CDR3 amino acid sequences are according to the AbM numbering system.
B6. The pharmaceutical composition of embodiment B2, wherein the VH CDR1, VH CDR2, VH CDR3, VL CDR1, VL CDR2, and VL CDR3 amino acid sequences are according to the Contact numbering system.
B7. The pharmaceutical composition of embodiment B2, wherein the VH CDR1, VH CDR2, VH CDR3, VL CDR1, VL CDR2, and VL CDR3 amino acid sequences are according to the IMGT numbering system.
B8. The pharmaceutical composition of any one of embodiments B1 to B7, wherein the antibody is a humanized antibody.
B9. The pharmaceutical composition of any one of embodiments B1 to B8, wherein the antibody is an IgG antibody.
B10. The pharmaceutical composition of embodiment B9, wherein the IgG antibody is an IgG1, IgG2, IgG3, or IgG4 antibody.
B11. The pharmaceutical composition of any one of embodiments B1 to B10, wherein the antibody comprises a kappa light chain.
B12. The pharmaceutical composition of any one of embodiments B1 to B11, wherein the antibody comprises a lambda light chain.
B13. The pharmaceutical composition of any one of embodiments B1 to B12, wherein the antibody is a monoclonal antibody.
B14. The pharmaceutical composition of any one of embodiments B1 to B13, wherein the antibody binds a Vβ17 antigen.
B15. The pharmaceutical composition of any one of embodiments B1 to B13, wherein antibody binds a Vβ17 epitope.
B16. The pharmaceutical composition of any one of embodiments B1 to B 15, wherein the antibody specifically binds to Vβ17.
B17. The pharmaceutical composition of any one of embodiments B1 to B16, wherein the VH CDR1, VH CDR2, VH CDR3, VL CDR1, VL CDR2 and VL CDR3 form a binding site for an antigen of the Vβ17.
B18. The pharmaceutical composition of any one of embodiments B1 to B16, wherein the VH CDR1, VH CDR2, VH CDR3, VL CDR1, VL CDR2 and VL CDR3 form a binding site for an epitope of the Vβ17.
B19. The pharmaceutical composition of any one of embodiments B1 to B18, wherein the Vβ17 is present on the surface of a T cell.
B20. The pharmaceutical composition of any one of embodiments B1 to B19, wherein the antibody is multivalent.
B21. The pharmaceutical composition of embodiment B20, wherein the antibody is capable of binding at least three antigens.
B22. The pharmaceutical composition of embodiment B20, wherein the antibody is capable of binding at least four antigens.
B23. The pharmaceutical composition of embodiment B20, wherein the antibody is capable of binding at least five antigens.
B24. The pharmaceutical composition of any one of embodiments B1 to B23, wherein the antibody is a multispecific antibody.
B25. The pharmaceutical composition of embodiment B24, wherein the antibody is a bispecific antibody.
B26. The pharmaceutical composition of embodiment B24, wherein the antibody is a trispecific antibody.
B27. The pharmaceutical composition of embodiment B24, wherein the antibody is a quadraspecific antibody.
B28. A method of producing the pharmaceutical composition of any one of embodiments B 1 to B27, comprising combining the antibody with a pharmaceutically acceptable carrier to obtain the pharmaceutical composition.

In one set of embodiments, provided are:
C1. A method of treating a disease in a subject, comprising administering an effective amount an antibody that binds Vβ17 to the subject, wherein the disease is caused all or in part by a target cell having the second target present on the surface of the target cell.
C2. The method of embodiment C1, wherein the antibody that binds Vβ17 comprises:
   (1) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:31; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:32;
   (2) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:65; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:66;
   (3) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:99; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 100;
   (4) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:133; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 134;
   (5) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:167; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO: 168;
   (6) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:201; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:202;
   (7) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:235; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:236;
   (8) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:269; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:270;
   (9) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:303; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:304;
   (10) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:337; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:338;
   (11) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:371; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:372;
   (12) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:405; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:406; or
   (13) (i) a VH comprising a VH CDR1, a VH CDR2, and a VH CDR3 having an amino acid sequence of a VH CDR1, a VH CDR2, and a VH CDR3, respectively, of a VH having an amino acid sequence of SEQ ID NO:439; and (ii) a VL comprising a VL CDR1, a VL CDR2, and a VL CDR3 having an amino acid sequence of a VL CDR1, a VL CDR2, and a VL CDR3, respectively, of a VL having an amino acid sequence of SEQ ID NO:440.
C3. The method of embodiment C2, wherein the VH CDR1, VH CDR2, VH CDR3, VL CDR1, VL CDR2, and VL CDR3 amino acid sequences are according to the Kabat numbering system.
C4. The method of embodiment C2, wherein the VH CDR1, VH CDR2, VH CDR3, VL CDR1, VL CDR2, and VL CDR3 amino acid sequences are according to the Chothia numbering system.
C5. The method of embodiment C2, wherein the VH CDR1, VH CDR2, VH CDR3, VL CDR1, VL CDR2, and VL CDR3 amino acid sequences are according to the AbM numbering system.
C6. The method of embodiment C2, wherein the VH CDR1, VH CDR2, VH CDR3, VL CDR1, VL CDR2, and VL CDR3 amino acid sequences are according to the Contact numbering system.
C7. The method of embodiment C2, wherein the VH CDR1, VH CDR2, VH CDR3, VL CDR1, VL CDR2, and VL CDR3 amino acid sequences are according to the IMGT numbering system.
C8. The method of any one of embodiments C1 to C7, wherein the antibody is a humanized antibody.
C9. The method of any one of embodiments C1 to C8, wherein the antibody is an IgG antibody.
C10. The method of embodiment C9, wherein the IgG antibody is an IgG1, IgG2, IgG3, or IgG4 antibody.
C11. The method of any one of embodiments C1 to C10, wherein the antibody comprises a kappa light chain.
C12. The method of any one of embodiments C1 to C10, wherein the antibody comprises a lambda light chain.
C13. The method of any one of embodiments C1 to C12, wherein the antibody is a monoclonal antibody.
C14. The method of any one of embodiments C1 to C13, wherein the antibody binds a Vβ17 antigen.
C15. The method of any one of embodiments C1 to C13, wherein antibody binds a Vβ17 epitope.
C16. The method of any one of embodiments C1 to C15, wherein the antibody specifically binds to Vβ17.
C17. The method of any one of embodiments C1 to C16, wherein the VH CDR1, VH CDR2, VH CDR3, VL CDR1, VL CDR2 and VL CDR3 form a binding site for an antigen of the Vβ17.
C18. The method of any one of embodiments C1 to C16, wherein the VH CDR1, VH CDR2, VH CDR3, VL CDR1, VL CDR2 and VL CDR3 form a binding site for an epitope of the Vβ17.
C19. The method of any one of embodiments C1 to C18, wherein the Vβ17 is present on the surface of a T cell.
C20. The method of any one of embodiments C1 to C19, wherein the antibody is multivalent.
C21. The method of embodiment C20, wherein the antibody is capable of binding at least three antigens.
C22. The method of embodiment C20, wherein the antibody is capable of binding at least four antigens.
C23. The method of embodiment C20, wherein the antibody is capable of binding at least five antigens.
C24. The method of any one of embodiments C1 to C23, wherein the antibody is a multispecific antibody.
C25. The method of embodiment C24, wherein the antibody is a bispecific antibody.
C26. The method of embodiment C24, wherein the antibody is a trispecific antibody.
C27. The method of embodiment C24, wherein the antibody is a quadraspecific antibody.
C28. The method of any one of embodiments C1 to C27, wherein the subject is a human.
C29. The method of any one of embodiments C1 to C27, wherein the subject is a subject in need thereof.
C30. A method of activating a T cell expressing Vβ17, comprising contacting the T cell with the antibody of any one of embodiments C1 to C29.
C31. The method of embodiment C30, wherein the contacting results in an increase in CD69, CD25, and/or Granzyme B expression, as compared to a control T cell expressing Vβ17.
C32. A process for making an antibody that binds to more than one target molecule, the process comprising: a step for performing a function of obtaining a first binding domain that binds to Vβ17 present on a T cell; a step for performing a function of obtaining a second binding domain that binds to a second target on the surface of a target cell; and a step for performing a function of providing an antibody that binds to Vβ17 present on a T cell and a second target on the surface of a target cell.
C33. The process of embodiment C32, wherein the step for performing a function of obtaining a second binding domain that binds to a second target on the surface of a target cell is repeated n times, and further comprising n steps for performing a function of providing a first binding domain that binds to Vβ17 present on a T cell and n number of target molecules, wherein n is at least 2.

Provided in the Examples herein are exemplary antibodies that bind to Vβ17.

Exemplary binding agents that bind to Vβ17 are provided herein, for example in the Examples, as well as Tables 1-6.

Particular embodiments of this invention are described herein. Upon reading the foregoing description, variations of the disclosed embodiments may become apparent to individuals working in the art, and it is expected that those skilled artisans may employ such variations as appropriate. Accordingly, it is intended that the invention be practiced otherwise than as specifically described herein, and that the invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context. A number of embodiments of the invention have been described. Nevertheless, it will be understood that various modifications may be made without departing from the spirit and scope of the invention. Accordingly, the descriptions in the Examples section are intended to illustrate but not limit the scope of invention described in the claims.

### EXAMPLES

### EXAMPLE 1: ANTIBODIES THAT BIND V017

### 1.1: Identification of Vβ17 monoclones

A cell based approach was used to identify Vβ17 specific monoclones.

Mice were tolerized with NS0-WT cells in neonatal stage and then immunized with TCRVα10.2*Vβ17.2 expressing NS0 cell line weekly for 3 boosters.

High titer was assessed by checking specificity towards Vβ17.2 using 3 variants of SKW3 cell line [SKW3-1 (Vα10*Vβ17), SKW3-2 (Vα12*Vβ17), SKW3-3 (Vα10*Vβ7/9)].

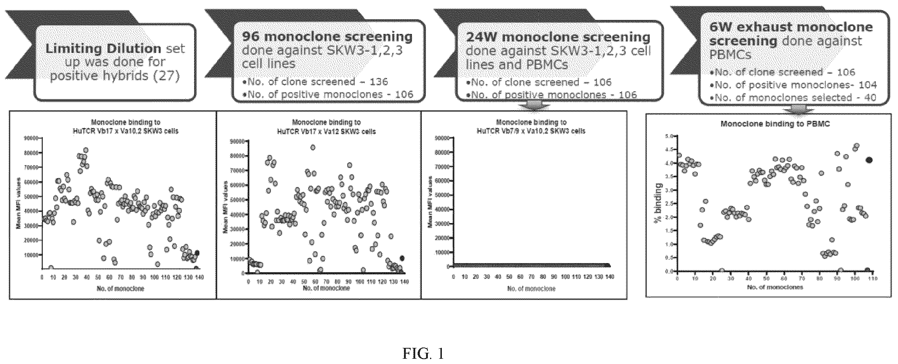

Several monoclones binding to Vβ17 were identified by flow cytometry using different cell lines and primary cells as shown in **FIG. 1****.** Anti TCR Vβ17-PE Ab, Beckman Coulter, Cat: IM2048 was used as a positive control. Goat anti mouse IgG PE, Southern Biotech, Cat: 1030-09, E2518-RJ69Y was used as a negative control.

### EXAMPLE 2: EVALUATION OF Vβ17 ANTIBODY PROPERTIES

### 2.1: Proliferation and activation

To test proliferation of Vβ17+ cells, 96 well ELISA plate was coated with Vβ17 antibodies or Control Antibody (B17B21) at 10ug/ml and incubated for 24 hour. PBMC were thawed and labeled with Cell trace violet to track the proliferation. 0.5M PBMC cells/well/200 ul of 10% RPMI media (without IL-2) were incubated for 24 hour in a CO₂ incubator. Cells were transferred from 96 well to 48 well culture plate with 200ul of 10% RPMI media (no IL-2). The CTV dilution was checked for proliferation and activation status using markers: CD25, CD69 and CD71.

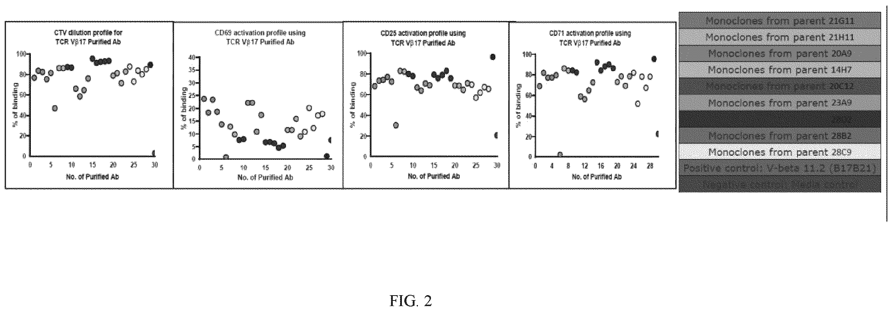

Monoclones from 7 parents showed good agonistic properties as shown in **FIG. 2****.** Selected 26 monoclones with good agonistic properties were taken for purification.

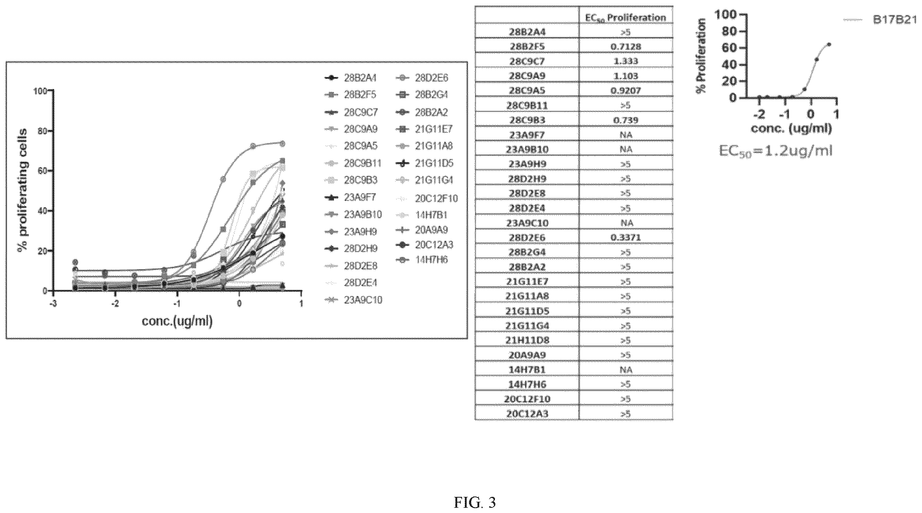

Differential proliferation kinetics were observed for the 26 purified antibodies. As shown in **FIG. 3****,** monoclones from parents 28B2, 28C9 and 28D2 showed good proliferation and were taken for further assays.

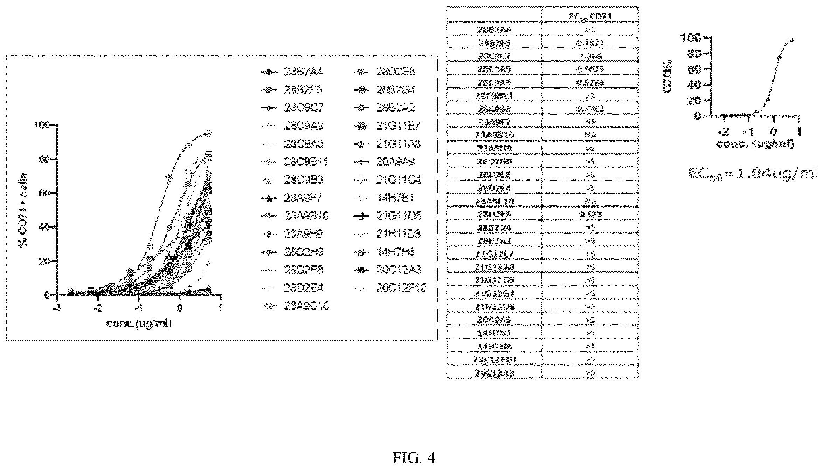

Differential CD71 upregulation were observed for the 26 purified antibodies. As shown in **FIG. 4****,** monoclones from parents 28B2, 28C9 and 28D2 showed good CD71 upregulation and were taken for further assays.

### 2.2: Vβ17 binding

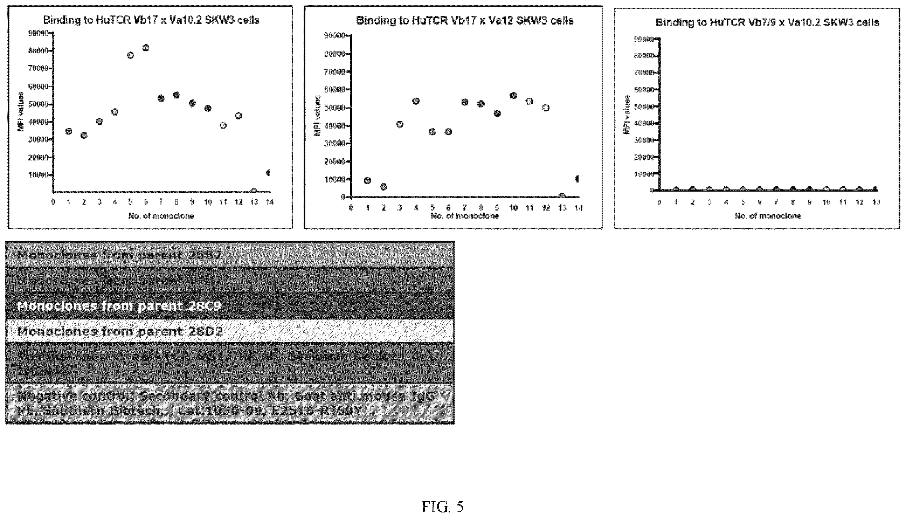

The 10 best agonistic monoclones binding to Vβ17 was assessed using SKW3 cell line variants. As shown in **FIG. 5****,** the 10 purified antibodies belonging to 3 different parents bound well to SKW3-1 and 2 cell lines and not to SKW3-3 cells, which does not express Vβ17. Purified antibodies of 14H7 parent did not bind well on SKW3-2 cell line and was included as a negative control.

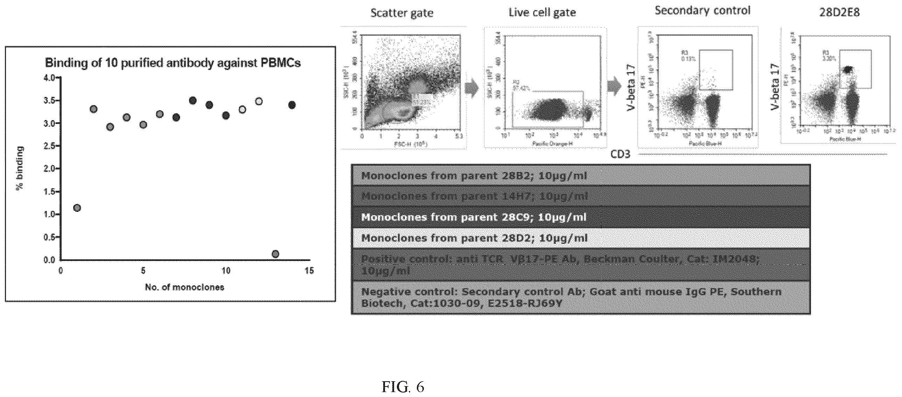

The 10 selected purified monoclones binding to PBMCs was assessed. As shown in **FIG. 6****,** the 10 purified antibodies belonging to 3 different parents bound well to T cells on PBMCs.

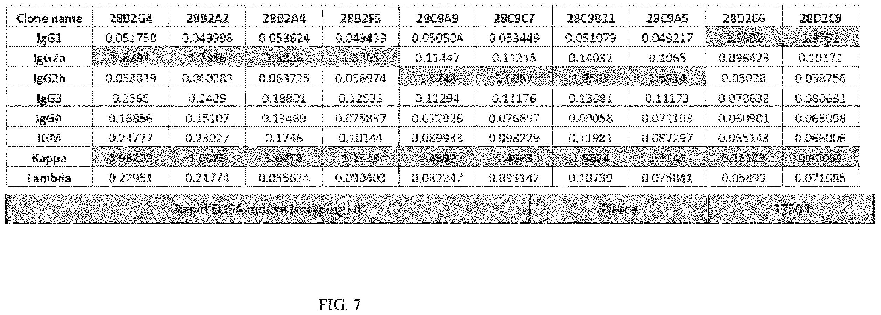

These 10 shortlisted monoclones differ in isotypes based on their hybrid parent as shown in **FIG. 7****.** The purified monoclonal antibodies from 3 different parent have different isotypes: 28D2 has IgG1, 28B2 has IgG2a and 28C9 has IgG2b.

### 2.3: Vβ17 antibody internalization

Internalization Assay used to screen the 10 shortlisted antibodies. MMAF and antibodies was added to 5000 cells/well/50 ul each at 50nM concentration, with Ab control, MMAF control and at 3-fold dilution till 7^{th} points. MMAF antibody was incubated with cells for 20 mins and assay was run in duplicates. The plate was incubated for 96 hr at 37 °C following which Cell Titre Glo was added to the plates. Luminescence was read in TECAN SPARK and % viability was calculated using the formula MMAF+Ab/MMAF X 100. Data was plotted in GraphPad Prism.

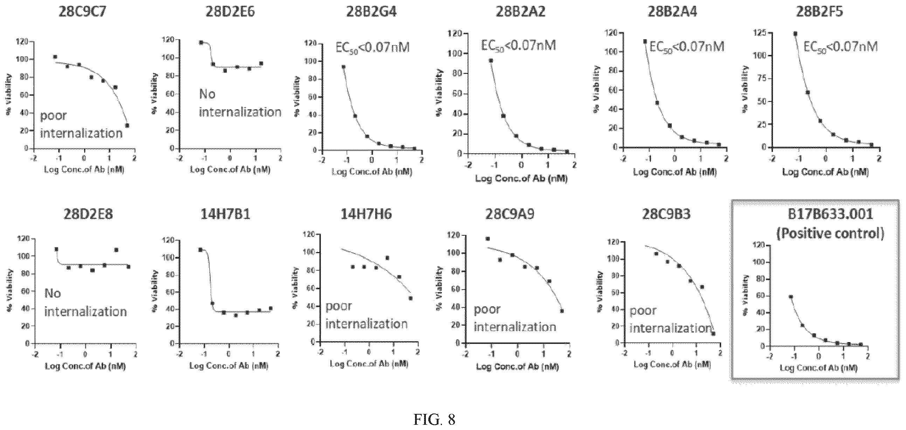

As shown in **FIG. 8****,** monoclones from 28B2 parent show good internalization. Monoclones from parent 28C9 which had very good agonistic property, show poor internalization. Monoclones from parent 28D2 and 14H7 show no internalization.

### EXAMPLE 3: EPITOPE BINNING OF Vβ17 MONOCLONES

Epitope binning helped segregate the final monoclones into separate epitope recognition groups and the antibodies recognizing different epitopes from that of the commercial antibody. The distinction in epitope recognition was supported by diversity in V-gene sequences.

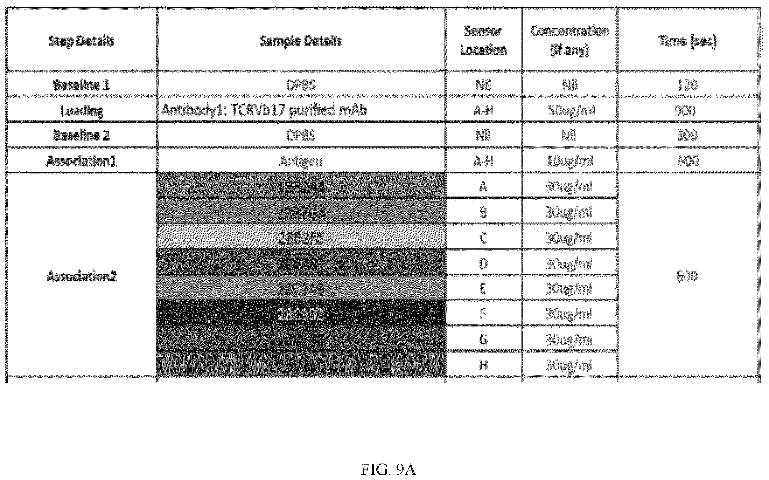

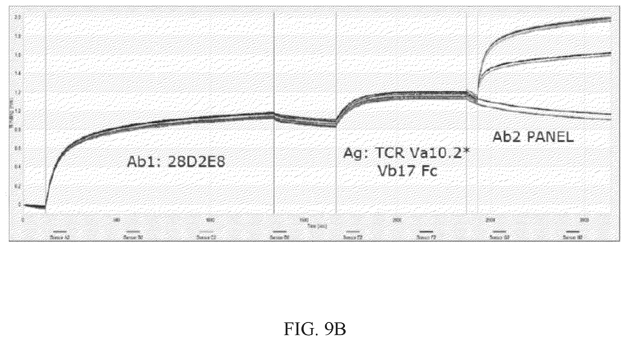

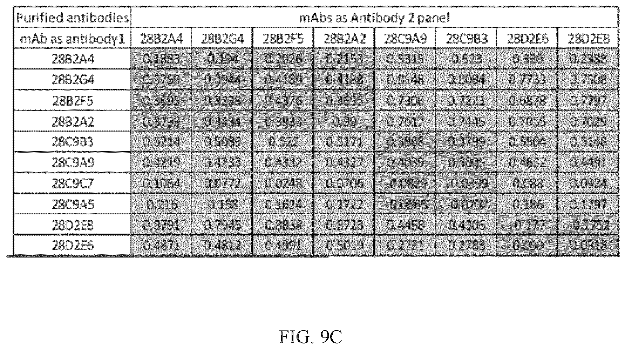

Epitope binning matrix for 10 shortlisted antibodies using Octet RED96 showed 3 separate specificities as shown in **FIGS. 9A-9C****.** The Antibody 1 were loaded onto anti-mouse IgG Fc capture (AMC) sensors and then made to bind to the antigen (TCRVα10.2*Vβ17 huFc), before running them on Antibody 2 panel. Purified monoclonal antibodies from 3 different parents binned into 3 different groups.

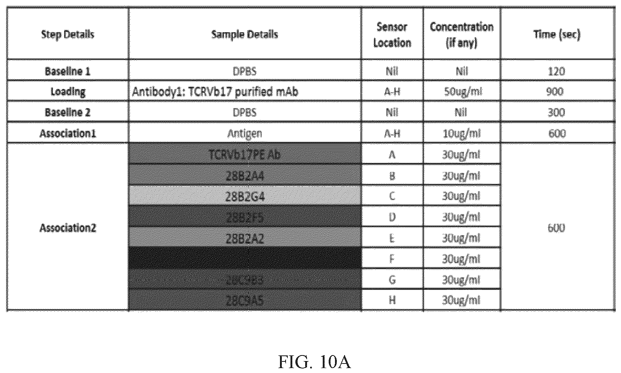

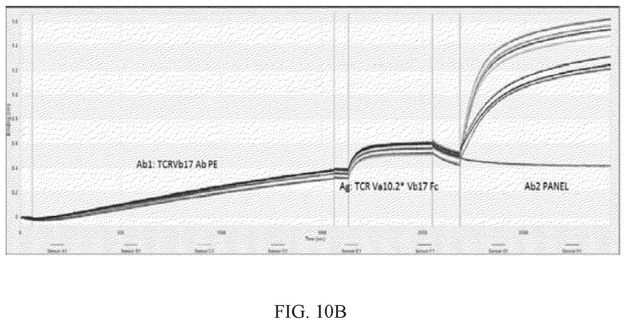

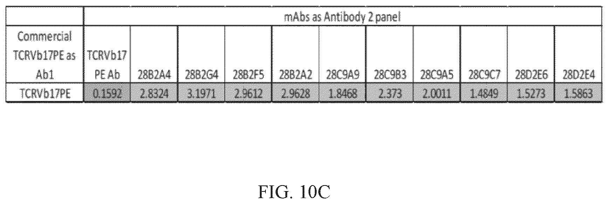

Furthermore, the 10 shortlisted antibodies bin differently compared to the commercial positive control antibody as was assessed using Octet RED96. As shown in **FIGS. 10A-10C****,** positive control antibody TCRVb 17-PE (Beckman coulter, Catalogue No: IM2048) bin differently from the 10 shortlisted antibodies.

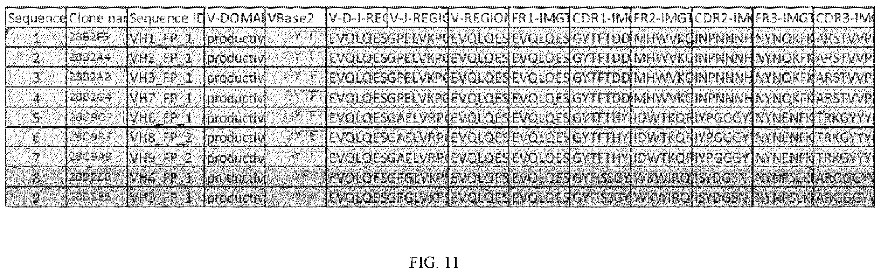

As shown in **FIG. 11****,** monoclones from the same parent have the same VH sequence. Heavy chain sequences of monoclones belonging to 3 different hybrids were diverse from each other.

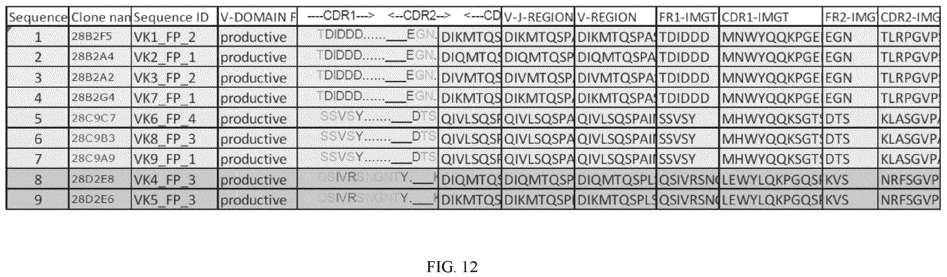

As shown in **FIG. 12****,** monoclones from the same parent have the same VK sequence. Light chain sequences of monoclones belonging to 3 different hybrids were diverse from each other.

### EXAMPLE 4: FURTHER CHARACTERIZATION OF OTHER Vβ17 ANTIBODIES

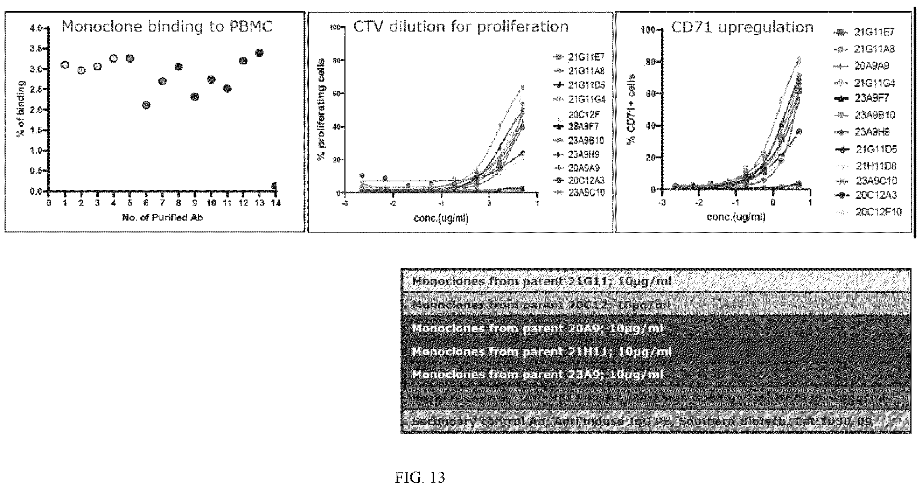

As shown in **FIG. 13****,** 12 other antibodies were tested for their proliferation and activation profiles. These 12 additional antibodies had moderate agonistic properties and good PBMC binding and were taken forward for Epitope binning and Cell based competition assays.

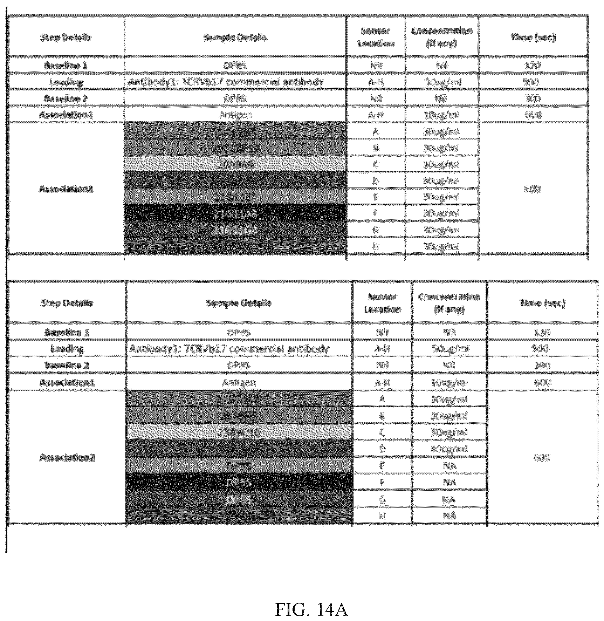

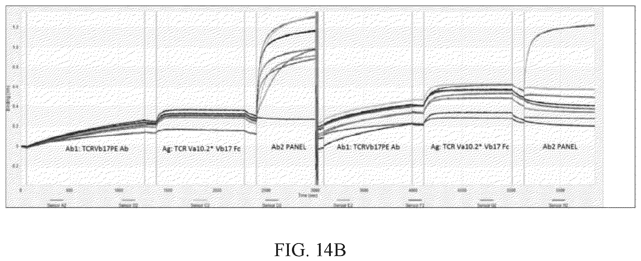

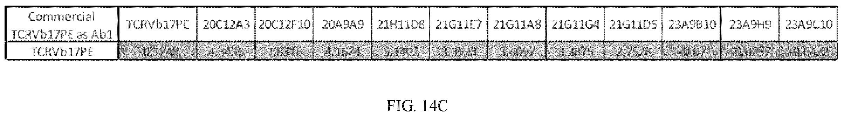

As shown in **FIGS. 14A-14C****,** monoclones from one hybrid parent 23A9 binned similarly with positive control antibody (TCRVb17-PE).

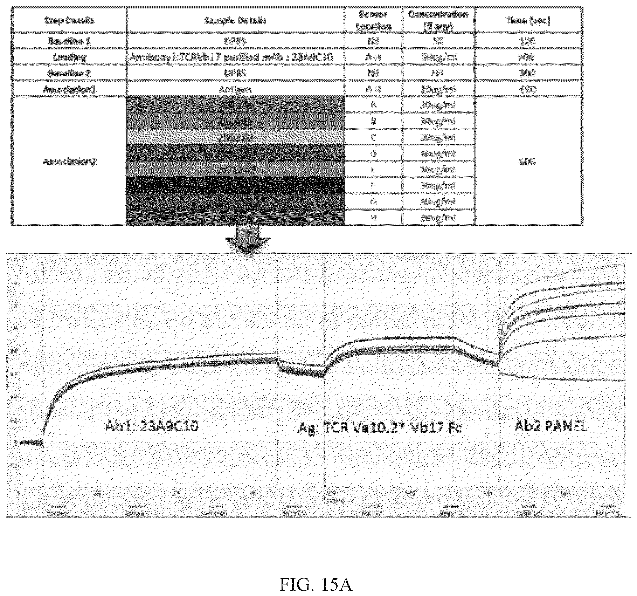

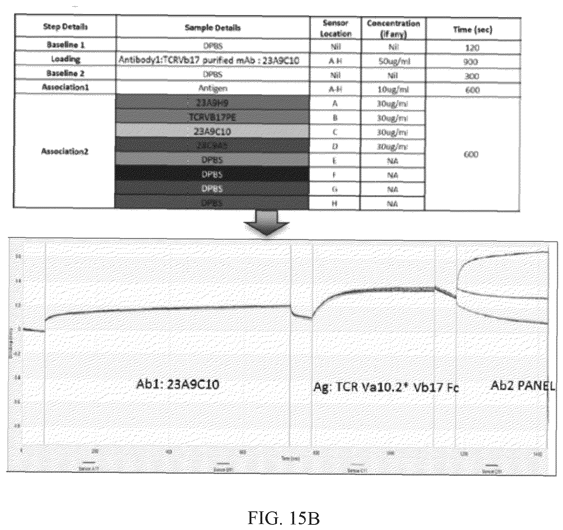

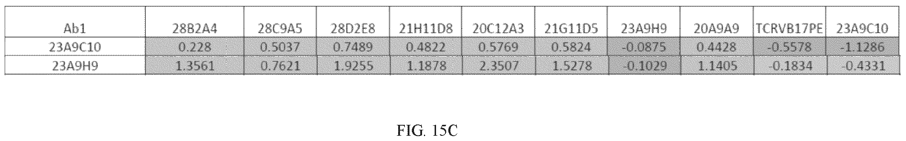

As shown in **FIGS. 15A-15C****,** Monoclones from 23A9 and commercial antibody bin similarly to each other, but differently to monoclones from other hybrid parents.

### EXAMPLE 5: CELL BASED COMPETITION ASSAY OF Vβ17 ANTIBODIES

Cells were treated with Live/dead dye for 15 min at 4°C followed by seeding of cells in 96 well plate. Cells were treated with different concentrations of humanized Vb17 antibody

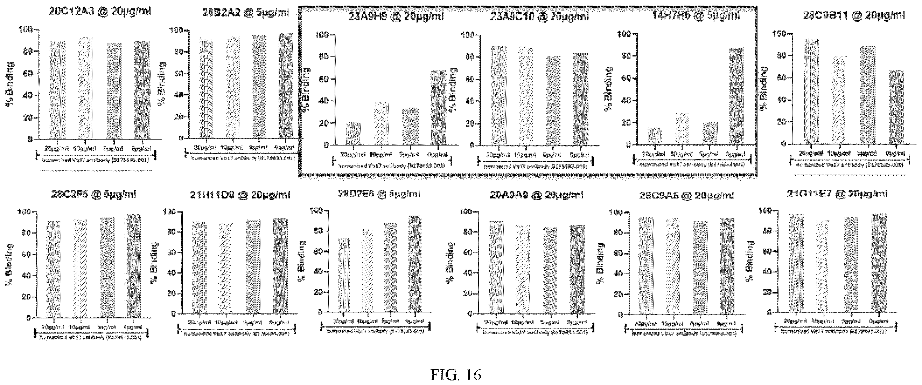

(B 17B633.001) and incubated for 40 min at 4°C. Cells were washed and incubated with 20ug/ml of purified mouse Vb17 antibodies for 40 min at 4°C. Cells were stained with secondary antibody, washed and read on Novocyte. As shown in **FIG. 16****,** most monoclones did not compete with the commercial antibody to bind to cells expressing Vβ17.

**Table 7. Antibody Binding to TCR Vβ17**

| **SEQ ID NO.** | **Clone Name** | **PBMC binding (%)** | **HuTCR Vb17 x Va10.2 (SKW3_1) MFI** | **HuTCR Vb17 x Va12 (SKW3-2) MFI** | **HuTCR Vb7/9 x Va10.2 (SKW3-3) MFI** | **Cell Internalization (EC50)** | **Proliferation (EC50, ug/ml)** | **CD71 Activation (EC50)** | **Selection** |
|---|---|---|---|---|---|---|---|---|---|
| | Positive control Ab (commercial Vb17PE (Beckman coulter, IM-2048) | 3.40% | 11337 | 10250 | 610 | <0.07nM | 1.2 | 1.04 | positive control antibody here the B17B21 humanized |
| | Secondary Ab control (Goat anti-mouse IgG PE) | 0.13% | 486 | 472 | 426 | | | | |
| 1 | 20A9A9 | 2.70% | 39502 | 43146 | 448 | 0.29nM | >5 | >5 | No |
| 2 | 20C12A3 | 2.12% | 59136 | 85598 | 455 | 0.06nM | >5 | >5 | No |
| 3 | 21G11A8 | 2.96% | 48723 | 38927 | 532 | poor internalization | >5 | >5 | YES |
| 4 | 21G11D5 | 3.06% | 47493 | 56261 | 427 | poor internalization | 2.0 | >5 | YES |
| 5 | 23 A9B 10 | 2.74% | 10062 | 6595 | 507 | 15.4nM | no proliferation | no upregulation | No |
| 6 | 23A9C10 | 3.20% | 7655 | 3210 | 458 | poor internalization | no proliferation | no upregulation | No |
| 7 | 23A9F7 | 2.32% | 8596 | 3403 | 466 | poor internalization | no proliferation | no upregulation | No |
| 8 | 28B2A2 | 3.13% | 77448 | 36541 | 434 | <0.07nM | poor proliferation | >5 | No |
| 9 | 28B2A4 | 2.97% | 45741 | 53757 | 472 | <0.07nM | >5 | >5 | YES |
| 10 | 28B2F5 | 3.20% | 81791 | 36648 | 438 | <0.07nM | 0.7 | 0.8 | YES |
| 11 | 28C9A5 | 3.27% | 55206 | 56857 | 438 | poor internalization | 0.9 | 0.9 | YES |
| 12 | 28D2E6 | 3.27% | 38042 | 53763 | 441 | no internalization | 0.3 | 0.3 | YES |
| 13 | 28D2E8 | 3.30% | 43575 | 49957 | 452 | no internalization | poor proliferation | >5 | No |

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the present description.
The invention further provides the following non-limiting numbered embodiments:
1. An antibody that binds Vβ17 comprising:
   (1) (i) a heavy chain variable region (VH) comprising a VH complementarity determining region 1 (CDR1), a VH CDR2, and a VH CDR3 having the amino acid sequences of a VH CDR1, a VH CDR2, and a VH CDR3, respectively, of a VH having the amino acid sequence of SEQ ID NO:31; and (ii) a light chain variable region (VL) comprising a VL CDR1, a VL CDR2, and a VL CDR3 having the amino acid sequences of a VL CDR1, a VL CDR2, and a VL CDR3, respectively, of a VL having the amino acid sequence of SEQ ID NO:32;
   (2) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:65; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:66;
   (3) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:99; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO: 100;
   (4) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO: 133; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO: 134;
   (5) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO: 167; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO: 168;
   (6) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:201; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:202;
   (7) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:235; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:236;
   (8) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:269; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:270;
   (9) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:303; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:304;
   (10) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:337; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:338;
   (11) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:371; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:372;
   (12) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:405; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:406; or
   (13) (i) a VH comprising a VH CDR1, a VH CDR2, and a VH CDR3 having the amino acid sequence of a VH CDR1, a VH CDR2, and a VH CDR3, respectively, of a VH having the amino acid sequence of SEQ ID NO:439; and (ii) a VL comprising a VL CDR1, a VL CDR2, and a VL CDR3 having the amino acid sequence of a VL CDR1, a VL CDR2, and a VL CDR3, respectively, of a VL having the amino acid sequence of SEQ ID NO:440.
2. The antibody of embodiment 1, wherein the VH CDR1, VH CDR2, VH CDR3, VL CDR1, VL CDR2, and VL CDR3 amino acid sequences are according to the Kabat numbering system.
3. The antibody of embodiment 1, wherein the VH CDR1, VH CDR2, VH CDR3, VL CDR1, VL CDR2, and VL CDR3 amino acid sequences are according to the Chothia numbering system.
4. The antibody of embodiment 1, wherein the VH CDR1, VH CDR2, VH CDR3, VL CDR1, VL CDR2, and VL CDR3 amino acid sequences are according to the AbM numbering system.
5. The antibody of embodiment 1, wherein the VH CDR1, VH CDR2, VH CDR3, VL CDR1, VL CDR2, and VL CDR3 amino acid sequences are according to the Contact numbering system.
6. The antibody of embodiment 1, wherein the VH CDR1, VH CDR2, VH CDR3, VL CDR1, VL CDR2, and VL CDR3 amino acid sequences are according to the IMGT numbering system.
7. The antibody of any one of embodiments 1 to 6, wherein the antibody is a humanized antibody.
8. The antibody of any one of embodiments 1 to 7, wherein the antibody is an IgG antibody.
9. The antibody of embodiment 8, wherein the IgG antibody is an IgG1, IgG2, IgG3, or IgG4 antibody.
10. The antibody of any one of embodiments 1 to 9, wherein the antibody comprises a kappa light chain.
11. The antibody of any one of embodiments 1 to 9, wherein the antibody comprises a lambda light chain.
12. The antibody of any one of embodiments 1 to 11, wherein the antibody is a monoclonal antibody.
13. The antibody of any one of embodiments 1 to 12, wherein the antibody binds a Vβ17 antigen.
14. The antibody of any one of embodiments 1 to 12, wherein antibody binds a Vβ17 epitope.
15. The antibody of any one of embodiments 1 to 14, wherein the antibody specifically binds to Vβ17.
16. The antibody of any one of embodiments 1 to 15, wherein the antibody is multivalent.
17. The antibody of embodiment 16, wherein the antibody is capable of binding at least three antigens.
18. The antibody of embodiment 16, wherein the antibody is capable of binding at least four antigens.
19. The antibody of embodiment 16, wherein the antibody is capable of binding at least five antigens.
20. The antibody of any one of embodiments 1 to 19, wherein the antibody is a multispecific antibody.
21. The antibody of embodiment 20, wherein the antibody is a bispecific antibody.
22. The antibody of embodiment 20, wherein the antibody is a trispecific antibody.
23. The antibody of embodiment 20, wherein the antibody is a quadraspecific antibody.
24. A nucleic acid encoding the antibody of any one of embodiments 1 to 23.
25. A vector comprising the nucleic acid of embodiment 24.
26. A host cell comprising the vector of embodiment 25.
27. A kit comprising the vector of embodiment 25 and instructions for using the vector.
28. A kit comprising the antibody of any one of embodiments 1 to 23 and instructions for using the antibody.
29. A pharmaceutical composition comprising the antibody of any one of embodiments 1 to 23 and a pharmaceutically acceptable carrier.
30. A method of producing the pharmaceutical composition of embodiment 29, comprising combining the antibody with a pharmaceutically acceptable carrier to obtain the pharmaceutical composition.
31. A method of activating a T cell expressing Vβ17, comprising contacting the antibody of any one of embodiments 1 to 23 with the T cell expressing Vβ17.
32. The method of embodiment 31, wherein the contacting increases CD69, CD25, and/or Granzyme B expression, as compared to a T cell expressing Vβ17 that is not contacted with the antibody of any one of embodiments 1 to 23.
33. A process for making an antibody that binds to more than one target molecule, the process comprising: a step for performing a function of obtaining a first binding domain that binds to Vβ17 present on a T cell; a step for performing a function of obtaining a second binding domain that binds to a second target on the surface of a target cell; and a step for performing a function of providing an antibody that binds to Vβ17 present on a T cell and a second target on the surface of a target cell.
34. The process of embodiment 33, wherein the step for performing a function of obtaining a second binding domain that binds to a second target on the surface of a target cell is repeated n times, and further comprising n steps for performing a function of providing a first binding domain that binds to Vβ17 present on a T cell and n number of target molecules, wherein n is at least 2.
35. A process for making the antibody of any one of embodiments 1 to 23, comprising growing the host cell of embodiment 26 under conditions to produce the antibody.
36. The process for making of embodiment 35, wherein the process further comprises isolating the antibody.
37. A method of directing a T cell expressing Vβ17 to a target cell, comprising contacting the antibody of any one of embodiments 1 to 23 with the target cell, wherein a second target is present on the surface of the target cell, and wherein the contacting directs the T cell to the target cell.
38. A method of inhibiting the growth or proliferation of a target cell, comprising contacting the antibody of any one of embodiments 1 to 23 with the target cell having a second target present on the surface of the target cell, wherein the contacting is in the presence of a T cell expressing the Vβ17, and wherein the contacting results in inhibition of the growth or proliferation of the target cell.
39. A method of eliminating a target cell in a subject, comprising contacting the antibody of any one of embodiments 1 to 23 with the target cell having a second target present on the surface of the target cell, wherein the contacting is in the presence of a T cell expressing the Vβ17, and wherein the contacting results in the elimination of the target cell.
40. A method of treating a disease in a subject, comprising administering an effective amount the antibody of any one of embodiments 1 to 23 to the subject, wherein the disease is caused all or in part by a target cell having a second target present on the surface of the target cell.
41. The method of embodiment 40, wherein the subject is a human.
42. The method of embodiment 40 or 41, wherein the subject is a subject in need thereof.

## Claims

1. A multispecific antibody that binds Vβ17, comprising a heavy chain variable region (VH) and a light chain variable region (VL), wherein:
(i) the VH comprises a CDR1, a CDR2, a CDR3 of a VH comprising the amino acid sequence set forth in SEQ ID NO: 133, and the VL comprises a CDR1, a CDR2, a CDR3 of a VH comprising the amino acid sequence set forth in SEQ ID NO: 134;
(ii) the VH comprises a CDR1, a CDR2, a CDR3 of a VH comprising the amino acid sequence set forth in SEQ ID NO: 31, and the VL comprises a CDR1, a CDR2, a CDR3 of a VH comprising the amino acid sequence set forth in SEQ ID NO: 32;
(iii) the VH comprises a CDR1, a CDR2, a CDR3 of a VH comprising the amino acid sequence set forth in SEQ ID NO: 65, and the VL comprises a CDR1, a CDR2, a CDR3 of a VH comprising the amino acid sequence set forth in SEQ ID NO: 66;
(iv) the VH comprises a CDR1, a CDR2, a CDR3 of a VH comprising the amino acid sequence set forth in SEQ ID NO: 99, and the VL comprises a CDR1, a CDR2, a CDR3 of a VH comprising the amino acid sequence set forth in SEQ ID NO: 100;
(v) the VH comprises a CDR1, a CDR2, a CDR3 of a VH comprising the amino acid sequence set forth in SEQ ID NO: 167, and the VL comprises a CDR1, a CDR2, a CDR3 of a VH comprising the amino acid sequence set forth in SEQ ID NO: 168;
(vi) the VH comprises a CDR1, a CDR2, a CDR3 of a VH comprising the amino acid sequence set forth in SEQ ID NO: 201, and the VL comprises a CDR1, a CDR2, a CDR3 of a VH comprising the amino acid sequence set forth in SEQ ID NO: 202;
(vii) the VH comprises a CDR1, a CDR2, a CDR3 of a VH comprising the amino acid sequence set forth in SEQ ID NO: 235, and the VL comprises a CDR1, a CDR2, a CDR3 of a VH comprising the amino acid sequence set forth in SEQ ID NO: 236;
(viii) the VH comprises a CDR1, a CDR2, a CDR3 of a VH comprising the amino acid sequence set forth in SEQ ID NO: 269, and the VL comprises a CDR1, a CDR2, a CDR3 of a VH comprising the amino acid sequence set forth in SEQ ID NO: 270;
(ix) the VH comprises a CDR1, a CDR2, a CDR3 of a VH comprising the amino acid sequence set forth in SEQ ID NO: 303, and the VL comprises a CDR1, a CDR2, a CDR3 of a VH comprising the amino acid sequence set forth in SEQ ID NO: 304;
(x) the VH comprises a CDR1, a CDR2, a CDR3 of a VH comprising the amino acid sequence set forth in SEQ ID NO: 337, and the VL comprises a CDR1, a CDR2, a CDR3 of a VH comprising the amino acid sequence set forth in SEQ ID NO: 338;
(xi) the VH comprises a CDR1, a CDR2, a CDR3 of a VH comprising the amino acid sequence set forth in SEQ ID NO: 371, and the VL comprises a CDR1, a CDR2, a CDR3 of a VH comprising the amino acid sequence set forth in SEQ ID NO: 372;
(xii) the VH comprises a CDR1, a CDR2, a CDR3 of a VH comprising the amino acid sequence set forth in SEQ ID NO: 405, and the VL comprises a CDR1, a CDR2, a CDR3 of a VH comprising the amino acid sequence set forth in SEQ ID NO: 406; or
(xiii) the VH comprises a CDR1, a CDR2, a CDR3 of a VH comprising the amino acid sequence set forth in SEQ ID NO: 439, and the VL comprises a CDR1, a CDR2, a CDR3 of a VH comprising the amino acid sequence set forth in SEQ ID NO: 440.

2. The antibody of claim 1, wherein:
(i) the VH comprises the amino acid sequence set forth in SEQ ID NO: 133, and the VL comprises the amino acid sequence set forth in SEQ ID NO: 134;
(ii) the VH comprises the amino acid sequence set forth in SEQ ID NO: 31, and the VL comprises the amino acid sequence set forth in SEQ ID NO: 32;
(iii) the VH comprises the amino acid sequence set forth in SEQ ID NO: 65, and the VL comprises the amino acid sequence set forth in SEQ ID NO: 66;
(iv) the VH comprises the amino acid sequence set forth in SEQ ID NO: 99, and the VL comprises the amino acid sequence set forth in SEQ ID NO: 100;
(v) the VH comprises the amino acid sequence set forth in SEQ ID NO: 167, and the VL comprises the amino acid sequence set forth in SEQ ID NO: 168;
(vi) the VH comprises the amino acid sequence set forth in SEQ ID NO: 201, and the VL comprises the amino acid sequence set forth in SEQ ID NO: 202;
(vii) the VH comprises the amino acid sequence set forth in SEQ ID NO: 235, and the VL comprises the amino acid sequence set forth in SEQ ID NO: 236;
(viii) the VH comprises the amino acid sequence set forth in SEQ ID NO: 269, and the VL comprises the amino acid sequence set forth in SEQ ID NO: 270;
(ix) the VH comprises the amino acid sequence set forth in SEQ ID NO: 303, and the VL comprises the amino acid sequence set forth in SEQ ID NO: 304;
(x) the VH comprises the amino acid sequence set forth in SEQ ID NO: 337, and the VL comprises the amino acid sequence set forth in SEQ ID NO: 338;
(xi) the VH comprises the amino acid sequence set forth in SEQ ID NO: 371, and the VL comprises the amino acid sequence set forth in SEQ ID NO: 372;
(xii) the VH comprises the amino acid sequence set forth in SEQ ID NO: 405, and the VL comprises the amino acid sequence set forth in SEQ ID NO: 406; or
(xiii) the VH comprises the amino acid sequence set forth in SEQ ID NO: 439, and the VL comprises the amino acid sequence set forth in SEQ ID NO: 440.

3. The antibody of claim 1 or 2, wherein:
(a) the antibody is an IgG antibody, optionally wherein the IgG antibody is an IgG1, IgG2, IgG3, or IgG4 antibody;
(b) the antibody comprises a kappa light chain or a lambda light chain; and/or
(c) the antibody is a bispecific antibody, a trispecific antibody, or a quadraspecific antibody.

4. A nucleic acid encoding the antibody of any one of claims 1-3.

5. A vector comprising the nucleic acid of claim 4.

6. A host cell comprising the vector of claim 5.

7. A kit comprising the antibody of any one of claims 1-3 and instructions for using the antibody.

8. A pharmaceutical composition comprising the antibody of any one of claims 1-3 and a pharmaceutically acceptable carrier.

9. A method of producing the pharmaceutical composition of claim 8, comprising combining the antibody with a pharmaceutically acceptable carrier to obtain the pharmaceutical composition.

10. A process for making the antibody of any one of claim 1-3, the process comprising growing the host cell of claim 6 under conditions to produce the antibody, optionally wherein the process further comprises isolating the antibody.

11. A method of:
(a) activating a T cell expressing Vβ17, comprising contacting the antibody of any one of claims 1-3 with the T cell expressing Vβ17, optionally wherein the contacting increases CD69, CD25, and/or Granzyme B expression, as compared to a T cell expressing Vβ17 that is not contacted with the antibody of any one of claims 1-3;
(b) directing a T cell expressing Vβ17 to a target cell, comprising contacting the antibody of any one of claims 1-3 with the target cell, wherein a second target is present on the surface of the target cell, and wherein the contacting directs the T cell to the target cell; or
(c) inhibiting the growth or proliferation of a target cell, comprising contacting the antibody of any one of claims 1-3 with the target cell having a second target present on the surface of the target cell, wherein the contacting is in the presence of a T cell expressing the Vβ17, and wherein the contacting results in inhibition of the growth or proliferation of the target cell.

12. The antibody of any one of claims 1-3 for use in a method of eliminating a target cell in a subject, comprising contacting the antibody with the target cell having a second target present on the surface of the target cell, wherein the contacting is in the presence of a T cell expressing the Vβ17, and wherein the contacting results in the elimination of the target cell, optionally wherein the target cell is a tumor cell.

13. The antibody of any one of claims 1-3 for use in a method of treating a disease in a subject, comprising administering an effective amount the antibody to the subject, wherein the disease is caused all or in part by a target cell having a second target present on the surface of the target cell.

14. The method of claim 11(b) or 11(c), wherein the target cell is a tumor cell.

15. The antibody for use of claim 13, wherein the disease is a cancer.
